(19) Europäisches Patentamt
European Patent Office
Office européen des brevets

(11) Veröffentlichungsnummer: **0 338 344 A2**

# (12) EUROPÄISCHE PATENTANMELDUNG

(21) Anmeldenummer: **89106122.8**

(22) Anmeldetag: **07.04.89**

(51) Int. Cl.⁴: **C07D 249/08 , A01N 43/653 , C07D 303/04 , C07D 303/08 , C07D 303/22 , C07C 49/233 , C07C 49/255**

(30) Priorität: **20.04.88 DE 3813253**

(43) Veröffentlichungstag der Anmeldung: **25.10.89 Patentblatt 89/43**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Büchel, Karl Heinz, Prof. Dr.**
**Dabringhausener Strasse 42**
**D-5093 Burscheid(DE)**
Erfinder: **Holmwood, Graham, Dr.**
**Krutscheider Weg 105**
**D-5600 Wuppertal 11(DE)**
Erfinder: **Hänssler, Gerd, Dr.**
**Am Arenzberg 58a**
**D-5090 Leverkusen 3(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**

(54) **Substituierte Triazolylmethylcarbinole.**

(57) Neue substituierte Triazolylmethylcarbinole der Formel

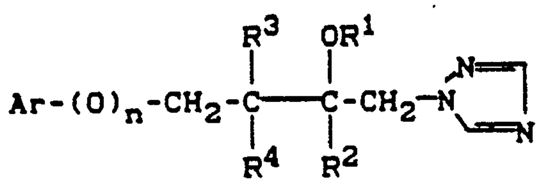

(I)

in welcher
n für die Zahlen 0 oder 1 steht,
Ar für gegebenenfalls substituiertes Phenyl oder für gegebenenfalls substituiertes Naphthyl steht,
$R^1$ für Wasserstoff, gegebenenfalls substituiertes Alkyl, gegebenenfalls substituiertes Alkenyl, gegebenenfalls substituiertes Alkinyl, gegebenenfalls substituiertes Aralkyl, Cycloalkyl oder gegebenenfalls substituiertes Heteroarylalkyl steht,
$R^2$ für Alkyl, Cycloalkyl, Cycloalkylalkyl oder Alkenyl steht,
$R^3$ für Methyl steht und
$R^4$ für Methyl steht oder
$R^3$ und $R^4$ gemeinsam für Ethan-1,2-diyl stehen,
sowie deren Säureadditions-Salze und Metallsalz-Komplexe,
mehrere Verfahren zur Herstellung der neuen Stoffe und deren Verwendung als Fungizide.
Neue Zwischenprodukte, Verfahren zu deren Herstellung und deren Verwendung zur Synthese von substituierten Triazolylmethylcarbinolen der Formel (I).

EP 0 338 344 A2

## Substituierte Triazolylmethylcarbinole

Die vorliegende Erfindung betrifft neue substituierte Triazolylmethylcarbinole, mehrere Verfahren zu ihrer Herstellung und ihre Verwendung als Fungizide.

Es ist bereits bekannt, daß bestimmte Triazolylmethylcarbinole fungizide Eigenschaften aufweisen (vergl. EP-OS 0 040 345). So kann zum Beispiel 1-(4-Chlorphenyl)-4-methyl-3-(1,2,4-triazol-1-yl-methyl)-pentan-3-ol zur Bekämpfung von Pilzen eingesetzt werden. Die Wirkung dieses Stoffes ist jedoch vor allem bei niedrigen Aufwandmengen nicht immer befriedigend.

Es wurden nun neue substituierte Triazolylmethylcarbinole der Formel

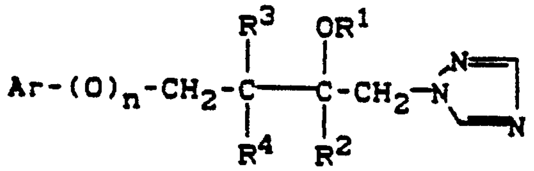
(I)

in welcher
n für die Zahlen 0 oder 1 steht,
Ar für gegebenenfalls substituiertes Phenyl oder für gegebenenfalls substituiertes Naphthyl steht,
$R^1$ für Wasserstoff, gegebenenfalls substituiertes Alkyl, gegebenenfalls substituiertes Alkenyl, gegebenenfalls substituiertes Alkinyl, gegebenenfalls substituiertes Aralkyl, Cycloalkyl oder gegebenenfalls substituiertes Heteroarylalkyl steht,
$R^2$ für Alkyl, Cycloalkyl, Cycloalkylalkyl oder Alkenyl steht,
$R^3$ für Methyl steht und
$R^4$ für Methyl steht oder
$R^3$ und $R^4$ gemeinsam für Ethan-1,2-diyl stehen,
sowie deren Säureadditions-Salze und Metallsalz-Komplexe gefunden.

Die neuen substituierten Triazolylmethylcarbinole der Formel (I) besitzen ein asymmetrisch substituiertes Kohlenstoffatom und können deshalb in den beiden optischen Isomerenformen anfallen. Die Erfindung betrifft sowohl die Isomerengemische als auch die einzelnen Isomeren.

Weiterhin wurde gefunden, daß man die neuen substituierten Triazolylmethylcarbinole der Formel (I) sowie deren Säureadditions-Salze und Metallsalz-Komplexe erhält, wenn man

(a) Triazolylmethylketone der Formel

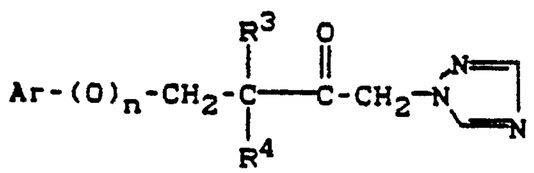
(II)

in welcher
n, Ar, $R^3$ und $R^4$ die oben angegebenen Bedeutungen haben,
mit Halogenverbindungen der Formel
$R^2$ - X (III)
in welcher
$R^2$ die oben angegebene Bedeutung hat und
X für Halogen steht,
in Gegenwart von Aluminium und gegebenenfalls unter Ultraschall und/oder in Gegenwart von Aluminiumaktivatoren in Gegenwart eines Verdünnungsmittels umsetzt
und anschließend mit Verbindungen der Formel
$R^1$ - Y (IV)
in welcher
$R^1$ die oben angegebene Bedeutung hat und
Y für Halogen steht,
in Gegenwart eines inerten Verdünnungsmittels umsetzt oder

(b) Oxirane der Formel

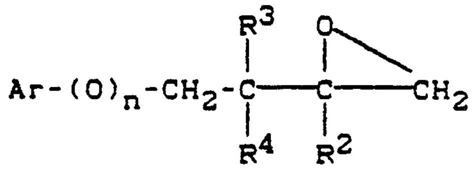 (V)

in welcher
n, Ar, $R^2$, $R^3$ und $R^4$ die oben angegebenen Bedeutungen haben,
mit 1,2,4-Triazol der Formel

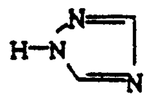 (VI)

gegebenenfalls in Gegenwart eines Säureakzeptors und gegebenenfalls in Gegenwart eines Radikalstarters sowie gegebenenfalls in Gegenwart eines Verdünnungsmittels umsetzt und gegebenenfalls die dabei anfallenden Verbindungen der Formel

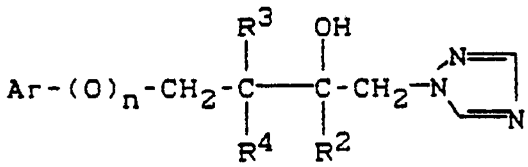 (Ia)

in welcher
Ar, $R^2$, $R^3$, $R^4$ und n die oben angegebene Bedeutung haben,
mit Verbindungen der Formel
$R^5$ - Y (IVa)
in welcher
Y die oben angegebene Bedeutung hat und
$R^5$ für gegebenenfalls substituiertes Alkyl, gegebenenfalls substituiertes Alkenyl, gegebenenfalls substituiertes Alkinyl, gegebenenfalls substituiertes Aralkyl, Cycloalkyl oder gegebenenfalls substituiertes Heteroarylalkyl steht,
in Gegenwart einer Base und gegebenenfalls in Gegenwart eines Verdünnungsmittels umsetzt,
und gegebenenfalls anschließend an die Verbindungen der Formel (I) eine Säure oder ein Metallsalz addiert.

Schließlich wurde gefunden, daß sich die neuen substituierten Triazolylmethylcarbinole der Formel (I) sowie deren Säureadditions-Salze und Metallsalz-Komplexe durch sehr gute fungizide Eigenschaften auszeichnen.

Überraschenderweise zeigen die erfindungsgemäßen substituierten Triazolylmethylcarbinole der Formel (I) sowie deren Säureadditions-Salze und Metallsalz-Komplexe wesentlich stärkere fungizide Wirkung ald das bekannte 1-(4-Chlorphenyl)-4-methyl-3-(1,2,4-triazol-1-yl-methyl)-pentan-3-ol, welches ein strukturell ähnlicher Wirkstoff gleicher Wirkungsrichtung ist.

Die erfindungsgemäßen Stoffe sind durch die Formel (I) allgemein definiert. Bevorzugt sind diejenigen substituierten Triazolylmethylcarbinole der Formel (I), in denen
n für die Zahlen 0 oder 1 steht,
Ar für Phenyl oder Naphthyl steht, wobei jeder dieser Reste einfach oder mehrfach, gleichartig oder verschieden substituiert sein kann durch Fluor, Chlor, Brom, Iod, Cyano, Nitro, Phenyl, Phenoxy, Benzyl, Formyl, Alkoximinomethyl mit 1 bis 4 Kohlenstoffatomen im Alkoxyteil, Alkylcarbonyl mit 1 bis 4 Kohlenstoffatomen im Alkylteil, Alkoximinoalkyl mit 1 bis 4 Kohlenstoffatomen im Alkoxyteil und 2 bis 4 Kohlenstoffatomen im Alkylteil, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Halogenatomen, insbesondere Fluor- und/oder Chloratomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 Halogenatomen,

insbesondere Fluor- und/oder Chloratomen, geradkettiges oder verzweigtes Alkylthio mit 1 bis 4 Kohlenstoffatomen, geradkettiges oder verzweigtes Halogenalkylthio mit 1 bis 4 Kohlenstoffatomen und 1 bis 9 Halogenatomen, insbe sondere Fluor und/oder Chloratomen, geradkettiges oder verzweigtes Alkylsulfinyl mit 1 bis 4 Kohlenstoffatomen, geradkettiges oder verzweigtes Halogenalkylsulfinyl mit 1 bis 4 Kohlenstoffatomen und 1 bis 9 Halogenatomen, insbesondere Fluor- und/oder Chloratomen, geradkettiges oder verzweigtes Alkylsulfonyl mit 1 bis 4 Kohlenstoffatomen und/oder durch geradkettiges oder verzweigtes halogenalkylsulfonyl mit 1 bis 4 Kohlenstoffatomen und 1 bis 9 Halogenatomen, insbesondere Fluor und/oder Chloratomen,

$R^1$ für Wasserstoff oder für geradkettiges oder verzweigtes Alkyl mit 1 bis 8 Kohlenstoffatomen steht, wobei jeder der Alkylreste einfach oder mehrfach, gleichartig oder verschieden substituiert sein kann durch Fluor, Chlor, Cyano, Alkylcarbonyl mit 1 bis 4 Kohlenstoffatomen im Alkylteil, Alkoxycarbonyl mit 1 bis 4 Kohlenstoffatomen im Alkoxyteil und/oder Alkoxy mit 1 bis 4 Kohlenstoffatomen, oder

$R^1$ für geradkettiges oder verzweigtes Alkenyl mit 2 bis 4 Kohlenstoffatomen steht, wobei jeder der Alkenylreste einfach oder mehrfach, gleichartig oder verschieden substituiert sein kann durch Fluor, Chlor, Cyano und/oder Alkoxycarbonyl mit 1 bis 4 Kohlenstoffatomen im Alkoxyteil, oder

$R^1$ für geradkettiges oder verzweigtes Alkinyl mit 2 bis 4 Kohlenstoffatomen steht, wobei jeder der Alkinylreste einfach oder mehrfach, gleichartig oder verschieden substituiert sein kann durch Fluor, Chlor, Cyano und/oder Alkoxycarbonyl mit 1 bis 4 Kohlenstoffatomen im Alkoxyteil, oder

$R^1$ für gegebenenfalls durch Fluor, Chlor, Cyano, Nitro, Methyl, Ethyl, Methoxy und/oder Trifluormethyl substituiertes Phenylalkyl mit 1 oder 2 Kohlenstoffatomen im Alkylteil steht, oder

$R^1$ für Cycloalkyl mit 3 bis 8 Kohlenstoffatomen steht, oder

$R^1$ für Pyridylmethyl, Pyrimidinylmethyl, Furylmethyl oder Thienylmethyl steht, wobei jeder dieser Reste einfach oder mehrfach, gleichartig oder verschieden substituiert sein kann durch Fluor, Chlor, Alkyl mit 1 bis 4 Kohlenstoffatomen und/oder Alkoxy mit 1 bis 4 Kohlenstoffatomen,

$R^2$ für geradkettiges oder verzweigtes Alkyl mit 1 bis 8 Kohlenstoffatomen, Cycloalkyl mit 3 bis 6 Kohlenstoffatomen, Cycloalkylalkyl mit 3 bis 6 Kohlenstoffatomen im Cycloalkylteil und 1 oder 2 Kohlenstoffatomen im Alkylteil steht, oder für geradkettiges oder verzweigtes Alkenyl mit 2 bis 6 Kohlenstoffatomen steht,

$R^3$ für Methyl steht und

$R^4$ für Methyl steht oder

$R^3$ und $R^4$ gemeinsam für Ethan-1,2-diyl stehen.

Besonders bevorzugt sind Verbindungen der Formel (I), in denen

n für die Zahlen 0 oder 1 steht,

Ar für Phenyl oder Naphthyl steht, wobei jeder dieser Reste einfach bis dreifach, gleichartig oder verschieden substituiert sein kann durch Fluor, Chlor, Brom, Iod, Cyano, Nitro, Phenyl, Phenoxy, Benzyl, Formyl, Alkoximinomethyl mit 1 oder 2 Kohlenstoffatomen inm Alkoxyteil, Alkylcarbonyl mit 1 oder 2 Kohlenstoffatomen im Alkylteil, Alkoximinoalkyl mit 1 oder 2 Kohlenstoffatomen im Alkoxyteil und 2 oder 3 Kohlenstoffatomen im Alkylteil, Alkyl mit 1 oder 2 Kohlenstoffatomen, Halogenalkyl mit 1 oder 2 Kohlenstoffatomen und 1 bis 5 Halogenatomen, insbesondere Fluor- und-oder Chloratomen, Alkoxy mit 1 oder 2 Kohlenstoffatomen, Halogenalkoxy mit 1 oder 2 Kohlenstoffatomen und 1 bis 5 Halogenatomen, insbesondere Fluor- und/oder Chloratomen, Alkylthio mit 1 oder 2 Kohlenstoffatomen, Halogenalkylthio mit 1 oder 2 Kohlenstoffatomen und 1 bis 5 Halogenatomen, insbesondere Fluor- und/oder Chloratomen, Alkylsulfinyl mit 1 oder 2 Kohlenstoffatomen, Halogenalkylsulfinyl mit 1 oder 2 Kohlenstoffatomen und 1 bis 5 Halogenatomen, insbesondere Fluor- und/oder Chloratomen, Alkylsulfonyl mit 1 oder 2 Kohlenstoffatomen und/oder durch Halogenalkylsulfonyl mit 1 oder 2 Kohlenstoffatomen und 1 bis 5 Halogenatomen, insbesondere Fluor- und/oder Chloratomen,

$R^1$ für Wasserstoff, Methyl, Ethyl, Propyl, Isopropyl, Butyl, Isobutyl, sec.-Butyl, Pentyl, Chlormethyl, Fluormethyl, Methylcarbonylmethyl, Methoxycarbonylmethyl, Methoxymethyl, Allyl, Propargyl oder für gegebenenfalls einfach bis dreifach, gleichartig oder verschieden durch Fluor, Chlor, Brom, Methyl, Methoxy und/oder Trifluormethyl substituiertes Benzyl steht, oder

$R^1$ für Cyclopropyl, Cyclobutyl, Cyclopentyl oder Cyclohexyl steht, oder

$R^1$ für Pyridylmethyl, Pyrimidinylmethyl, Furylmethyl oder Thienylmethyl steht, wobei jeder dieser Reste einfach oder zweifach, gleichartig oder verschieden substituiert sein kann durch Fluor, Chlor, Methyl und/oder Methoxy,

$R^2$ für geradkettiges oder verzweigtes Alkyl mit 1 bis 6 Kohlenstoffatomen, Cyclopropyl, Cyclopropylmethyl oder Alkenyl mit 2 bis 4 Kohlenstoffatomen steht,

$R^3$ für Methyl steht und

$R^4$ für Methyl steht oder

$R^3$ und $R^4$ gemeinsam für Ethan-1,2-diyl stehen.

Eine Gruppe ganz besonders bevorzugter substituierter Triazolylmethylcarbinole sind die Verbindungen der Formel (I), in denen

n für die Zahl 0 steht,

Ar für gegebenenfalls in der 4-Position durch Fluor, Chlor, Brom, Methyl, Trifluormethyl, Methoxy, Difluormethoxy oder Trifluormethoxy substituiertes Phenyl steht, oder für gegebenenfalls in der 4-Position sowie zusätzlich gleich oder verschieden in der 2- oder 3-Position durch Fluor, Chlor, Brom, Methyl, Trifluormethyl, Methoxy, Difluormethoxy oder Trifluormethoxy substituiertes Phenyl steht,

$R^1$ für Wasserstoff, Methyl, Ethyl, Propyl, Isopropyl, Butyl, Isobutyl, sec.-Butyl, Pentyl, Cyclopropyl oder Allyl steht,

$R^2$ für Alkyl mit 1 bis 6 Kohlenstoffatomen, Cyclopropyl oder Allyl steht,

$R^3$ für Methyl steht und

$R^4$ für Methyl steht.

Eine andere Gruppe ganz besonders bevorzugter erfindungsgemäßer Stoffe sind die Verbindungen der Formel (I), in denen

n für die Zahl 1 steht,

Ar für gegebenenfalls in der 4-Position durch Fluor, Chlor, Brom, Methyl, Trifluormethyl, Methoxy, Difluormethoxy oder Trifluormethoxy substituiertes Phenyl steht, oder für gegebenenfalls in der 4-Position sowie zusätzlich gleich oder verschieden in der 2- oder 3-Position durch Fluor, Chlor, Brom, Methyl, Trifluormethyl, Methoxy, Difluormethoxy oder Trifluormethoxy substituiertes Phenyl steht,

$R^1$ für Wasserstoff, Methyl, Ethyl, Propyl, Isopropyl, Butyl, Isobutyl, sec.-Butyl, Pentyl, Cyclopropyl oder Allyl steht,

$R^2$ für Alkyl mit 1 bis 6 Kohlenstoffatomen, Cyclopropyl oder Allyl steht,

$R^3$ für Methyl steht und

$R^4$ für Methyl steht.

Eine weitere Gruppe ganz besonders bevorzugter erfindungsgemäßer Stoffe sind die Verbindungen der Formel (I), in denen

n für die Zahl 0 steht,

Ar für gegebenenfalls in der 4-Position durch Fluor, Chlor, Brom, Methyl, Trifluormethyl, Methoxy, Difluormethoxy oder Trifluormethoxy substituiertes Phenyl steht, oder für gegebenenfalls in der 4-Position sowie zusätzlich gleich oder verschieden in der 2- oder 3-Position durch Fluor, Chlor, Brom, Methyl, Trifluormethyl, Methoxy, Difluormethoxy oder Trifluormethoxy substituiertes Phenyl steht,

$R^1$ für Wasserstoff, Methyl, Ethyl, Propyl, Isopropyl, Butyl, Isobutyl, sec.-Butyl, Pentyl, Cyclopropyl oder Allyl steht,

$R^2$ für Alkyl mit 1 bis 6 Kohlenstoffatomen, Cyclopropyl oder Allyl steht und

$R^3$ und $R^4$ gemeinsam für Ethan-1,2-diyl stehen.

Eine zusätzliche Gruppe ganz besonders bevorzugter erfindungsgemäßer Stoffe sind schließlich die Verbindungen der Formel (I), in denen

n für die Zahl 1 steht,

Ar für gegebenenfalls in der 4-Position durch Fluor, Chlor, Brom, Methyl, Trifluormethyl, Methoxy, Difluormethoxy oder Trifluormethoxy substituiertes Phenyl steht, oder für gegebenenfalls in der 4-Position sowie zusätzlich gleich oder verschieden in der 2- oder 3-Position durch Fluor, Chlor, Brom, Methyl, Trifluormethyl, Methoxy, Difluormethoxy oder Trifluormethoxy substituiertes Phenyl steht,

$R^1$ für Wasserstoff, Methyl, Ethyl, Propyl, Isopropyl, Butyl, Isobutyl, sec.-Butyl, Pentyl, Cyclopropyl oder Allyl steht,

$R^2$ für Alkyl mit 1 bis 6 Kohlenstoffatomen, Cyclopropyl oder Allyl steht und

$R^3$ und $R^4$ gemeinsam für Ethan-1,2-diyl stehen.

Bevorzugte erfindungsgemäße Verbindungen sind auch Additionsprodukte aus Säuren und denjenigen Verbindungen der Formel (I), in denen n, Ar, $R^1$, $R^2$, $R^3$ und $R^4$ diejenigen Bedeutungen haben, die im Zusammenhang mit der Beschreibung der erfindungsgemäßen Stoffe bereits vorzugsweise für diese Substituenten bzw. den Index n genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der

Elemente und denjenigen Verbindungen der Formel (I), in denen n, Ar, $R^1$, $R^2$, $R^3$ und $R^4$ die Bedeutungen haben, die im Zusammenhang mit der Beschreibung der erfindungsgemäßen Stoffe bereits vorzugsweise für diese Substituenten bzw. den Index n genannt wurden.

Hierbei sind Salze des Kupfers, Zinks, Mangans, Magnesiums, Zinns, Eisens und des Nickels besonders bevorzugt.

Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Beispiele für die erfindungsgemäßen Verbindungen der Formel (I) sind in der nachstehenden Tabelle 1 aufgeführt (vgl. auch die Herstellungsbeispiele).

**Tabelle: 1**

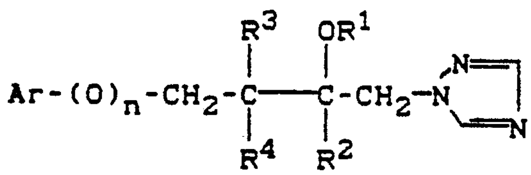 (I)

| Ar | n | $R^1$ | $R^2$ | $R^3$ | $R^4$ |
|---|---|---|---|---|---|
| 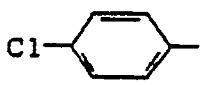 | 0 | H | $-CH_3$ | $CH_3$ | $CH_3$ |
| 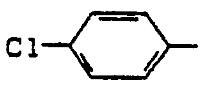 | 0 | H | $-C_2H_5$ | $CH_3$ | $CH_3$ |
| 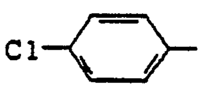 | 0 | H | $-C_3H_7$-n | $CH_3$ | $CH_3$ |
| 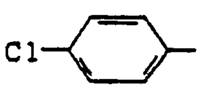 | 0 | H | $-CH(CH_3)_2$ | $CH_3$ | $CH_3$ |
| 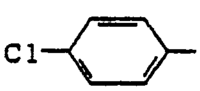 | 0 | H | 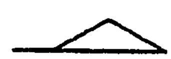 | $CH_3$ | $CH_3$ |
| 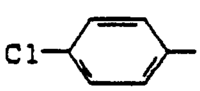 | 0 | H | $-CH_2CH=CH_2$ | $CH_3$ | $CH_3$ |
| 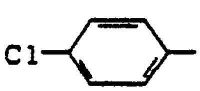 | 0 | H | $-(CH_2)_3-CH_3$ | $CH_3$ | $CH_3$ |
| 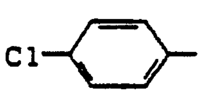 | 0 | H | $-CH_2CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 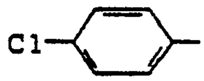 | 0 | H | $-(CH_2)_4-CH_3$ | $CH_3$ | $CH_3$ |
| 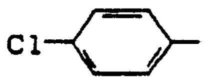 | 0 | H | $-(CH_2)_5-CH_3$ | $CH_3$ | $CH_3$ |
| 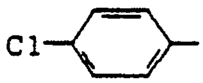 | 1 | H | $-CH_3$ | $CH_3$ | $CH_3$ |
| 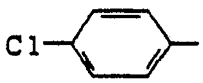 | 1 | H | $-C_2H_5$ | $CH_3$ | $CH_3$ |
| 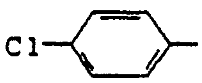 | 1 | H | $-C_3H_7$-n | $CH_3$ | $CH_3$ |
| 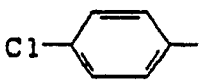 | 1 | H | $-CH(CH_3)_2$ | $CH_3$ | $CH_3$ |
| 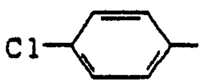 | 1 | H | 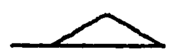 | $CH_3$ | $CH_3$ |
| 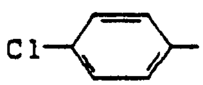 | 1 | H | $-CH_2CH=CH_2$ | $CH_3$ | $CH_3$ |
| 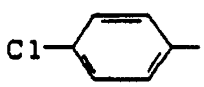 | 1 | H | $-(CH_2)_3-CH_3$ | $CH_3$ | $CH_3$ |
| 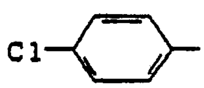 | 1 | H | $-CH_2CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 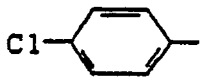 | 1 | H | $-(CH_2)_4-CH_3$ | $CH_3$ | $CH_3$ |
| 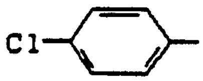 | 1 | H | $-(CH_2)_5-CH_3$ | $CH_3$ | $CH_3$ |
| 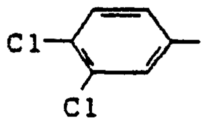 | 0 | H | $-CH_3$ | $CH_3$ | $CH_3$ |
| 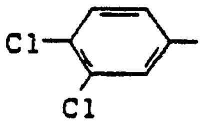 | 1 | H | $-CH_3$ | $CH_3$ | $CH_3$ |
| 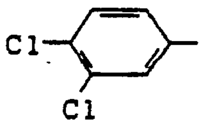 | 0 | H | $-C_2H_5$ | $CH_3$ | $CH_3$ |
| 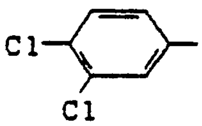 | 1 | H | $-C_2H_5$ | $CH_3$ | $CH_3$ |
| 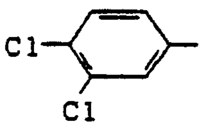 | 0 | H | $-CH_2CH_2CH_3$ | $CH_3$ | $CH_3$ |
| 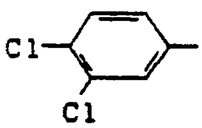 | 1 | H | $-CH_2CH_2CH_3$ | $CH_3$ | $CH_3$ |
| 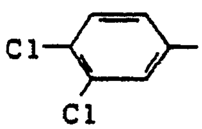 | 0 | H | $-CH(CH_3)_2$ | $CH_3$ | $CH_3$ |
| 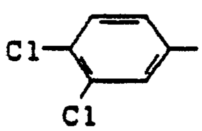 | 1 | H | $-CH(CH_3)_2$ | $CH_3$ | $CH_3$ |

<u>T a b e l l e: 1</u> - Fortsetzung

| Ar | n | $R^1$ | $R^2$ | $R^3$ | $R^4$ |
|---|---|---|---|---|---|
| 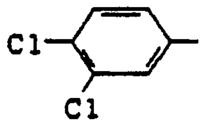 | 0 | H | 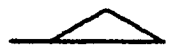 | $CH_3$ | $CH_3$ |
| 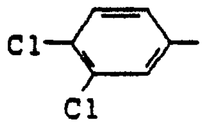 | 1 | H | 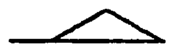 | $CH_3$ | $CH_3$ |
| 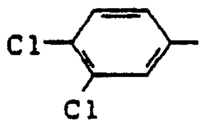 | 0 | H | $-CH_2CH=CH_2$ | $CH_3$ | $CH_3$ |
| 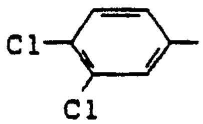 | 1 | H | $-CH_2CH=CH_2$ | $CH_3$ | $CH_3$ |
| 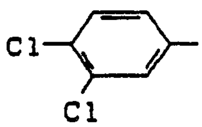 | 0 | H | $-(CH_2)_3-CH_3$ | $CH_3$ | $CH_3$ |
| 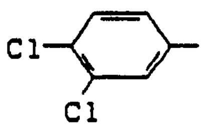 | 1 | H | $-(CH_2)_3-CH_3$ | $CH_3$ | $CH_3$ |
| 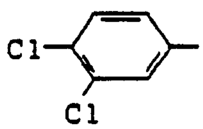 | 0 | H | $-CH_2CH(CH_3)_2$ | $CH_3$ | $CH_3$ |
| 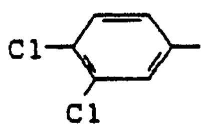 | 1 | H | $-CH_2CH(CH_3)_2$ | $CH_3$ | $CH_3$ |
| 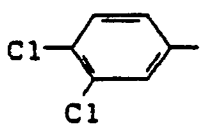 | 0 | H | $-(CH_2)_4-CH_3$ | $CH_3$ | $CH_3$ |
| 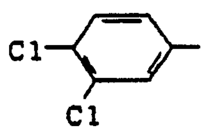 | 1 | H | $-(CH_2)_4-CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 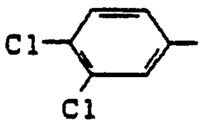 | 0 | H | $-(CH_2)_5-CH_3$ | $CH_3$ | $CH_3$ |
| 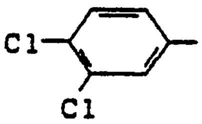 | 1 | H | $-(CH_2)_5-CH_3$ | $CH_3$ | $CH_3$ |
| 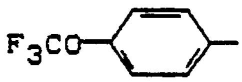 | 0 | H | $-CH_3$ | $CH_3$ | $CH_3$ |
| 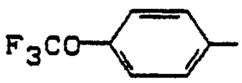 | 1 | H | $-CH_3$ | $CH_3$ | $CH_3$ |
| 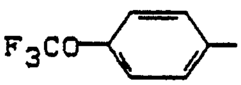 | 0 | H | $-C_2H_5$ | $CH_3$ | $CH_3$ |
| 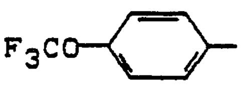 | 1 | H | $-C_2H_5$ | $CH_3$ | $CH_3$ |
| 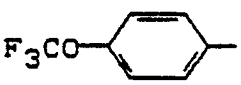 | 0 | H | $-CH_2CH_2CH_3$ | $CH_3$ | $CH_3$ |
| 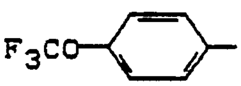 | 1 | H | $-CH_2CH_2CH_3$ | $CH_3$ | $CH_3$ |
| 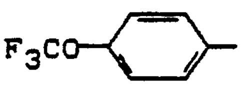 | 0 | H | $-CH(CH_3)_2$ | $CH_3$ | $CH_3$ |
| 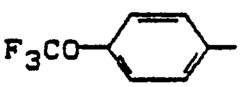 | 1 | H | $-CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 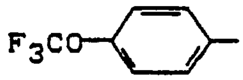 | 0 | H | 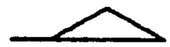 | $CH_3$ | $CH_3$ |
| 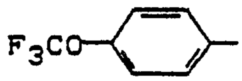 | 1 | H | 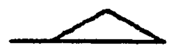 | $CH_3$ | $CH_3$ |
| 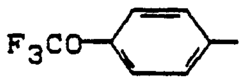 | 0 | H | $-CH_2CH=CH_2$ | $CH_3$ | $CH_3$ |
| 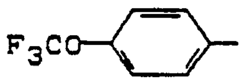 | 1 | H | $-CH_2CH=CH_2$ | $CH_3$ | $CH_3$ |
| 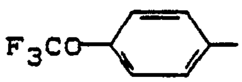 | 0 | H | $-(CH_2)_3-CH_3$ | $CH_3$ | $CH_3$ |
| 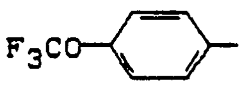 | 1 | H | $-(CH_2)_3-CH_3$ | $CH_3$ | $CH_3$ |
| 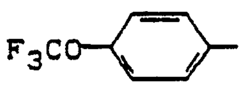 | 0 | H | $-CH_2CH(CH_3)_2$ | $CH_3$ | $CH_3$ |
| 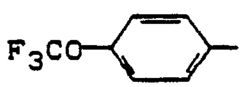 | 1 | H | $-CH_2CH(CH_3)_2$ | $CH_3$ | $CH_3$ |
| 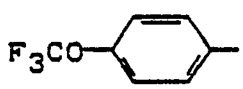 | 0 | H | $-(CH_2)_4-CH_3$ | $CH_3$ | $CH_3$ |
| 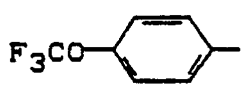 | 1 | H | $-(CH_2)_4-CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 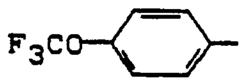 | 0 | H | $-(CH_2)_5-CH_3$ | $CH_3$ | $CH_3$ |
| 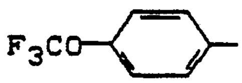 | 1 | H | $-(CH_2)_5-CH_3$ | $CH_3$ | $CH_3$ |
| 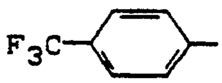 | 0 | H | $-CH_3$ | $CH_3$ | $CH_3$ |
| 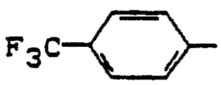 | 1 | H | $-CH_3$ | $CH_3$ | $CH_3$ |
| 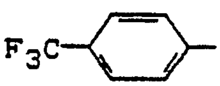 | 0 | H | $-C_2H_5$ | $CH_3$ | $CH_3$ |
| 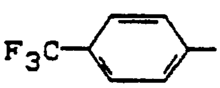 | 1 | H | $-C_2H_5$ | $CH_3$ | $CH_3$ |
| 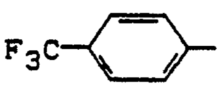 | 0 | H | $-CH_2CH_2CH_3$ | $CH_3$ | $CH_3$ |
| 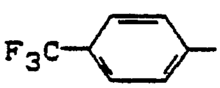 | 1 | H | $-CH_2CH_2CH_3$ | $CH_3$ | $CH_3$ |
| 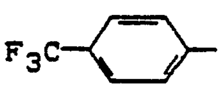 | 0 | H | $-CH(CH_3)_2$ | $CH_3$ | $CH_3$ |
| 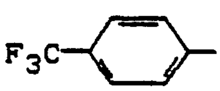 | 1 | H | $-CH(CH_3)_2$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 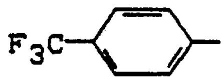 | 0 | H | 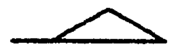 | $CH_3$ | $CH_3$ |
| 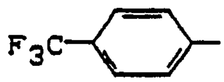 | 1 | H | 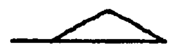 | $CH_3$ | $CH_3$ |
| 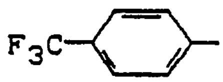 | 0 | H | $-CH_2CH=CH_2$ | $CH_3$ | $CH_3$ |
| 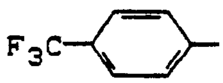 | 1 | H | $-CH_2CH=CH_2$ | $CH_3$ | $CH_3$ |
| 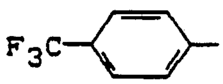 | 0 | H | $-(CH_2)_3-CH_3$ | $CH_3$ | $CH_3$ |
| 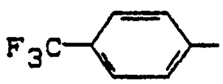 | 1 | H | $-(CH_2)_3-CH_3$ | $CH_3$ | $CH_3$ |
| 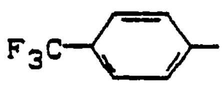 | 0 | H | $-CH_2CH(CH_3)_2$ | $CH_3$ | $CH_3$ |
| 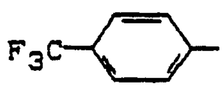 | 1 | H | $-CH_2CH(CH_3)_2$ | $CH_3$ | $CH_3$ |
| 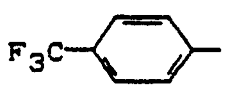 | 0 | H | $-(CH_2)_4-CH_3$ | $CH_3$ | $CH_3$ |
| 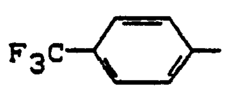 | 1 | H | $-(CH_2)_4-CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 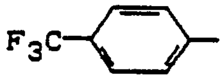 | 0 | H | $-(CH_2)_5-CH_3$ | $CH_3$ | $CH_3$ |
| 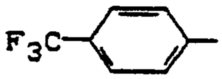 | 1 | H | $-(CH_2)_5-CH_3$ | $CH_3$ | $CH_3$ |
| 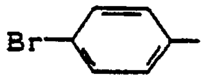 | 0 | H | $-CH_3$ | $CH_3$ | $CH_3$ |
| 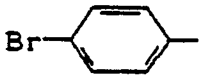 | 1 | H | $-CH_3$ | $CH_3$ | $CH_3$ |
| 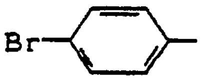 | 0 | H | $-C_2H_5$ | $CH_3$ | $CH_3$ |
| 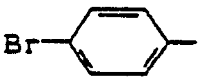 | 1 | H | $-C_2H_5$ | $CH_3$ | $CH_3$ |
| 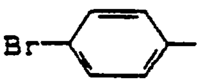 | 0 | H | $-CH_2CH_2CH_3$ | $CH_3$ | $CH_3$ |
| 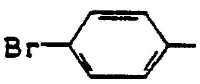 | 1 | H | $-CH_2CH_2CH_3$ | $CH_3$ | $CH_3$ |
| 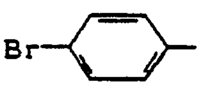 | 0 | H | $-CH(CH_3)_2$ | $CH_3$ | $CH_3$ |
| 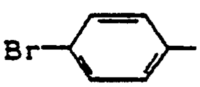 | 1 | H | $-CH(CH_3)_2$ | $CH_3$ | $CH_3$ |
| 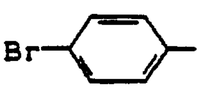 | 0 | H | 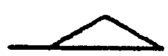 | $CH_3$ | $CH_3$ |
| 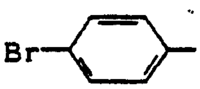 | 1 | H | 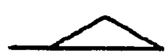 | $CH_3$ | $CH_3$ |

Tabelle: 1 - Fortsetzung

| Ar | n | $R^1$ | $R^2$ | $R^3$ | $R^4$ |
|---|---|---|---|---|---|
| 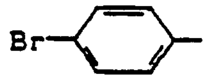 | 0 | H | $-CH_2CH=CH_2$ | $CH_3$ | $CH_3$ |
| 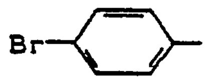 | 1 | H | $-CH_2CH=CH_2$ | $CH_3$ | $CH_3$ |
| 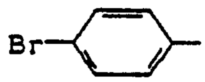 | 0 | H | $-(CH_2)_3-CH_3$ | $CH_3$ | $CH_3$ |
| 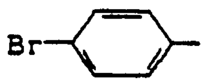 | 1 | H | $-(CH_2)_3-CH_3$ | $CH_3$ | $CH_3$ |
| 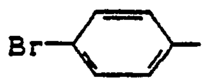 | 0 | H | $-CH_2CH(CH_3)_2$ | $CH_3$ | $CH_3$ |
| 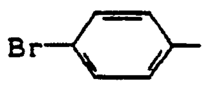 | 1 | H | $-CH_2CH(CH_3)_2$ | $CH_3$ | $CH_3$ |
| 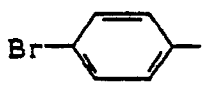 | 0 | H | $-(CH_2)_4-CH_3$ | $CH_3$ | $CH_3$ |
| 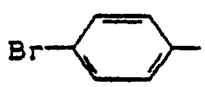 | 1 | H | $-(CH_2)_4-CH_3$ | $CH_3$ | $CH_3$ |
| 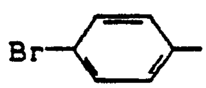 | 0 | H | $-(CH_2)_5-CH_3$ | $CH_3$ | $CH_3$ |
| 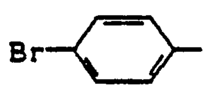 | 1 | H | $-(CH_2)_5-CH_3$ | $CH_3$ | $CH_3$ |

Tabelle 1 - Fortsetzung

| Ar | n | $R^1$ | $R^2$ | $R^3$ | $R^4$ |
|---|---|---|---|---|---|
| 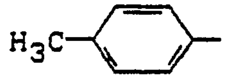 | 0 | H | $-CH_3$ | $CH_3$ | $CH_3$ |
| 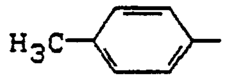 | 1 | H | $-CH_3$ | $CH_3$ | $CH_3$ |
| 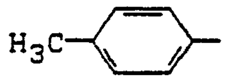 | 0 | H | $-C_2H_5$ | $CH_3$ | $CH_3$ |
| 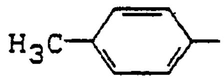 | 1 | H | $-C_2H_5$ | $CH_3$ | $CH_3$ |
| 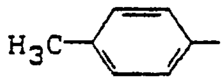 | 0 | H | $-CH_2CH_2CH_3$ | $CH_3$ | $CH_3$ |
| 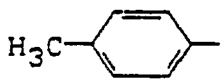 | 1 | H | $-CH_2CH_2CH_3$ | $CH_3$ | $CH_3$ |
| 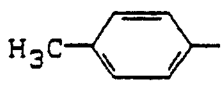 | 0 | H | $-CH(CH_3)_2$ | $CH_3$ | $CH_3$ |
| 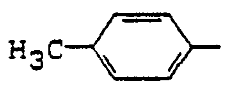 | 1 | H | $-CH(CH_3)_2$ | $CH_3$ | $CH_3$ |
| 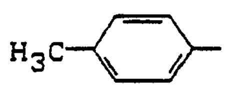 | 0 | H | 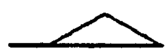 | $CH_3$ | $CH_3$ |
| 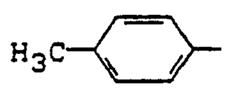 | 1 | H | 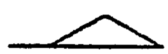 | $CH_3$ | $CH_3$ |
| 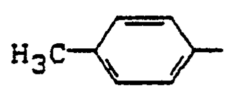 | 0 | H | $-CH_2CH=CH_2$ | $CH_3$ | $CH_3$ |
| 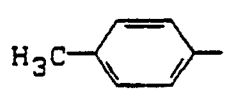 | 1 | H | $-CH_2CH=CH_2$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 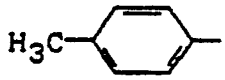 | 0 | H | $-(CH_2)_3-CH_3$ | $CH_3$ | $CH_3$ |
| 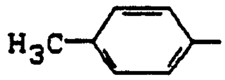 | 1 | H | $-(CH_2)_3-CH_3$ | $CH_3$ | $CH_3$ |
| 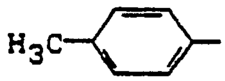 | 0 | H | $-CH_2CH(CH_3)_2$ | $CH_3$ | $CH_3$ |
| 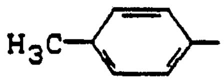 | 1 | H | $-CH_2CH(CH_3)_2$ | $CH_3$ | $CH_3$ |
| 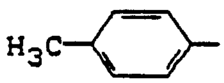 | 0 | H | $-(CH_2)_4-CH_3$ | $CH_3$ | $CH_3$ |
| 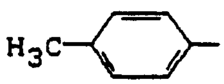 | 1 | H | $-(CH_2)_4-CH_3$ | $CH_3$ | $CH_3$ |
| 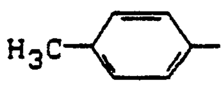 | 0 | H | $-(CH_2)_5-CH_3$ | $CH_3$ | $CH_3$ |
| 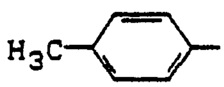 | 1 | H | $-(CH_2)_5-CH_3$ | $CH_3$ | $CH_3$ |
| 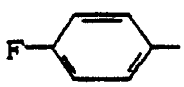 | 0 | H | $-CH_3$ | $CH_3$ | $CH_3$ |
| 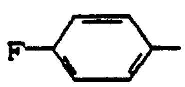 | 1 | H | $-CH_3$ | $CH_3$ | $CH_3$ |
| 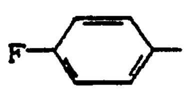 | 0 | H | $-C_2H_5$ | $CH_3$ | $CH_3$ |
| 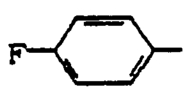 | 1 | H | $-C_2H_5$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 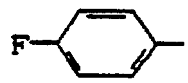 | 0 | H | $-CH_2CH_2CH_3$ | $CH_3$ | $CH_3$ |
| 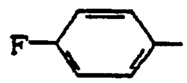 | 1 | H | $-CH_2CH_2CH_3$ | $CH_3$ | $CH_3$ |
| 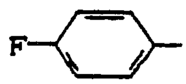 | 0 | H | $-CH(CH_3)_2$ | $CH_3$ | $CH_3$ |
| 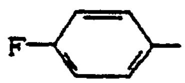 | 1 | H | $-CH(CH_3)_2$ | $CH_3$ | $CH_3$ |
| 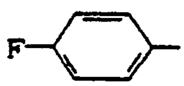 | 0 | H | 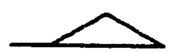 | $CH_3$ | $CH_3$ |
| 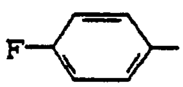 | 1 | H | 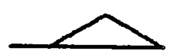 | $CH_3$ | $CH_3$ |
| 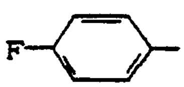 | 0 | H | $-CH_2CH=CH_2$ | $CH_3$ | $CH_3$ |
| 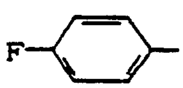 | 1 | H | $-CH_2CH=CH_2$ | $CH_3$ | $CH_3$ |
| 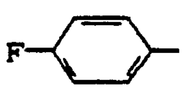 | 0 | H | $-(CH_2)_3-CH_3$ | $CH_3$ | $CH_3$ |
| 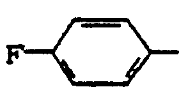 | 1 | H | $-(CH_2)_3-CH_3$ | $CH_3$ | $CH_3$ |
| 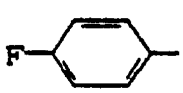 | 0 | H | $-CH_2CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 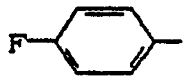 | 1 | H | $-CH_2CH(CH_3)_2$ | $CH_3$ | $CH_3$ |
| 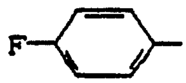 | 0 | H | $-(CH_2)_4-CH_3$ | $CH_3$ | $CH_3$ |
| 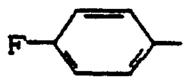 | 1 | H | $-(CH_2)_4-CH_3$ | $CH_3$ | $CH_3$ |
| 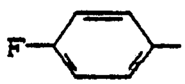 | 0 | H | $-(CH_2)_5-CH_3$ | $CH_3$ | $CH_3$ |
| 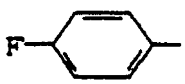 | 1 | H | $-(CH_2)_5-CH_3$ | $CH_3$ | $CH_3$ |
| 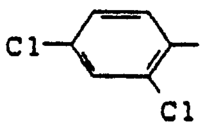 | 0 | H | $-CH_3$ | $CH_3$ | $CH_3$ |
| 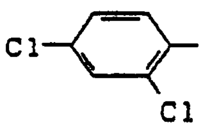 | 1 | H | $-CH_3$ | $CH_3$ | $CH_3$ |
| 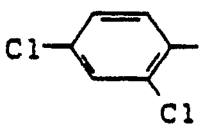 | 0 | H | $-C_2H_5$ | $CH_3$ | $CH_3$ |
| 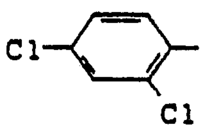 | 1 | H | $-C_2H_5$ | $CH_3$ | $CH_3$ |
| 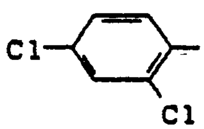 | 0 | H | $-CH_2CH_2CH_3$ | $CH_3$ | $CH_3$ |
| 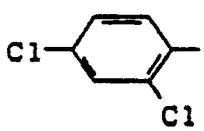 | 1 | H | $-CH_2CH_2CH_3$ | $CH_3$ | $CH_3$ |
| 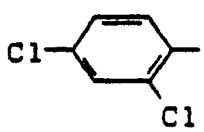 | 0 | H | $-CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 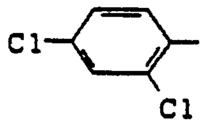 | 1 | H | $-CH(CH_3)_2$ | $CH_3$ | $CH_3$ |
| 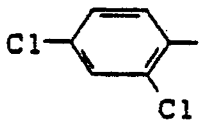 | 0 | H | 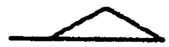 | $CH_3$ | $CH_3$ |
| 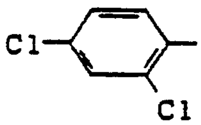 | 1 | H | 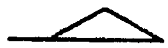 | $CH_3$ | $CH_3$ |
| 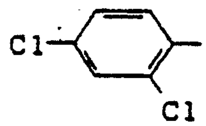 | 0 | H | $-CH_2CH=CH_2$ | $CH_3$ | $CH_3$ |
| 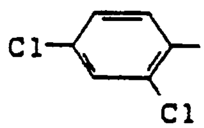 | 1 | H | $-CH_2CH=CH_2$ | $CH_3$ | $CH_3$ |
| 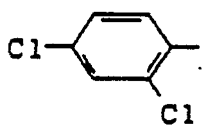 | 0 | H | $-(CH_2)_3-CH_3$ | $CH_3$ | $CH_3$ |
| 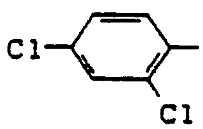 | 1 | H | $-(CH_2)_3-CH_3$ | $CH_3$ | $CH_3$ |
| 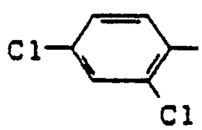 | 0 | H | $-CH_2CH(CH_3)_2$ | $CH_3$ | $CH_3$ |
| 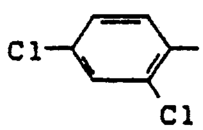 | 1 | H | $-CH_2CH(CH_3)_2$ | $CH_3$ | $CH_3$ |
| 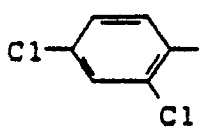 | 0 | H | $-(CH_2)_4-CH_3$ | $CH_3$ | $CH_3$ |
| 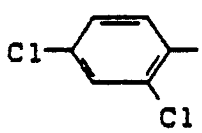 | 1 | H | $-(CH_2)_4-CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 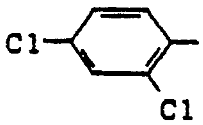 | 0 | H | $-(CH_2)_5-CH_3$ | $CH_3$ | $CH_3$ |
| 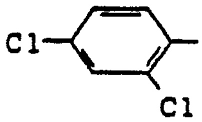 | 1 | H | $-(CH_2)_5-CH_3$ | $CH_3$ | $CH_3$ |
| 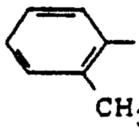 | 0 | H | $-CH_3$ | $CH_3$ | $CH_3$ |
| 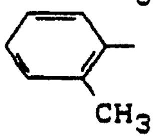 | 1 | H | $-CH_3$ | $CH_3$ | $CH_3$ |
| 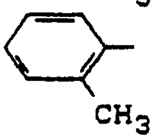 | 0 | H | $-C_2H_5$ | $CH_3$ | $CH_3$ |
| 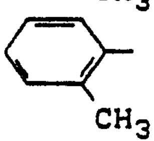 | 1 | H | $-C_2H_5$ | $CH_3$ | $CH_3$ |
| 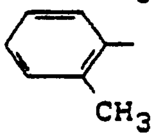 | 0 | H | $-CH_2CH_2CH_3$ | $CH_3$ | $CH_3$ |
| 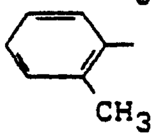 | 1 | H | $-CH_2CH_2CH_3$ | $CH_3$ | $CH_3$ |
| 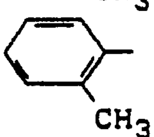 | 0 | H | $-CH(CH_3)_2$ | $CH_3$ | $CH_3$ |
| 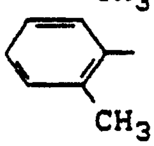 | 1 | H | $-CH(CH_3)_2$ | $CH_3$ | $CH_3$ |
| 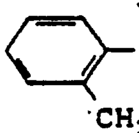 | 0 | H | 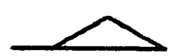 | $CH_3$ | $CH_3$ |

Tabelle: 1 - Fortsetzung

| Ar | n | $R^1$ | $R^2$ | $R^3$ | $R^4$ |
|---|---|---|---|---|---|
| 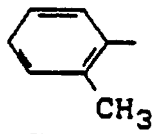 | 1 | H | 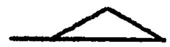 | $CH_3$ | $CH_3$ |
| 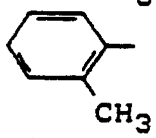 | 0 | H | $-CH_2CH=CH_2$ | $CH_3$ | $CH_3$ |
| 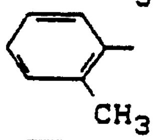 | 1 | H | $-CH_2CH=CH_2$ | $CH_3$ | $CH_3$ |
| 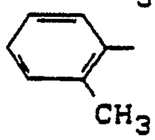 | 0 | H | $-(CH_2)_3-CH_3$ | $CH_3$ | $CH_3$ |
| 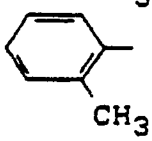 | 1 | H | $-(CH_2)_3-CH_3$ | $CH_3$ | $CH_3$ |
| 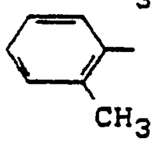 | 0 | H | $-CH_2CH(CH_3)_2$ | $CH_3$ | $CH_3$ |
| 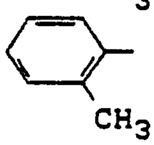 | 1 | H | $-CH_2CH(CH_3)_2$ | $CH_3$ | $CH_3$ |
| 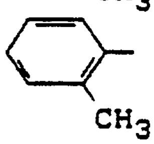 | 0 | H | $-(CH_2)_4-CH_3$ | $CH_3$ | $CH_3$ |
| 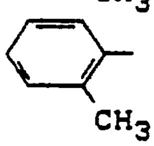 | 1 | H | $-(CH_2)_4-CH_3$ | $CH_3$ | $CH_3$ |
| 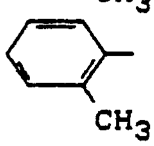 | 0 | H | $-(CH_2)_5-CH_3$ | $CH_3$ | $CH_3$ |
| 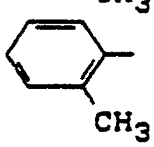 | 1 | H | $-(CH_2)_5-CH_3$ | $CH_3$ | $CH_3$ |
| 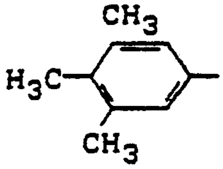 | 0 | H | $-CH_3$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 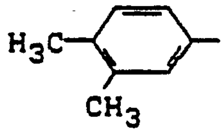 | 1 | H | $-CH_3$ | $CH_3$ | $CH_3$ |
| 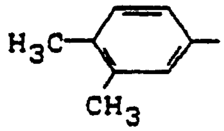 | 0 | H | $-C_2H_5$ | $CH_3$ | $CH_3$ |
| 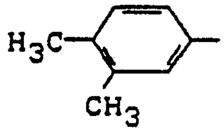 | 1 | H | $-C_2H_5$ | $CH_3$ | $CH_3$ |
| 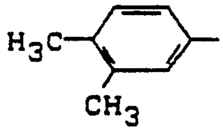 | 0 | H | $-CH_2CH_2CH_3$ | $CH_3$ | $CH_3$ |
| 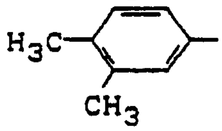 | 1 | H | $-CH_2CH_2CH_3$ | $CH_3$ | $CH_3$ |
| 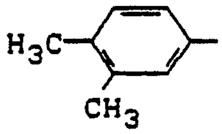 | 0 | H | $-CH(CH_3)_2$ | $CH_3$ | $CH_3$ |
| 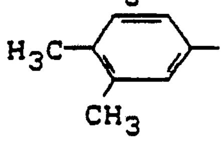 | 1 | H | $-CH(CH_3)_2$ | $CH_3$ | $CH_3$ |
| 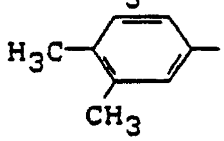 | 0 | H | 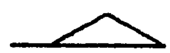 | $CH_3$ | $CH_3$ |
| 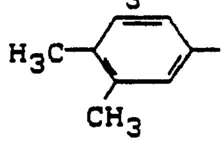 | 1 | H | 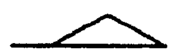 | $CH_3$ | $CH_3$ |
| 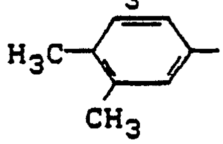 | 0 | H | $-CH_2CH=CH_2$ | $CH_3$ | $CH_3$ |
| 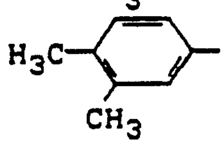 | 1 | H | $-CH_2CH=CH_2$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 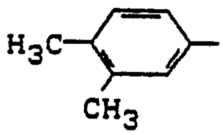 | 0 | H | $-(CH_2)_3-CH_3$ | $CH_3$ | $CH_3$ |
| 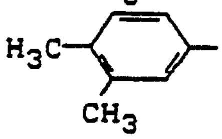 | 1 | H | $-(CH_2)_3-CH_3$ | $CH_3$ | $CH_3$ |
| 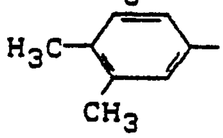 | 0 | H | $-CH_2CH(CH_3)_2$ | $CH_3$ | $CH_3$ |
| 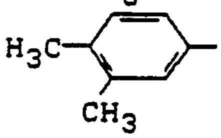 | 1 | H | $-CH_2CH(CH_3)_2$ | $CH_3$ | $CH_3$ |
| 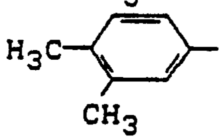 | 0 | H | $-(CH_2)_4-CH_3$ | $CH_3$ | $CH_3$ |
| 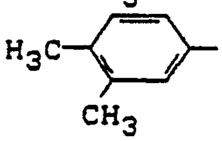 | 1 | H | $-(CH_2)_4-CH_3$ | $CH_3$ | $CH_3$ |
| 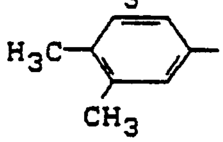 | 0 | H | $-(CH_2)_5-CH_3$ | $CH_3$ | $CH_3$ |
| 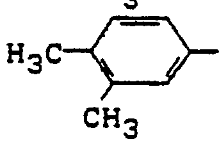 | 1 | H | $-(CH_2)_5-CH_3$ | $CH_3$ | $CH_3$ |
| 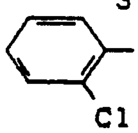 | 0 | H | $-CH_3$ | $CH_3$ | $CH_3$ |
| 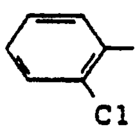 | 1 | H | $-CH_3$ | $CH_3$ | $CH_3$ |
| 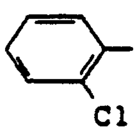 | 0 | H | $-C_2H_5$ | $CH_3$ | $CH_3$ |
| 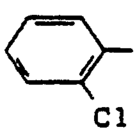 | 1 | H | $-C_2H_5$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 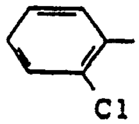 | 0 | H | $-CH_2CH_2CH_3$ | $CH_3$ | $CH_3$ |
| 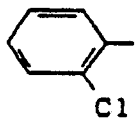 | 1 | H | $-CH_2CH_2CH_3$ | $CH_3$ | $CH_3$ |
| 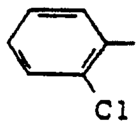 | 0 | H | $-CH(CH_3)_2$ | $CH_3$ | $CH_3$ |
| 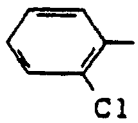 | 1 | H | $-CH(CH_3)_2$ | $CH_3$ | $CH_3$ |
| 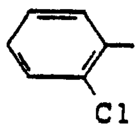 | 0 | H | 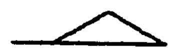 | $CH_3$ | $CH_3$ |
| 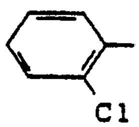 | 1 | H | 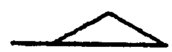 | $CH_3$ | $CH_3$ |
| 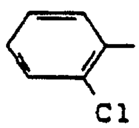 | 0 | H | $-CH_2CH=CH_2$ | $CH_3$ | $CH_3$ |
| 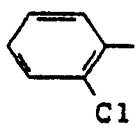 | 1 | H | $-CH_2CH=CH_2$ | $CH_3$ | $CH_3$ |
| 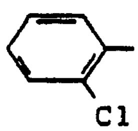 | 0 | H | $-(CH_2)_3-CH_3$ | $CH_3$ | $CH_3$ |
| 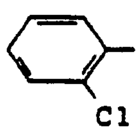 | 1 | H | $-(CH_2)_3-CH_3$ | $CH_3$ | $CH_3$ |
| 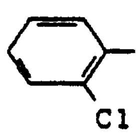 | 0 | H | $-CH_2CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 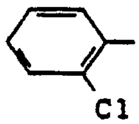 | 1 | H | $-CH_2CH(CH_3)_2$ | $CH_3$ | $CH_3$ |
| 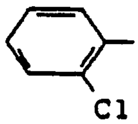 | 0 | H | $-(CH_2)_4-CH_3$ | $CH_3$ | $CH_3$ |
| 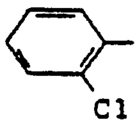 | 1 | H | $-(CH_2)_4-CH_3$ | $CH_3$ | $CH_3$ |
| 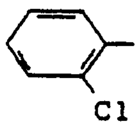 | 0 | H | $-(CH_2)_5-CH_3$ | $CH_3$ | $CH_3$ |
| 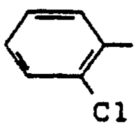 | 1 | H | $-(CH_2)_5-CH_3$ | $CH_3$ | $CH_3$ |
| 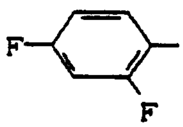 | 0 | H | $-CH_3$ | $CH_3$ | $CH_3$ |
| 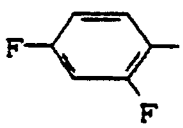 | 1 | H | $-CH_3$ | $CH_3$ | $CH_3$ |
| 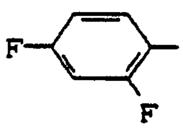 | 0 | H | $-C_2H_5$ | $CH_3$ | $CH_3$ |
| 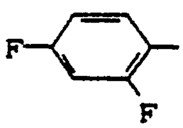 | 1 | H | $-C_2H_5$ | $CH_3$ | $CH_3$ |
| 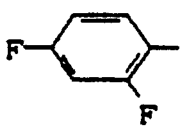 | 0 | H | $-CH_2CH_2CH_3$ | $CH_3$ | $CH_3$ |
| 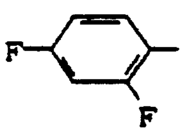 | 1 | H | $-CH_2CH_2CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 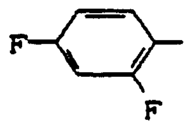 | 0 | H | $-CH(CH_3)_2$ | $CH_3$ | $CH_3$ |
| 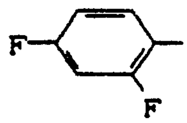 | 1 | H | $-CH(CH_3)_2$ | $CH_3$ | $CH_3$ |
| 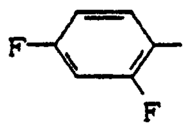 | 0 | H | 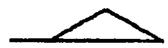 | $CH_3$ | $CH_3$ |
| 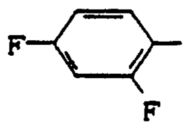 | 1 | H | 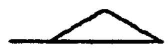 | $CH_3$ | $CH_3$ |
| 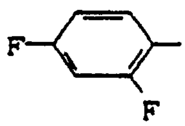 | 0 | H | $-CH_2CH{=}CH_2$ | $CH_3$ | $CH_3$ |
| 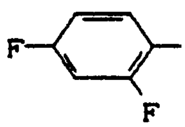 | 1 | H | $-CH_2CH{=}CH_2$ | $CH_3$ | $CH_3$ |
| 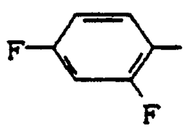 | 0 | H | $-(CH_2)_3-CH_3$ | $CH_3$ | $CH_3$ |
| 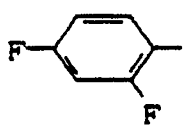 | 1 | H | $-(CH_2)_3-CH_3$ | $CH_3$ | $CH_3$ |
| 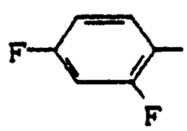 | 0 | H | $-CH_2CH(CH_3)_2$ | $CH_3$ | $CH_3$ |
| 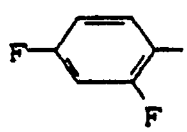 | 1 | H | $-CH_2CH(CH_3)_2$ | $CH_3$ | $CH_3$ |
| 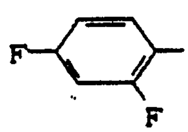 | 0 | H | $-(CH_2)_4-CH_3$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 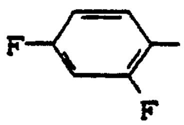 | 1 | H | $-(CH_2)_4-CH_3$ | $CH_3$ | $CH_3$ |
| 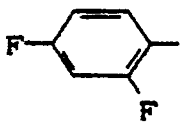 | 0 | H | $-(CH_2)_5-CH_3$ | $CH_3$ | $CH_3$ |
| 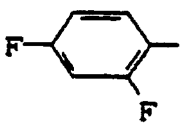 | 1 | H | $-(CH_2)_5-CH_3$ | $CH_3$ | $CH_3$ |
| 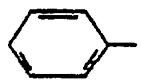 | 0 | H | $-CH_3$ | $CH_3$ | $CH_3$ |
| 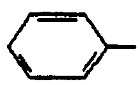 | 1 | H | $-CH_3$ | $CH_3$ | $CH_3$ |
| 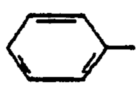 | 0 | H | $-C_2H_5$ | $CH_3$ | $CH_3$ |
| 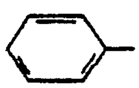 | 1 | H | $-C_2H_5$ | $CH_3$ | $CH_3$ |
| 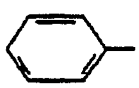 | 0 | H | $-CH_2CH_2CH_3$ | $CH_3$ | $CH_3$ |
| 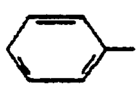 | 1 | H | $-CH_2CH_2CH_3$ | $CH_3$ | $CH_3$ |
| 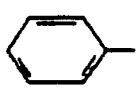 | 0 | H | $-CH(CH_3)_2$ | $CH_3$ | $CH_3$ |
| 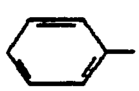 | 1 | H | $-CH(CH_3)_2$ | $CH_3$ | $CH_3$ |
| 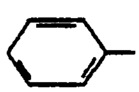 | 0 | H | 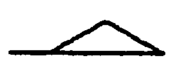 | $CH_3$ | $CH_3$ |
| 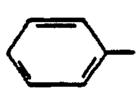 | 1 | H | 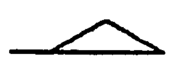 | $CH_3$ | $CH_3$ |

<u>T a b e l l e: 1</u> - Fortsetzung

| Ar | n | $R^1$ | $R^2$ | $R^3$ | $R^4$ |
|---|---|---|---|---|---|
| 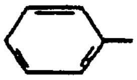 | 0 | H | $-CH_2CH{=}CH_2$ | $CH_3$ | $CH_3$ |
| 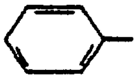 | 1 | H | $-CH_2CH{=}CH_2$ | $CH_3$ | $CH_3$ |
| 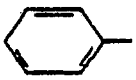 | 0 | H | $-(CH_2)_3-CH_3$ | $CH_3$ | $CH_3$ |
| 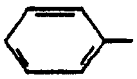 | 1 | H | $-(CH_2)_3-CH_3$ | $CH_3$ | $CH_3$ |
| 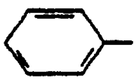 | 0 | H | $-CH_2CH(CH_3)_2$ | $CH_3$ | $CH_3$ |
| 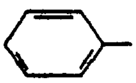 | 1 | H | $-CH_2CH(CH_3)_2$ | $CH_3$ | $CH_3$ |
| 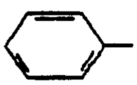 | 0 | H | $-(CH_2)_4-CH_3$ | $CH_3$ | $CH_3$ |
| 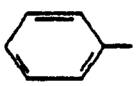 | 1 | H | $-(CH_2)_4-CH_3$ | $CH_3$ | $CH_3$ |
| 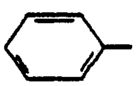 | 0 | H | $-(CH_2)_5-CH_3$ | $CH_3$ | $CH_3$ |
| 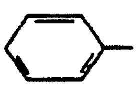 | 1 | H | $-(CH_2)_5-CH_3$ | $CH_3$ | $CH_3$ |
| 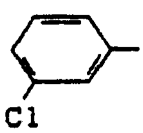 | 0 | H | $-CH_3$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 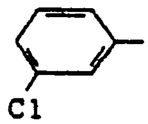 | 1 | H | $-CH_3$ | $CH_3$ | $CH_3$ |
| 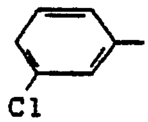 | 0 | H | $-C_2H_5$ | $CH_3$ | $CH_3$ |
| 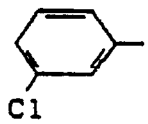 | 1 | H | $-C_2H_5$ | $CH_3$ | $CH_3$ |
| 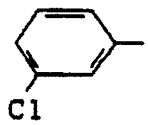 | 0 | H | $-CH_2CH_2CH_3$ | $CH_3$ | $CH_3$ |
| 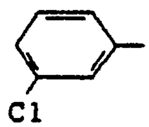 | 1 | H | $-CH_2CH_2CH_3$ | $CH_3$ | $CH_3$ |
| 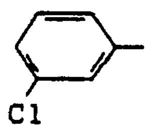 | 0 | H | $-CH(CH_3)_2$ | $CH_3$ | $CH_3$ |
| 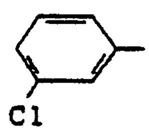 | 1 | H | $-CH(CH_3)_2$ | $CH_3$ | $CH_3$ |
| 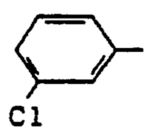 | 0 | H | 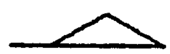 | $CH_3$ | $CH_3$ |
| 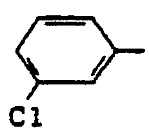 | 1 | H | 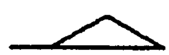 | $CH_3$ | $CH_3$ |
| 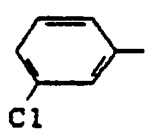 | 0 | H | $-CH_2CH=CH_2$ | $CH_3$ | $CH_3$ |
| 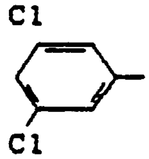 | 1 | H | $-CH_2CH=CH_2$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 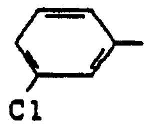 | 0 | H | $-(CH_2)_3-CH_3$ | $CH_3$ | $CH_3$ |
| 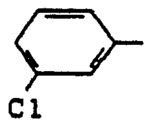 | 1 | H | $-(CH_2)_3-CH_3$ | $CH_3$ | $CH_3$ |
| 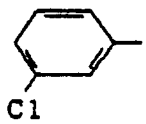 | 0 | H | $-CH_2CH(CH_3)_2$ | $CH_3$ | $CH_3$ |
| 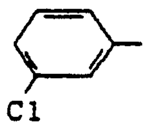 | 1 | H | $-CH_2CH(CH_3)_2$ | $CH_3$ | $CH_3$ |
| 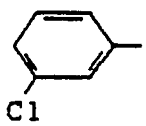 | 0 | H | $-(CH_2)_4-CH_3$ | $CH_3$ | $CH_3$ |
| 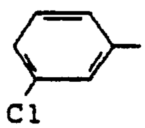 | 1 | H | $-(CH_2)_4-CH_3$ | $CH_3$ | $CH_3$ |
| 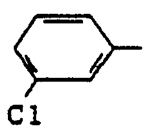 | 0 | H | $-(CH_2)_5-CH_3$ | $CH_3$ | $CH_3$ |
| 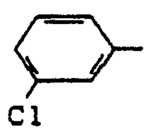 | 1 | H | $-(CH_2)_5-CH_3$ | $CH_3$ | $CH_3$ |
| 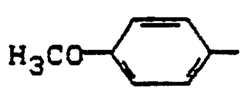 | 1 | H | $-CH_3$ | $CH_3$ | $CH_3$ |
| 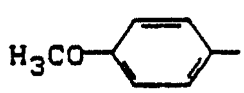 | 0 | H | $-C_2H_5$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 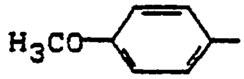 | 1 | H | $-C_2H_5$ | $CH_3$ | $CH_3$ |
| 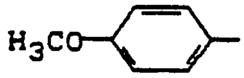 | 0 | H | $-CH_2CH_2CH_3$ | $CH_3$ | $CH_3$ |
| 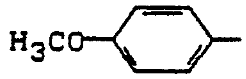 | 1 | H | $-CH_2CH_2CH_3$ | $CH_3$ | $CH_3$ |
| 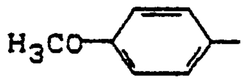 | 0 | H | $-CH(CH_3)_2$ | $CH_3$ | $CH_3$ |
| 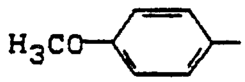 | 1 | H | $-CH(CH_3)_2$ | $CH_3$ | $CH_3$ |
| 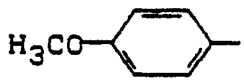 | 0 | H | 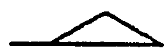 | $CH_3$ | $CH_3$ |
| 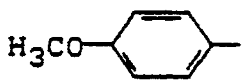 | 1 | H | 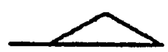 | $CH_3$ | $CH_3$ |
| 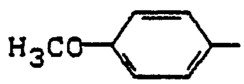 | 0 | H | $-CH_2CH=CH_2$ | $CH_3$ | $CH_3$ |
| 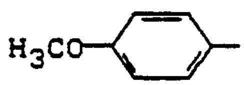 | 1 | H | $-CH_2CH=CH_2$ | $CH_3$ | $CH_3$ |
| 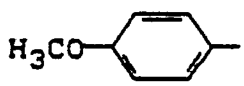 | 0 | H | $-(CH_2)_3-CH_3$ | $CH_3$ | $CH_3$ |
| 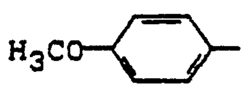 | 1 | H | $-(CH_2)_3-CH_3$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 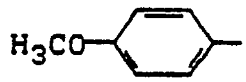 | 0 | H | $-CH_2CH(CH_3)_2$ | $CH_3$ | $CH_3$ |
| 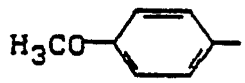 | 1 | H | $-CH_2CH(CH_3)_2$ | $CH_3$ | $CH_3$ |
| 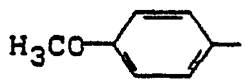 | 0 | H | $-(CH_2)_4-CH_3$ | $CH_3$ | $CH_3$ |
| 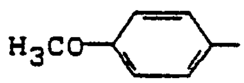 | 1 | H | $-(CH_2)_4-CH_3$ | $CH_3$ | $CH_3$ |
| 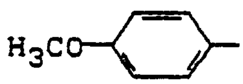 | 0 | H | $-(CH_2)_5-CH_3$ | $CH_3$ | $CH_3$ |
| 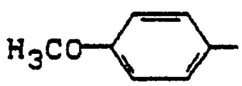 | 1 | H | $-(CH_2)_5-CH_3$ | $CH_3$ | $CH_3$ |
| 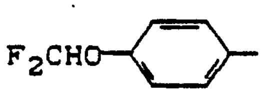 | 0 | H | $-CH_3$ | $CH_3$ | $CH_3$ |
| 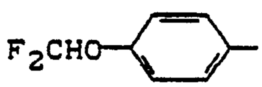 | 1 | H | $-CH_3$ | $CH_3$ | $CH_3$ |
| 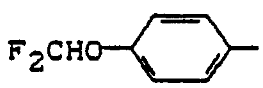 | 0 | H | $-C_2H_5$ | $CH_3$ | $CH_3$ |
| 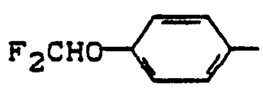 | 1 | H | $-C_2H_5$ | $CH_3$ | $CH_3$ |
| 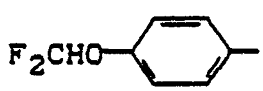 | 0 | H | $-CH_2CH_2CH_3$ | $CH_3$ | $CH_3$ |
| 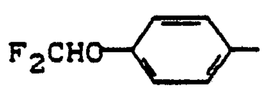 | 1 | H | $-CH_2CH_2CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 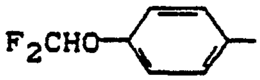 | 0 | H | $-CH(CH_3)_2$ | $CH_3$ | $CH_3$ |
| 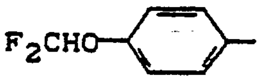 | 1 | H | $-CH(CH_3)_2$ | $CH_3$ | $CH_3$ |
| 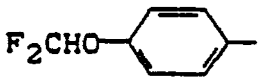 | 0 | H | 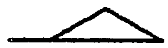 | $CH_3$ | $CH_3$ |
| 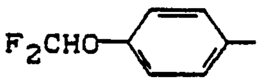 | 1 | H | 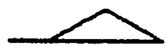 | $CH_3$ | $CH_3$ |
| 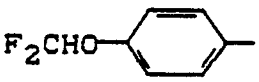 | 0 | H | $-CH_2CH{=}CH_2$ | $CH_3$ | $CH_3$ |
| 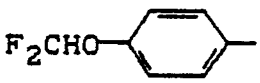 | 1 | H | $-CH_2CH{=}CH_2$ | $CH_3$ | $CH_3$ |
| 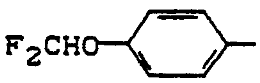 | 0 | H | $-(CH_2)_3-CH_3$ | $CH_3$ | $CH_3$ |
| 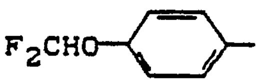 | 1 | H | $-(CH_2)_3-CH_3$ | $CH_3$ | $CH_3$ |
| 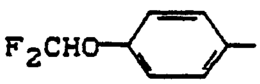 | 0 | H | $-CH_2CH(CH_3)_2$ | $CH_3$ | $CH_3$ |
| 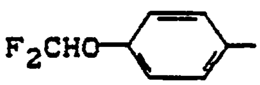 | 1 | H | $-CH_2CH(CH_3)_2$ | $CH_3$ | $CH_3$ |
| 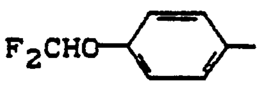 | 0 | H | $-(CH_2)_4-CH_3$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 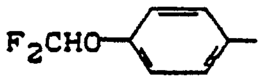 | 1 | H | $-(CH_2)_4-CH_3$ | $CH_3$ | $CH_3$ |
| 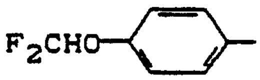 | 0 | H | $-(CH_2)_5-CH_3$ | $CH_3$ | $CH_3$ |
| 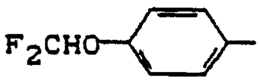 | 1 | H | $-(CH_2)_5-CH_3$ | $CH_3$ | $CH_3$ |
| 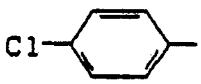 | 0 | $-CH_3$ | $-CH_3$ | $CH_3$ | $CH_3$ |
| 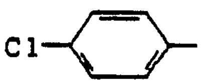 | 1 | $-CH_2CH=CH_2$ | $-C_2H_5$ | $CH_3$ | $CH_3$ |
| 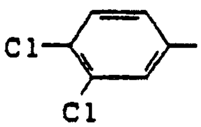 | 0 | $-CH_3$ | $-CH_2-CH_2-CH_3$ | $CH_3$ | $CH_3$ |
| 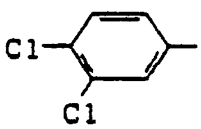 | 1 | $-CH_2CH=CH_2$ | $-CH(CH_3)_2$ | $CH_3$ | $CH_3$ |
| 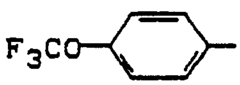 | 0 | $-CH_3$ | 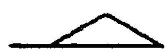 | $CH_3$ | $CH_3$ |
| 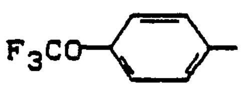 | 1 | $-CH_2CH=CH_2$ | $-CH_2CH=CH_2$ | $CH_3$ | $CH_3$ |
| 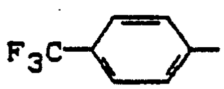 | 0 | $-CH_3$ | $-(CH_2)_3-CH_3$ | $CH_3$ | $CH_3$ |
| 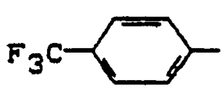 | 1 | $-CH_2CH=CH_2$ | $-CH_2CH(CH_3)_2$ | $CH_3$ | $CH_3$ |

T a b e l l e: 1 - Fortsetzung

| Ar | n | $R^1$ | $R^2$ | $R^3$ | $R^4$ |
|---|---|---|---|---|---|
| 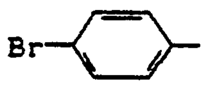 | 0 | $-CH_3$ | $-(CH_2)_4-CH_3$ | $CH_3$ | $CH_3$ |
| 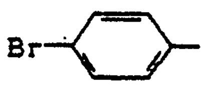 | 1 | $-CH_2CH=CH_2$ | $-(CH_2)_5-CH_3$ | $CH_3$ | $CH_3$ |
| 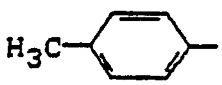 | 1 | $-CH_2CH=CH_2$ | $-CH_3$ | $CH_3$ | $CH_3$ |
| 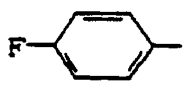 | 0 | $-CH_3$ | $-C_2H_5$ | $CH_3$ | $CH_3$ |
| 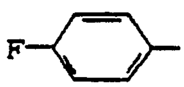 | 1 | $-CH_2CH=CH_2$ | $-CH_2CH_2CH_3$ | $CH_3$ | $CH_3$ |
| 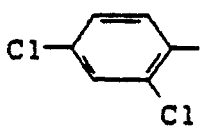 | 0 | $-CH_3$ | $-CH_3$ | $CH_3$ | $CH_3$ |
| 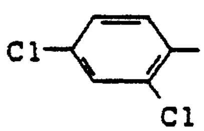 | 1 | $-CH_2CH=CH_2$ | $-C_2H_5$ | $CH_3$ | $CH_3$ |
| 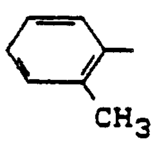 | 0 | $-CH_3$ | $-CH_2CH_2CH_3$ | $CH_3$ | $CH_3$ |
| 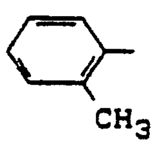 | 1 | $-CH_2CH=CH_2$ | $-CH(CH_3)_2$ | $CH_3$ | $CH_3$ |

Tabelle: 1 - Fortsetzung

| Ar | n | $R^1$ | $R^2$ | $R^3$ | $R^4$ |
|---|---|---|---|---|---|
| 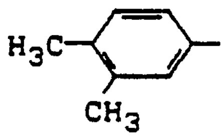 | 0 | $-CH_3$ | 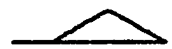 | $CH_3$ | $CH_3$ |
| 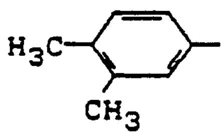 | 1 | $-CH_2CH{=}CH_2$ | $-CH_2CH{=}CH_2$ | $CH_3$ | $CH_3$ |
| 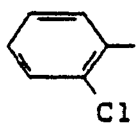 | 0 | $-CH_3$ | $-(CH_2)_3-CH_3$ | $CH_3$ | $CH_3$ |
| 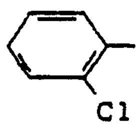 | 1 | $-CH_2CH{=}CH_2$ | $-CH_2CH(CH_3)_2$ | $CH_3$ | $CH_3$ |
| 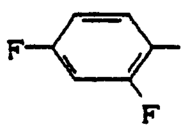 | 0 | $-CH_3$ | $-(CH_2)_4-CH_3$ | $CH_3$ | $CH_3$ |
| 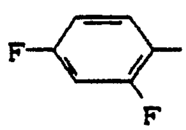 | 1 | $-CH_2CH{=}CH_2$ | $-(CH_2)_5-CH_3$ | $CH_3$ | $CH_3$ |
| 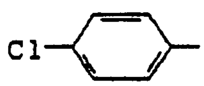 | 0 | H | $-CH_3$ | $-CH_2-CH_2-$ | |
| 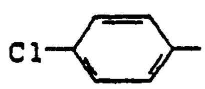 | 1 | H | $-CH_3$ | $-CH_2-CH_2-$ | |
| 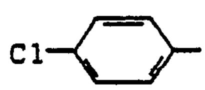 | 0 | H | $-C_2H_5$ | $-CH_2-CH_2-$ | |
| 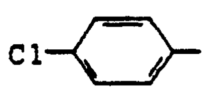 | 1 | H | $-C_2H_5$ | $-CH_2-CH_2-$ | |

T a b e l l e : 1 - Fortsetzung

| Ar | n | $R^1$ | $R^2$ | $R^3$ $R^4$ |
|---|---|---|---|---|
| 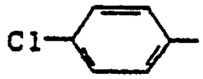 | 0 | H | $-CH_2CH_2CH_3$ | $-CH_2-CH_2-$ |
| 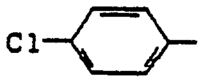 | 1 | H | $-CH_2CH_2CH_3$ | $-CH_2-CH_2-$ |
| 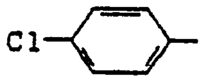 | 0 | H | $-CH(CH_3)_2$ | $-CH_2-CH_2-$ |
| 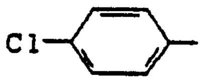 | 1 | H | $-CH(CH_3)_2$ | $-CH_2-CH_2-$ |
| 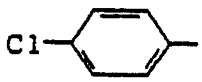 | 0 | H | 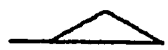 | $-CH_2-CH_2-$ |
| 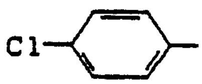 | 1 | H | 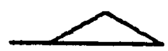 | $-CH_2-CH_2-$ |
| 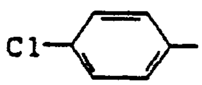 | 0 | H | $-CH_2CH=CH_2$ | $-CH_2-CH_2-$ |
| 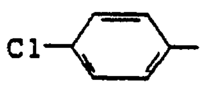 | 1 | H | $-CH_2CH=CH_2$ | $-CH_2-CH_2-$ |
| 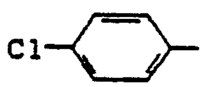 | 0 | H | $-(CH_2)_3-CH_3$ | $-CH_2-CH_2-$ |
| 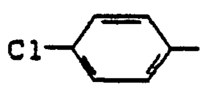 | 1 | H | $-(CH_2)_3-CH_3$ | $-CH_2-CH_2-$ |

Tabelle: 1 - Fortsetzung

| Ar | n | $R^1$ | $R^2$ | $R^3$ $R^4$ |
|---|---|---|---|---|
| 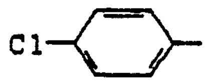 | 0 | H | $-CH_2CH(CH_3)_2$ | $-CH_2-CH_2-$ |
| 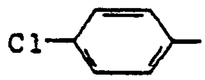 | 1 | H | $-CH_2CH(CH_3)_2$ | $-CH_2-CH_2-$ |
| 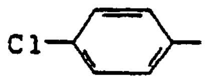 | 0 | H | $-(CH_2)_4-CH_3$ | $-CH_2-CH_2-$ |
| 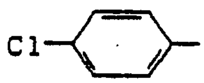 | 1 | H | $-(CH_2)_4-CH_3$ | $-CH_2-CH_2-$ |
| 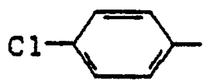 | 0 | H | $-(CH_2)_5-CH_3$ | $-CH_2-CH_2-$ |
| 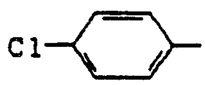 | 1 | H | $-(CH_2)_5-CH_3$ | $-CH_2-CH_2-$ |
| 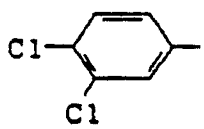 | 0 | H | $-CH_3$ | $-CH_2-CH_2-$ |
| 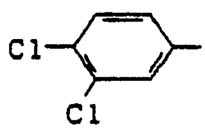 | 1 | H | $-CH_3$ | $-CH_2-CH_2-$ |
| 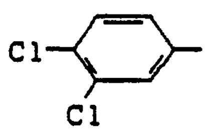 | 0 | H | $-C_2H_5$ | $-CH_2-CH_2-$ |
| 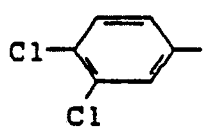 | 1 | H | $-C_2H_5$ | $-CH_2-CH_2-$ |
| 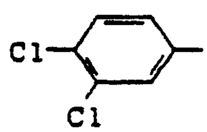 | 0 | H | $-CH_2CH_2CH_3$ | $-CH_2-CH_2-$ |

T a b e l l e: 1 - Fortsetzung

| Ar | n | $R^1$ | $R^2$ | $R^3$ | $R^4$ |
|---|---|---|---|---|---|
| 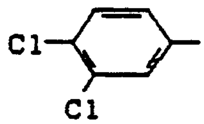 | 1 | H | $-CH_2CH_2CH_3$ | $-CH_2-CH_2-$ | |
| 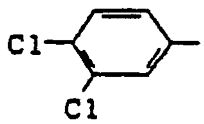 | 0 | H | $-CH(CH_3)_2$ | $-CH_2-CH_2-$ | |
| 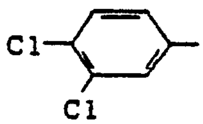 | 1 | H | $-CH(CH_3)_2$ | $-CH_2-CH_2-$ | |
| 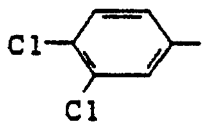 | 0 | H | 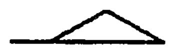 | $-CH_2-CH_2-$ | |
| 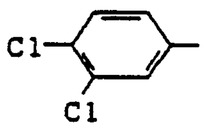 | 1 | H | 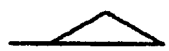 | $-CH_2-CH_2-$ | |
| 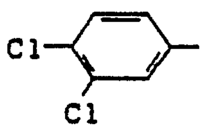 | 0 | H | $-CH_2CH=CH_2$ | $-CH_2-CH_2-$ | |
| 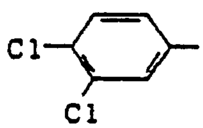 | 1 | H | $-CH_2CH=CH_2$ | $-CH_2-CH_2-$ | |
| 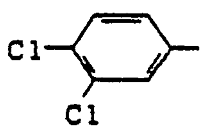 | 0 | H | $-(CH_2)_3-CH_3$ | $-CH_2-CH_2-$ | |
| 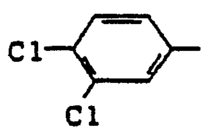 | 1 | H | $-(CH_2)_3-CH_3$ | $-CH_2-CH_2-$ | |
| 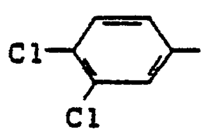 | 0 | H | $-CH_2CH(CH_3)_2$ | $-CH_2-CH_2-$ | |

Tabelle: 1 - Fortsetzung

| Ar | n | $R^1$ | $R^2$ | $R^3$ | $R^4$ |
|---|---|---|---|---|---|
| 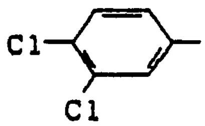 | 1 | H | $-CH_2CH(CH_3)_2$ | $-CH_2-CH_2-$ | |
| 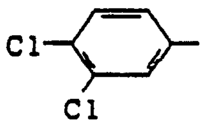 | 0 | H | $-(CH_2)_4-CH_3$ | $-CH_2-CH_2-$ | |
| 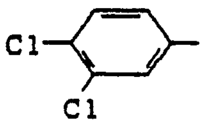 | 1 | H | $-(CH_2)_4-CH_3$ | $-CH_2-CH_2-$ | |
| 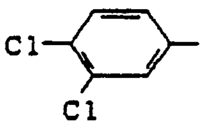 | 0 | H | $-(CH_2)_5-CH_3$ | $-CH_2-CH_2-$ | |
| 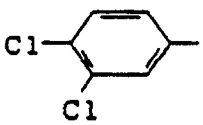 | 1 | H | $-(CH_2)_5-CH_3$ | $-CH_2-CH_2-$ | |
| 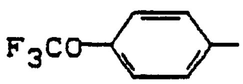 | 0 | H | $-CH_3$ | $-CH_2-CH_2-$ | |
| 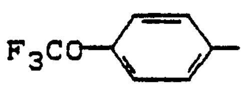 | 1 | H | $-CH_3$ | $-CH_2-CH_2-$ | |
| 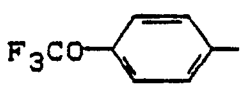 | 0 | H | $-C_2H_5$ | $-CH_2-CH_2-$ | |
| 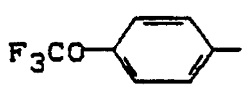 | 1 | H | $-C_2H_5$ | $-CH_2-CH_2-$ | |
| 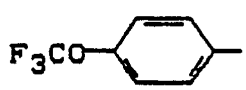 | 0 | H | $-CH_2CH_2CH_3$ | $-CH_2-CH_2-$ | |

Tabelle: 1 - Fortsetzung

| Ar | n | $R^1$ | $R^2$ | $R^3$ $R^4$ |
|---|---|---|---|---|
| 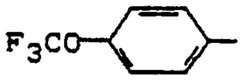 | 1 | H | $-CH_2CH_2CH_3$ | $-CH_2-CH_2-$ |
| 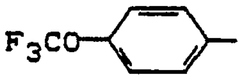 | 0 | H | $-CH(CH_3)_2$ | $-CH_2-CH_2-$ |
| 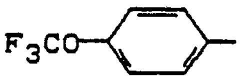 | 1 | H | $-CH(CH_3)_2$ | $-CH_2-CH_2-$ |
| 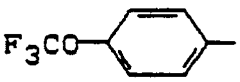 | 0 | H | 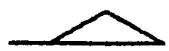 | $-CH_2-CH_2-$ |
| 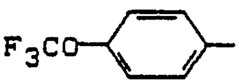 | 1 | H | 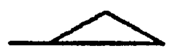 | $-CH_2-CH_2-$ |
| 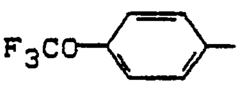 | 0 | H | $-CH_2CH=CH_2$ | $-CH_2-CH_2-$ |
| 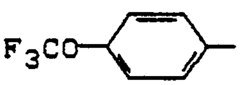 | 1 | H | $-CH_2CH=CH_2$ | $-CH_2-CH_2-$ |
| 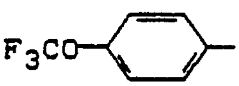 | 0 | H | $-(CH_2)_3-CH_3$ | $-CH_2-CH_2-$ |
| 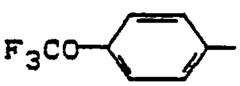 | 1 | H | $-(CH_2)_3-CH_3$ | $-CH_2-CH_2-$ |
| 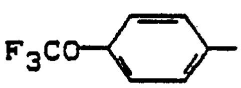 | 0 | H | $-CH_2CH(CH_3)_2$ | $-CH_2-CH_2-$ |

T a b e l l e: 1 - Fortsetzung

| Ar | n | $R^1$ | $R^2$ | $R^3$ | $R^4$ |
|---|---|---|---|---|---|
| 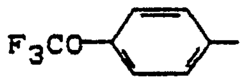 | 1 | H | $-CH_2CH(CH_3)_2$ | $-CH_2-CH_2-$ | |
| 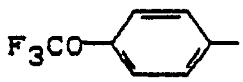 | 0 | H | $-(CH_2)_4-CH_3$ | $-CH_2-CH_2-$ | |
| 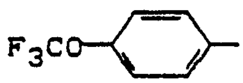 | 1 | H | $-(CH_2)_4-CH_3$ | $-CH_2-CH_2-$ | |
| 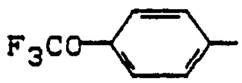 | 0 | H | $-(CH_2)_5-CH_3$ | $-CH_2-CH_2-$ | |
| 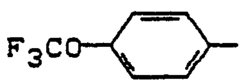 | 1 | H | $-(CH_2)_5-CH_3$ | $-CH_2-CH_2-$ | |
| 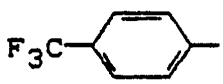 | 0 | H | $-CH_3$ | $-CH_2-CH_2-$ | |
| 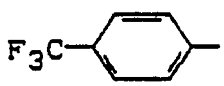 | 1 | H | $-CH_3$ | $-CH_2-CH_2-$ | |
| 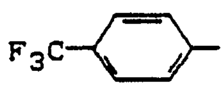 | 0 | H | $-C_2H_5$ | $-CH_2-CH_2-$ | |
| 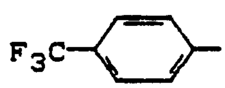 | 1 | H | $-C_2H_5$ | $-CH_2-CH_2-$ | |
| 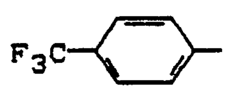 | 0 | H | $-CH_2CH_2CH_3$ | $-CH_2-CH_2-$ | |

<u>Tabelle: 1</u> - Fortsetzung

| Ar | n | $R^1$ | $R^2$ | $R^3$ | $R^4$ |
|---|---|---|---|---|---|
| 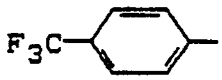 | 1 | H | $-CH_2CH_2CH_3$ | $-CH_2-CH_2-$ | |
| 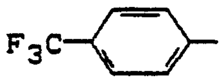 | 0 | H | $-CH(CH_3)_2$ | $-CH_2-CH_2-$ | |
| 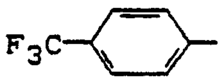 | 1 | H | $-CH(CH_3)_2$ | $-CH_2-CH_2-$ | |
| 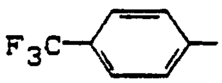 | 0 | H | 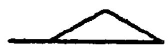 | $-CH_2-CH_2-$ | |
| 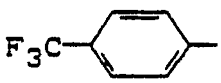 | 1 | H | 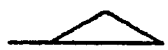 | $-CH_2-CH_2-$ | |
| 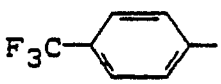 | 0 | H | $-CH_2CH=CH_2$ | $-CH_2-CH_2-$ | |
| 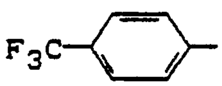 | 1 | H | $-CH_2CH=CH_2$ | $-CH_2-CH_2-$ | |
| 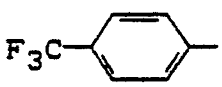 | 0 | H | $-(CH_2)_3-CH_3$ | $-CH_2-CH_2-$ | |
| 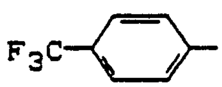 | 1 | H | $-(CH_2)_3-CH_3$ | $-CH_2-CH_2-$ | |
| 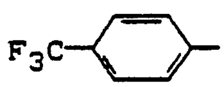 | 0 | H | $-CH_2CH(CH_3)_2$ | $-CH_2-CH_2-$ | |

Tabelle: 1 - Fortsetzung

| Ar | n | $R^1$ | $R^2$ | $R^3$ $R^4$ |
|---|---|---|---|---|
| 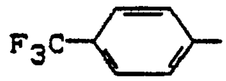 | 1 | H | $-CH_2CH(CH_3)_2$ | $-CH_2-CH_2-$ |
| 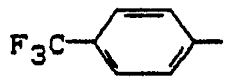 | 0 | H | $-(CH_2)_4-CH_3$ | $-CH_2-CH_2-$ |
| 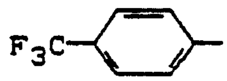 | 1 | H | $-(CH_2)_4-CH_3$ | $-CH_2-CH_2-$ |
| 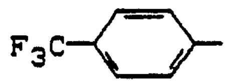 | 0 | H | $-(CH_2)_5-CH_3$ | $-CH_2-CH_2-$ |
| 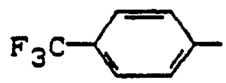 | 1 | H | $-(CH_2)_5-CH_3$ | $-CH_2-CH_2-$ |
| 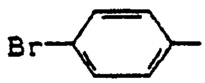 | 0 | H | $-CH_3$ | $-CH_2-CH_2-$ |
| 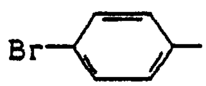 | 1 | H | $-CH_3$ | $-CH_2-CH_2-$ |
| 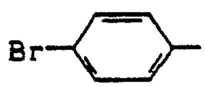 | 0 | H | $-C_2H_5$ | $-CH_2-CH_2-$ |
| 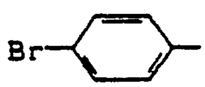 | 1 | H | $-C_2H_5$ | $-CH_2-CH_2-$ |
| 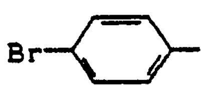 | 0 | H | $-CH_2CH_2CH_3$ | $-CH_2-CH_2-$ |

Tabelle: 1 - Fortsetzung

| Ar | n | $R^1$ | $R^2$ | $R^3$ $R^4$ |
|---|---|---|---|---|
| 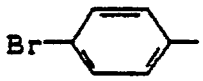 | 1 | H | $-CH_2CH_2CH_3$ | $-CH_2-CH_2-$ |
| 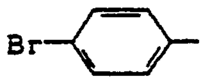 | 0 | H | $-CH(CH_3)_2$ | $-CH_2-CH_2-$ |
| 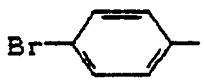 | 1 | H | $-CH(CH_3)_2$ | $-CH_2-CH_2-$ |
| 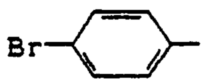 | 0 | H | 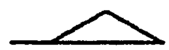 | $-CH_2-CH_2-$ |
| 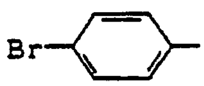 | 1 | H | 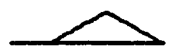 | $-CH_2-CH_2-$ |
| 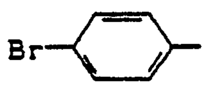 | 0 | H | $-CH_2CH=CH_2$ | $-CH_2-CH_2-$ |
| 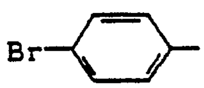 | 1 | H | $-CH_2CH=CH_2$ | $-CH_2-CH_2-$ |
| 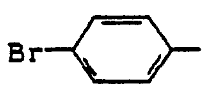 | 0 | H | $-(CH_2)_3-CH_3$ | $-CH_2-CH_2-$ |
| 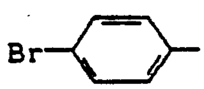 | 1 | H | $-(CH_2)_3-CH_3$ | $-CH_2-CH_2-$ |
| 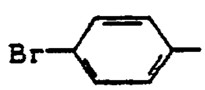 | 0 | H | $-CH_2CH(CH_3)_2$ | $-CH_2-CH_2-$ |

T a b e l l e: 1 - Fortsetzung

| Ar | n | $R^1$ | $R^2$ | $R^3$ $R^4$ |
|---|---|---|---|---|
| 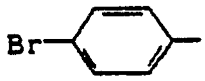 | 1 | H | $-CH_2CH(CH_3)_2$ | $-CH_2-CH_2-$ |
| 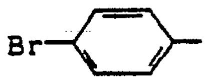 | 0 | H | $-(CH_2)_4-CH_3$ | $-CH_2-CH_2-$ |
| 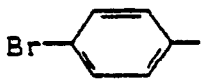 | 1 | H | $-(CH_2)_4-CH_3$ | $-CH_2-CH_2-$ |
| 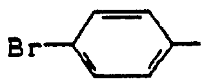 | 0 | H | $-(CH_2)_5-CH_3$ | $-CH_2-CH_2-$ |
| 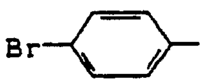 | 1 | H | $-(CH_2)_5-CH_3$ | $-CH_2-CH_2-$ |
| 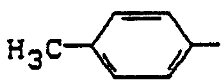 | 0 | H | $-CH_3$ | $-CH_2-CH_2-$ |
| 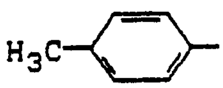 | 1 | H | $-CH_3$ | $-CH_2-CH_2-$ |
| 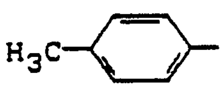 | 0 | H | $-C_2H_5$ | $-CH_2-CH_2-$ |
| 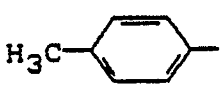 | 1 | H | $-C_2H_5$ | $-CH_2-CH_2-$ |
| 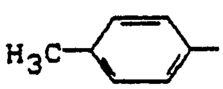 | 0 | H | $-CH_2CH_2CH_3$ | $-CH_2-CH_2-$ |
| 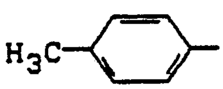 | 1 | H | $-CH_2CH_2CH_3$ | $-CH_2-CH_2-$ |

T a b e l l e: 1 - Fortsetzung

| Ar | n | $R^1$ | $R^2$ | $R^3$ $R^4$ |
|---|---|---|---|---|
| 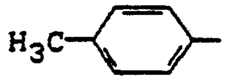 | 0 | H | $-CH(CH_3)_2$ | $-CH_2-CH_2-$ |
| 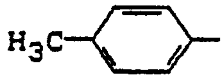 | 1 | H | $-CH(CH_3)_2$ | $-CH_2-CH_2-$ |
| 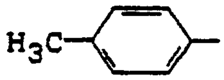 | 0 | H | 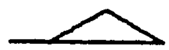 | $-CH_2-CH_2-$ |
| 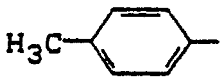 | 1 | H | 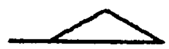 | $-CH_2-CH_2-$ |
| 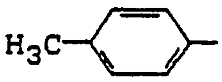 | 0 | H | $-CH_2CH=CH_2$ | $-CH_2-CH_2-$ |
| 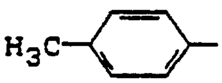 | 1 | H | $-CH_2CH=CH_2$ | $-CH_2-CH_2-$ |
| 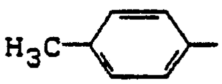 | 0 | H | $-(CH_2)_3-CH_3$ | $-CH_2-CH_2-$ |
| 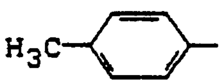 | 1 | H | $-(CH_2)_3-CH_3$ | $-CH_2-CH_2-$ |
| 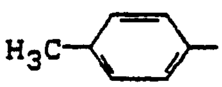 | 0 | H | $-CH_2CH(CH_3)_2$ | $-CH_2-CH_2-$ |
| 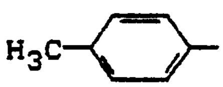 | 1 | H | $-CH_2CH(CH_3)_2$ | $-CH_2-CH_2-$ |

<u>T a b e l l e: 1</u> - Fortsetzung

| Ar | n | $R^1$ | $R^2$ | $R^3$ $R^4$ |
|---|---|---|---|---|
| 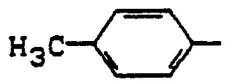 | 0 | H | $-(CH_2)_4-CH_3$ | $-CH_2-CH_2-$ |
| 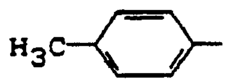 | 1 | H | $-(CH_2)_4-CH_3$ | $-CH_2-CH_2-$ |
| 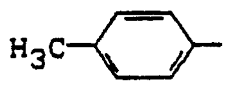 | 0 | H | $-(CH_2)_5-CH_3$ | $-CH_2-CH_2-$ |
| 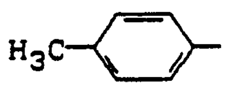 | 1 | H | $-(CH_2)_5-CH_3$ | $-CH_2-CH_2-$ |
| 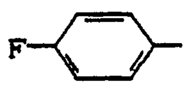 | 0 | H | $-CH_3$ | $-CH_2-CH_2-$ |
| 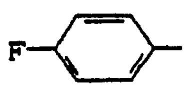 | 1 | H | $-CH_3$ | $-CH_2-CH_2-$ |
| 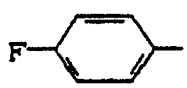 | 0 | H | $-C_2H_5$ | $-CH_2-CH_2-$ |
| 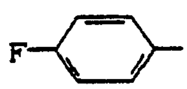 | 1 | H | $-C_2H_5$ | $-CH_2-CH_2-$ |
| 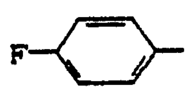 | 0 | H | $-CH_2CH_2CH_3$ | $-CH_2-CH_2-$ |
| 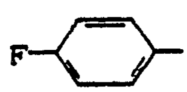 | 1 | H | $-CH_2CH_2CH_3$ | $-CH_2-CH_2-$ |
| 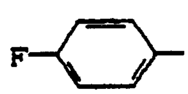 | 0 | H | $-CH(CH_3)_2$ | $-CH_2-CH_2-$ |

T a b e l l e: 1 - Fortsetzung

| Ar | n | $R^1$ | $R^2$ | $R^3$ | $R^4$ |
|---|---|---|---|---|---|
| 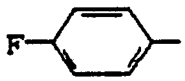 | 1 | H | $-CH(CH_3)_2$ | $-CH_2-CH_2-$ | |
| 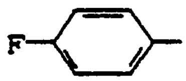 | 0 | H | 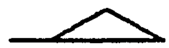 | $-CH_2-CH_2-$ | |
| 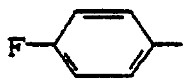 | 1 | H | 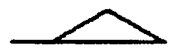 | $-CH_2-CH_2-$ | |
| 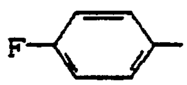 | 0 | H | $-CH_2CH=CH_2$ | $-CH_2-CH_2-$ | |
| 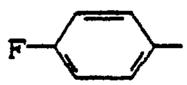 | 1 | H | $-CH_2CH=CH_2$ | $-CH_2-CH_2-$ | |
| 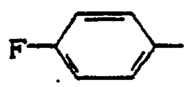 | 0 | H | $-(CH_2)_3-CH_3$ | $-CH_2-CH_2-$ | |
| 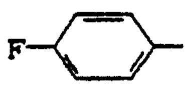 | 1 | H | $-(CH_2)_3-CH_3$ | $-CH_2-CH_2-$ | |
| 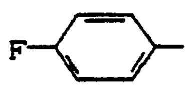 | 0 | H | $-CH_2CH(CH_3)_2$ | $-CH_2-CH_2-$ | |
| 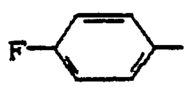 | 1 | H | $-CH_2CH(CH_3)_2$ | $-CH_2-CH_2-$ | |
| 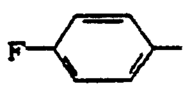 | 0 | H | $-(CH_2)_4-CH_3$ | $-CH_2-CH_2-$ | |
| 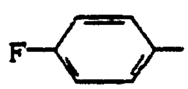 | 1 | H | $-(CH_2)_4-CH_3$ | $-CH_2-CH_2-$ | |

Tabelle: 1 - Fortsetzung

| Ar | n | $R^1$ | $R^2$ | $R^3$ $R^4$ |
|---|---|---|---|---|
| 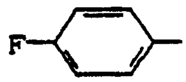 | 0 | H | $-(CH_2)_5-CH_3$ | $-CH_2-CH_2-$ |
| 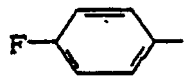 | 1 | H | $-(CH_2)_5-CH_3$ | $-CH_2-CH_2-$ |
| 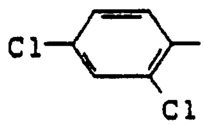 | 0 | H | $-CH_3$ | $-CH_2-CH_2-$ |
| 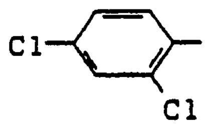 | 1 | H | $-CH_3$ | $-CH_2-CH_2-$ |
| 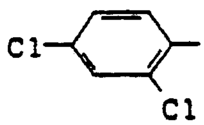 | 0 | H | $-C_2H_5$ | $-CH_2-CH_2-$ |
| 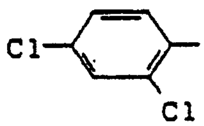 | 1 | H | $-C_2H_5$ | $-CH_2-CH_2-$ |
| 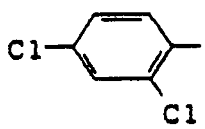 | 0 | H | $-CH_2CH_2CH_3$ | $-CH_2-CH_2-$ |
| 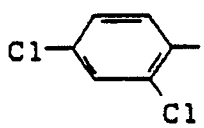 | 1 | H | $-CH_2CH_2CH_3$ | $-CH_2-CH_2-$ |
| 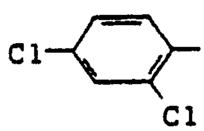 | 0 | H | $-CH(CH_3)_2$ | $-CH_2-CH_2-$ |
| 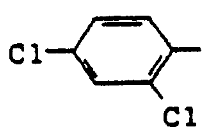 | 1 | H | $-CH(CH_3)_2$ | $-CH_2-CH_2-$ |

T a b e l l e: 1 - Fortsetzung

| Ar | n | $R^1$ | $R^2$ | $R^3$ $R^4$ |
|---|---|---|---|---|
| 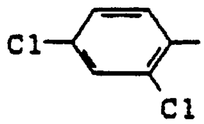 | 0 | H | 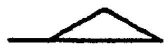 | $-CH_2-CH_2-$ |
| 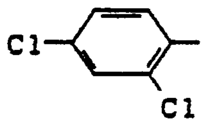 | 1 | H | 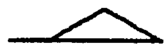 | $-CH_2-CH_2-$ |
| 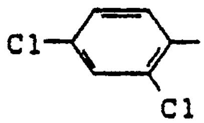 | 0 | H | $-CH_2CH=CH_2$ | $-CH_2-CH_2-$ |
| 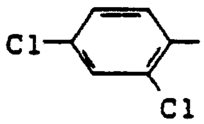 | 1 | H | $-CH_2CH=CH_2$ | $-CH_2-CH_2-$ |
| 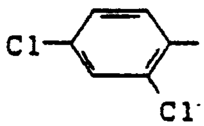 | 0 | H | $-(CH_2)_3-CH_3$ | $-CH_2-CH_2-$ |
| 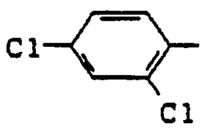 | 1 | H | $-(CH_2)_3-CH_3$ | $-CH_2-CH_2-$ |
| 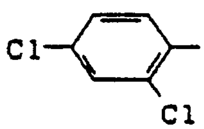 | 0 | H | $-CH_2CH(CH_3)_2$ | $-CH_2-CH_2-$ |
| 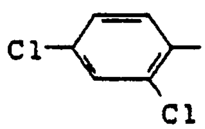 | 1 | H | $-CH_2CH(CH_3)_2$ | $-CH_2-CH_2-$ |
| 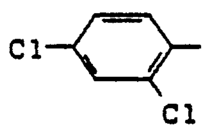 | 0 | H | $-(CH_2)_4-CH_3$ | $-CH_2-CH_2-$ |
| 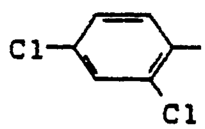 | 1 | H | $-(CH_2)_4-CH_3$ | $-CH_2-CH_2-$ |
| 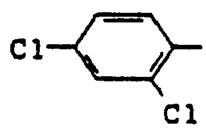 | 0 | H | $-(CH_2)_5-CH_3$ | $-CH_2-CH_2-$ |

T a b e l l e: 1 - Fortsetzung

| Ar | n | $R^1$ | $R^2$ | $R^3$ $R^4$ |
|---|---|---|---|---|
| 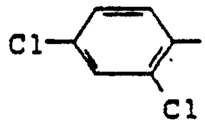 | 1 | H | $-(CH_2)_5-CH_3$ | $-CH_2-CH_2-$ |
| 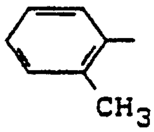 | 0 | H | $-CH_3$ | $-CH_2-CH_2-$ |
| 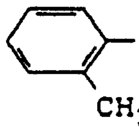 | 1 | H | $-CH_3$ | $-CH_2-CH_2-$ |
| 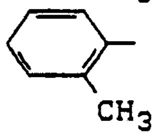 | 0 | H | $-C_2H_5$ | $-CH_2-CH_2-$ |
| 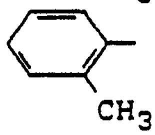 | 1 | H | $-C_2H_5$ | $-CH_2-CH_2-$ |
| 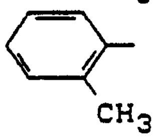 | 0 | H | $-CH_2CH_2CH_3$ | $-CH_2-CH_2-$ |
| 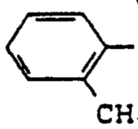 | 1 | H | $-CH_2CH_2CH_3$ | $-CH_2-CH_2-$ |
| 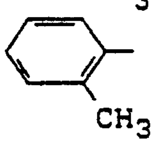 | 0 | H | $-CH(CH_3)_2$ | $-CH_2-CH_2-$ |
| 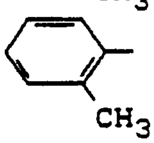 | 1 | H | $-CH(CH_3)_2$ | $-CH_2-CH_2-$ |
| 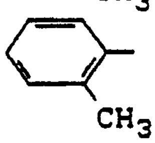 | 0 | H | 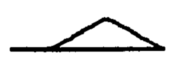 | $-CH_2-CH_2-$ |

T a b e l l e: 1 - Fortsetzung

| Ar | n | $R^1$ | $R^2$ | $R^3$ $R^4$ |
|---|---|---|---|---|
| 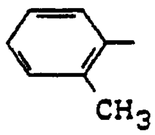 | 1 | H | 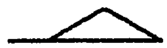 | $-CH_2-CH_2-$ |
| 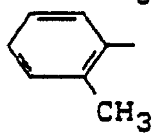 | 0 | H | $-CH_2CH=CH_2$ | $-CH_2-CH_2-$ |
| 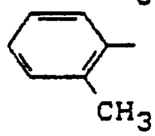 | 1 | H | $-CH_2CH=CH_2$ | $-CH_2-CH_2-$ |
| 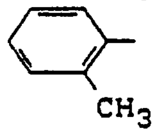 | 0 | H | $-(CH_2)_3-CH_3$ | $-CH_2-CH_2-$ |
| 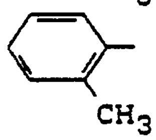 | 1 | H | $-(CH_2)_3-CH_3$ | $-CH_2-CH_2-$ |
| 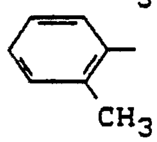 | 0 | H | $-CH_2CH(CH_3)_2$ | $-CH_2-CH_2-$ |
| 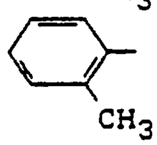 | 1 | H | $-CH_2CH(CH_3)_2$ | $-CH_2-CH_2-$ |
| 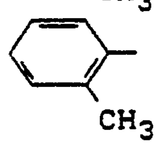 | 0 | H | $-(CH_2)_4-CH_3$ | $-CH_2-CH_2-$ |
| 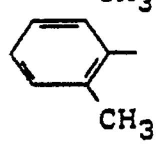 | 1 | H | $-(CH_2)_4-CH_3$ | $-CH_2-CH_2-$ |
| 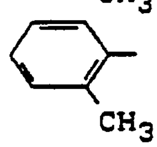 | 0 | H | $-(CH_2)_5-CH_3$ | $-CH_2-CH_2-$ |
| 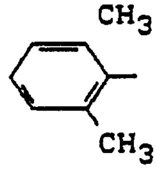 | 1 | H | $-(CH_2)_5-CH_3$ | $-CH_2-CH_2-$ |

T a b e l l e: 1 - Fortsetzung

| Ar | n | $R^1$ | $R^2$ | $R^3$ $R^4$ |
|---|---|---|---|---|
| 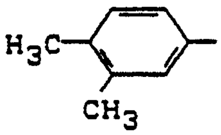 | 0 | H | $-CH_3$ | $-CH_2-CH_2-$ |
| 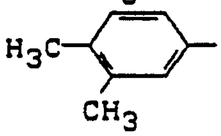 | 1 | H | $-CH_3$ | $-CH_2-CH_2-$ |
| 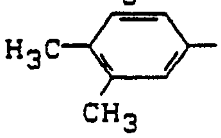 | 0 | H | $-C_2H_5$ | $-CH_2-CH_2-$ |
| 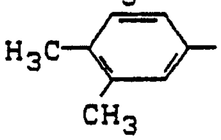 | 1 | H | $-C_2H_5$ | $-CH_2-CH_2-$ |
| 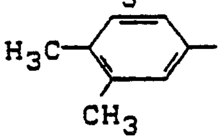 | 0 | H | $-CH_2CH_2CH_3$ | $-CH_2-CH_2-$ |
| 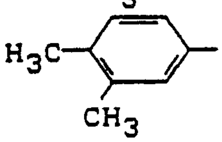 | 1 | H | $-CH_2CH_2CH_3$ | $-CH_2-CH_2-$ |
| 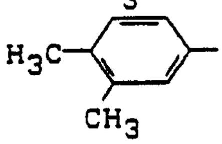 | 0 | H | $-CH(CH_3)_2$ | $-CH_2-CH_2-$ |
| 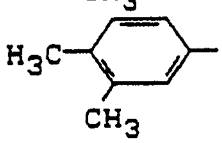 | 1 | H | $-CH(CH_3)_2$ | $-CH_2-CH_2-$ |
| 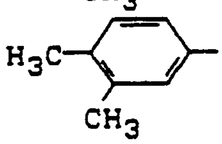 | 0 | H | 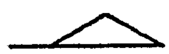 | $-CH_2-CH_2-$ |
| 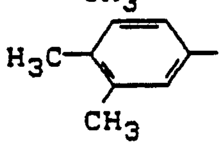 | 1 | H | 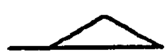 | $-CH_2-CH_2-$ |

T a b e l l e: 1 - Fortsetzung

| Ar | n | $R^1$ | $R^2$ | $R^3$ $R^4$ |
|---|---|---|---|---|
| 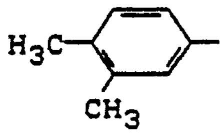 | 0 | H | $-CH_2CH=CH_2$ | $-CH_2-CH_2-$ |
| 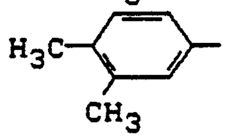 | 1 | H | $-CH_2CH=CH_2$ | $-CH_2-CH_2-$ |
| 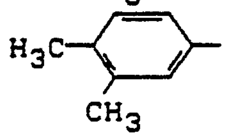 | 0 | H | $-(CH_2)_3-CH_3$ | $-CH_2-CH_2-$ |
| 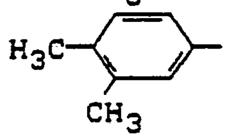 | 1 | H | $-(CH_2)_3-CH_3$ | $-CH_2-CH_2-$ |
| 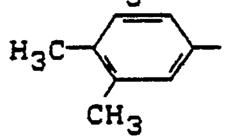 | 0 | H | $-CH_2CH(CH_3)_2$ | $-CH_2-CH_2-$ |
| 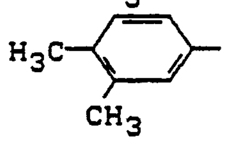 | 1 | H | $-CH_2CH(CH_3)_2$ | $-CH_2-CH_2-$ |
| 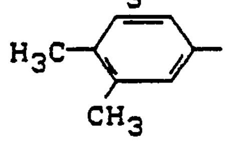 | 0 | H | $-(CH_2)_4-CH_3$ | $-CH_2-CH_2-$ |
| 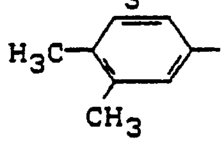 | 1 | H | $-(CH_2)_4-CH_3$ | $-CH_2-CH_2-$ |
| 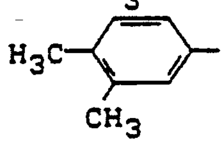 | 0 | H | $-(CH_2)_5-CH_3$ | $-CH_2-CH_2-$ |
| 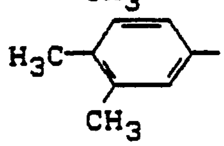 | 1 | H | $-(CH_2)_5-CH_3$ | $-CH_2-CH_2-$ |

T a b e l l e: 1 - Fortsetzung

| Ar | n | $R^1$ | $R^2$ | $R^3$ $R^4$ |
|---|---|---|---|---|
| 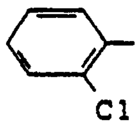 | 0 | H | $-CH_3$ | $-CH_2-CH_2-$ |
| 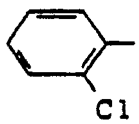 | 1 | H | $-CH_3$ | $-CH_2-CH_2-$ |
| 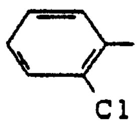 | 0 | H | $-C_2H_5$ | $-CH_2-CH_2-$ |
| 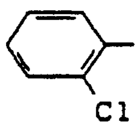 | 1 | H | $-C_2H_5$ | $-CH_2-CH_2-$ |
| 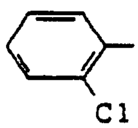 | 0 | H | $-CH_2CH_2CH_3$ | $-CH_2-CH_2-$ |
| 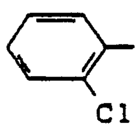 | 1 | H | $-CH_2CH_2CH_3$ | $-CH_2-CH_2-$ |
| 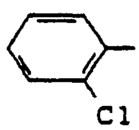 | 0 | H | $-CH(CH_3)_2$ | $-CH_2-CH_2-$ |
| 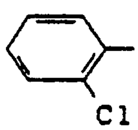 | 1 | H | $-CH(CH_3)_2$ | $-CH_2-CH_2-$ |
| 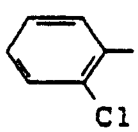 | 0 | H | 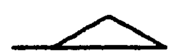 | $-CH_2-CH_2-$ |
| 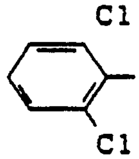 | 1 | H | 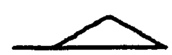 | $-CH_2-CH_2-$ |

Tabelle: 1 - Fortsetzung

| Ar | n | $R^1$ | $R^2$ | $R^3$ $R^4$ |
|---|---|---|---|---|
| 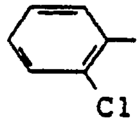 | 0 | H | $-CH_2CH=CH_2$ | $-CH_2-CH_2-$ |
| 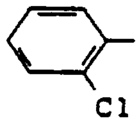 | 1 | H | $-CH_2CH=CH_2$ | $-CH_2-CH_2-$ |
| 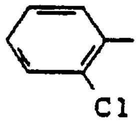 | 0 | H | $-(CH_2)_3-CH_3$ | $-CH_2-CH_2-$ |
| 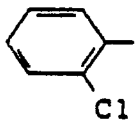 | 1 | H | $-(CH_2)_3-CH_3$ | $-CH_2-CH_2-$ |
| 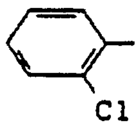 | 0 | H | $-CH_2CH(CH_3)_2$ | $-CH_2-CH_2-$ |
| 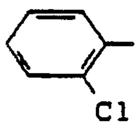 | 1 | H | $-CH_2CH(CH_3)_2$ | $-CH_2-CH_2-$ |
| 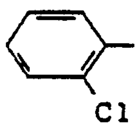 | 0 | H | $-(CH_2)_4-CH_3$ | $-CH_2-CH_2-$ |
| 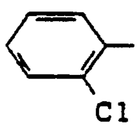 | 1 | H | $-(CH_2)_4-CH_3$ | $-CH_2-CH_2-$ |
| 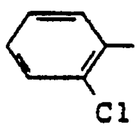 | 0 | H | $-(CH_2)_5-CH_3$ | $-CH_2-CH_2-$ |
| 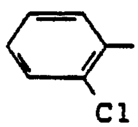 | 1 | H | $-(CH_2)_5-CH_3$ | $-CH_2-CH_2-$ |

T a b e l l e: 1 - Fortsetzung

| Ar | n | $R^1$ | $R^2$ | $R^3$ $R^4$ |
|---|---|---|---|---|
| 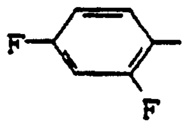 | 0 | H | $-CH_3$ | $-CH_2-CH_2-$ |
| 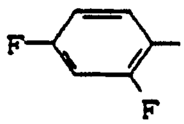 | 1 | H | $-CH_3$ | $-CH_2-CH_2-$ |
| 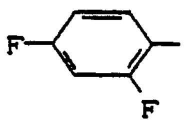 | 0 | H | $-C_2H_5$ | $-CH_2-CH_2-$ |
| 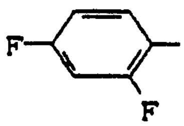 | 1 | H | $-C_2H_5$ | $-CH_2-CH_2-$ |
| 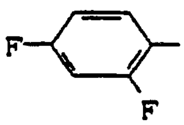 | 0 | H | $-CH_2CH_2CH_3$ | $-CH_2-CH_2-$ |
| 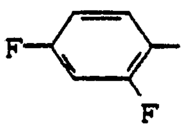 | 1 | H | $-CH_2CH_2CH_3$ | $-CH_2-CH_2-$ |
| 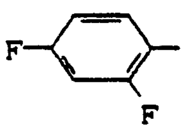 | 0 | H | $-CH(CH_3)_2$ | $-CH_2-CH_2-$ |
| 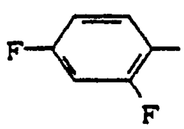 | 1 | H | $-CH(CH_3)_2$ | $-CH_2-CH_2-$ |
| 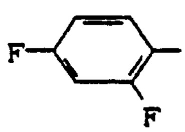 | 0 | H | 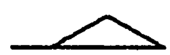 | $-CH_2-CH_2-$ |
| 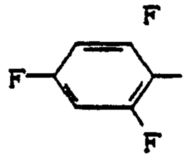 | 1 | H | 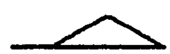 | $-CH_2-CH_2-$ |

Tabelle: 1 - Fortsetzung

| Ar | n | $R^1$ | $R^2$ | $R^3$ $R^4$ |
|---|---|---|---|---|
| 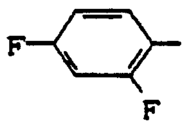 | 0 | H | $-CH_2CH=CH_2$ | $-CH_2-CH_2-$ |
| 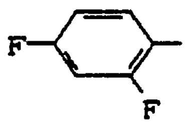 | 1 | H | $-CH_2CH=CH_2$ | $-CH_2-CH_2-$ |
| 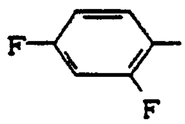 | 0 | H | $-(CH_2)_3-CH_3$ | $-CH_2-CH_2-$ |
| 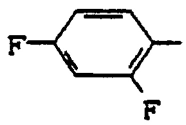 | 1 | H | $-(CH_2)_3-CH_3$ | $-CH_2-CH_2-$ |
| 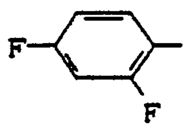 | 0 | H | $-CH_2CH(CH_3)_2$ | $-CH_2-CH_2-$ |
| 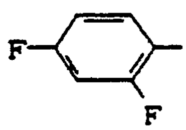 | 1 | H | $-CH_2CH(CH_3)_2$ | $-CH_2-CH_2-$ |
| 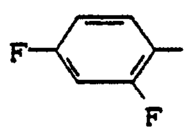 | 0 | H | $-(CH_2)_4-CH_3$ | $-CH_2-CH_2-$ |
| 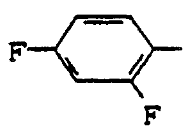 | 1 | H | $-(CH_2)_4-CH_3$ | $-CH_2-CH_2-$ |
| 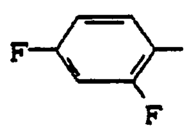 | 0 | H | $-(CH_2)_5-CH_3$ | $-CH_2-CH_2-$ |
| 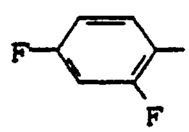 | 1 | H | $-(CH_2)_5-CH_3$ | $-CH_2-CH_2-$ |

Tabelle: 1 - Fortsetzung

| Ar | n | $R^1$ | $R^2$ | $R^3$ $R^4$ |
|---|---|---|---|---|
| 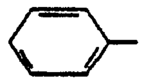 | 0 | H | $-CH_3$ | $-CH_2-CH_2-$ |
| 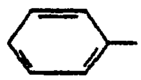 | 1 | H | $-CH_3$ | $-CH_2-CH_2-$ |
| 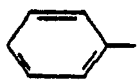 | 0 | H | $-C_2H_5$ | $-CH_2-CH_2-$ |
| 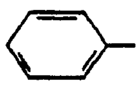 | 1 | H | $-C_2H_5$ | $-CH_2-CH_2-$ |
| 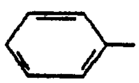 | 0 | H | $-CH_2CH_2CH_3$ | $-CH_2-CH_2-$ |
| 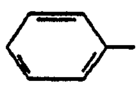 | 1 | H | $-CH_2CH_2CH_3$ | $-CH_2-CH_2-$ |
| 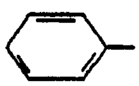 | 0 | H | $-CH(CH_3)_2$ | $-CH_2-CH_2-$ |
| 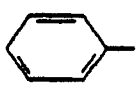 | 1 | H | $-CH(CH_3)_2$ | $-CH_2-CH_2-$ |
| 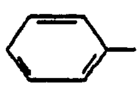 | 0 | H | 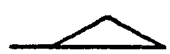 | $-CH_2-CH_2-$ |
| 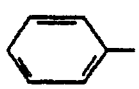 | 1 | H | 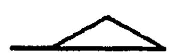 | $-CH_2-CH_2-$ |

T a b e l l e: 1 - Fortsetzung

| Ar | n | $R^1$ | $R^2$ | $R^3$ $R^4$ |
|---|---|---|---|---|
| 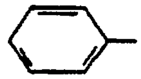 | 0 | H | $-CH_2CH=CH_2$ | $-CH_2-CH_2-$ |
| 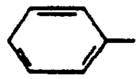 | 1 | H | $-CH_2CH=CH_2$ | $-CH_2-CH_2-$ |
| 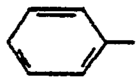 | 0 | H | $-(CH_2)_3-CH_3$ | $-CH_2-CH_2-$ |
| 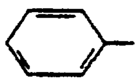 | 1 | H | $-(CH_2)_3-CH_3$ | $-CH_2-CH_2-$ |
| 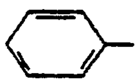 | 0 | H | $-CH_2CH(CH_3)_2$ | $-CH_2-CH_2-$ |
| 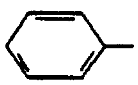 | 1 | H | $-CH_2CH(CH_3)_2$ | $-CH_2-CH_2-$ |
| 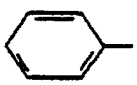 | 0 | H | $-(CH_2)_4-CH_3$ | $-CH_2-CH_2-$ |
| 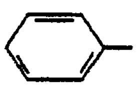 | 1 | H | $-(CH_2)_4-CH_3$ | $-CH_2-CH_2-$ |
| 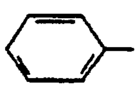 | 0 | H | $-(CH_2)_5-CH_3$ | $-CH_2-CH_2-$ |
| 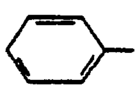 | 1 | H | $-(CH_2)_5-CH_3$ | $-CH_2-CH_2-$ |

T a b e l l e: 1 - Fortsetzung

| Ar | n | $R^1$ | $R^2$ | $R^3$ $R^4$ |
|---|---|---|---|---|
| 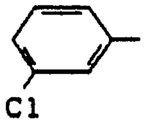 | 0 | H | $-CH_3$ | $-CH_2-CH_2-$ |
| 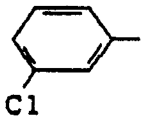 | 1 | H | $-CH_3$ | $-CH_2-CH_2-$ |
| 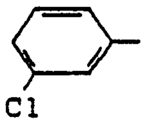 | 0 | H | $-C_2H_5$ | $-CH_2-CH_2-$ |
| 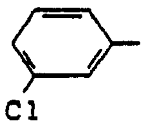 | 1 | H | $-C_2H_5$ | $-CH_2-CH_2-$ |
| 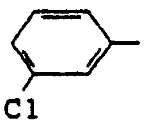 | 0 | H | $-CH_2CH_2CH_3$ | $-CH_2-CH_2-$ |
| 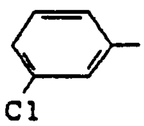 | 1 | H | $-CH_2CH_2CH_3$ | $-CH_2-CH_2-$ |
| 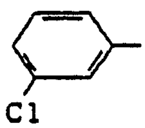 | 0 | H | $-CH(CH_3)_2$ | $-CH_2-CH_2-$ |
| 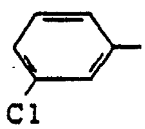 | 1 | H | $-CH(CH_3)_2$ | $-CH_2-CH_2-$ |
| 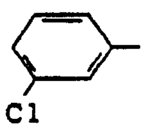 | 0 | H | 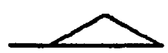 | $-CH_2-CH_2-$ |
| 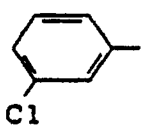 | 1 | H | 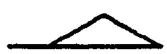 | $-CH_2-CH_2-$ |

T a b e l l e: 1 - Fortsetzung

| Ar | n | $R^1$ | $R^2$ | $R^3$ | $R^4$ |
|---|---|---|---|---|---|
| 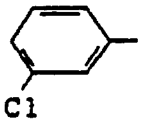 | 0 | H | $-CH_2CH=CH_2$ | $-CH_2-CH_2-$ | |
| 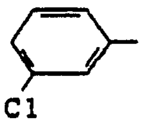 | 1 | H | $-CH_2CH=CH_2$ | $-CH_2-CH_2-$ | |
| 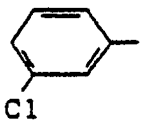 | 0 | H | $-(CH_2)_3-CH_3$ | $-CH_2-CH_2-$ | |
| 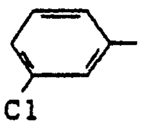 | 1 | H | $-(CH_2)_3-CH_3$ | $-CH_2-CH_2-$ | |
| 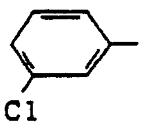 | 0 | H | $-CH_2CH(CH_3)_2$ | $-CH_2-CH_2-$ | |
| 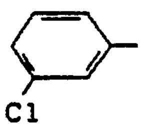 | 1 | H | $-CH_2CH(CH_3)_2$ | $-CH_2-CH_2-$ | |
| 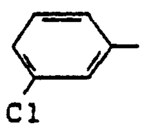 | 0 | H | $-(CH_2)_4-CH_3$ | $-CH_2-CH_2-$ | |
| 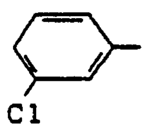 | 1 | H | $-(CH_2)_4-CH_3$ | $-CH_2-CH_2-$ | |
| 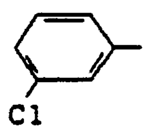 | 0 | H | $-(CH_2)_5-CH_3$ | $-CH_2-CH_2-$ | |
| 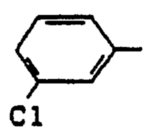 | 1 | H | $-(CH_2)_5-CH_3$ | $-CH_2-CH_2-$ | |

Verwendet man 1-(4-Fluor-benzyl)-1-(1,2,4-triazol-1-yl-methyl-carbonyl)-cyclopropan und Ethylbromid als Ausgangsstoffe, Aluminium-Blättchen als Reaktionshilfsmittel und Allylbromid als weitere Reaktionskomponente, so kann der Verlauf des erfindungsgemäßen Verfahrens (a) durch das folgende Formelschema wiedergegeben werden:

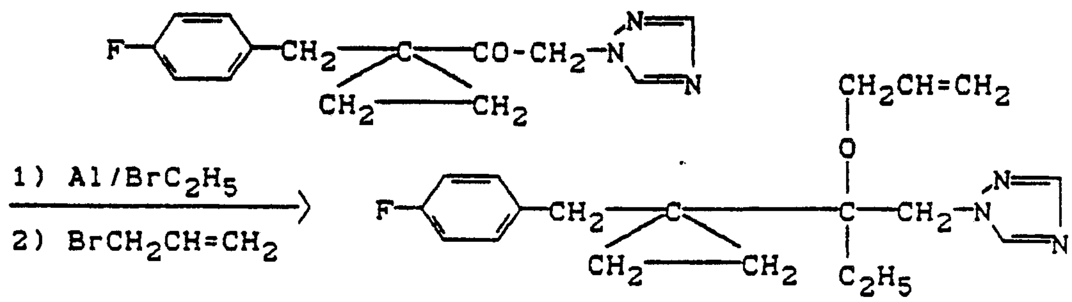


Verwendet man 2-[2-(4-Chlorphenoxy)-1,1-dimethyl-ethyl]-2-methyl-oxiran und 1,2,4-Triazol als Ausgangsstoffe, so kann der Verlauf des erfindungsgemäßen Verfahrens (b) durch das folgende Formelschema wiedergegeben werden:

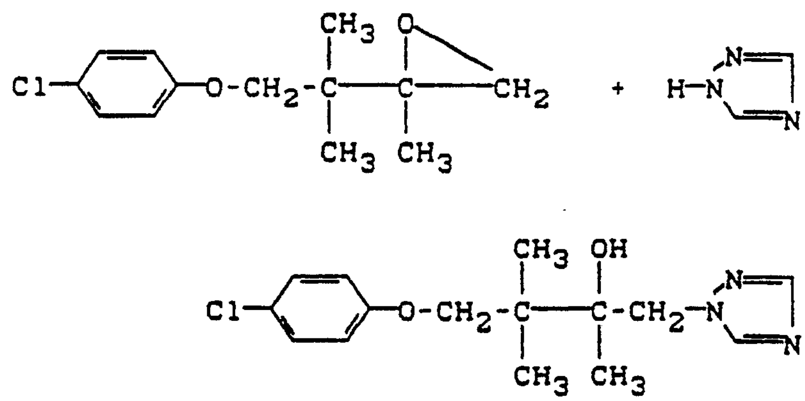

Die beim erfindungsgemäßen Verfahren (a) als Ausgangsstoffe zu verwendenden Triazolylmethylketone sind durch die Formel (II) allgemein definiert.

In Formel (II) haben n, Ar, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Ar, $R^3$ und $R^4$ angegeben wurden.

Beispiele für die Ausgangsstoffe der Formel (II) sind in der nachstehenden Tabelle 2 aufgeführt:

**Tabelle 2:**

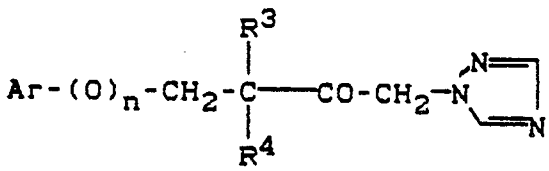 (II)

| Ar | n | $R^3$ | $R^4$ |
|---|---|---|---|
| 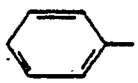 | 0 | $-CH_3$ | $-CH_3$ |
| 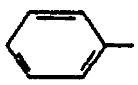 | 1 | $-CH_3$ | $-CH_3$ |
| 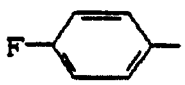 | 0 | $-CH_3$ | $-CH_3$ |

T a b e l l e  2: (Fortsetzung)

| Ar | n | $R^3$ | $R^4$ |
|---|---|---|---|
| 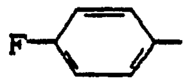 | 1 | $-CH_3$ | $-CH_3$ |
| 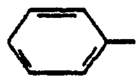 | 0 | $-CH_2-CH_2-$ | |
| 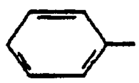 | 1 | $-CH_2-CH_2-$ | |
| 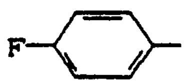 | 0 | $-CH_2-CH_2-$ | |
| 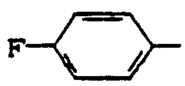 | 1 | $-CH_2-CH_2-$ | |
| 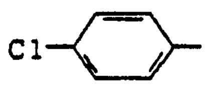 | 0 | $-CH_3$ | $-CH_3$ |
| 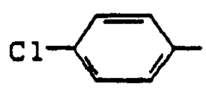 | 1 | $-CH_3$ | $-CH_3$ |
| 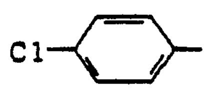 | 0 | $-CH_2-CH_2-$ | |
| 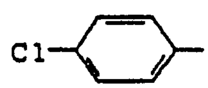 | 1 | $-CH_2-CH_2-$ | |

Tabelle 2: (Fortsetzung)

| Ar | n | $R^3$ | $R^4$ |
|---|---|---|---|
| 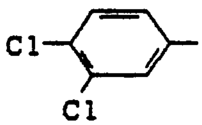 | 0 | $-CH_3$ | $-CH_3$ |
| 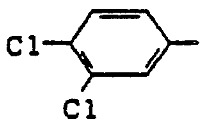 | 1 | $-CH_3$ | $-CH_3$ |
| 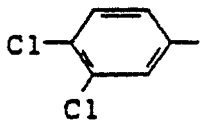 | 0 | $-CH_2-CH_2-$ | |
| 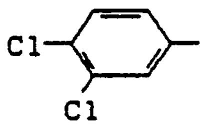 | 1 | $-CH_2-CH_2-$ | |
| 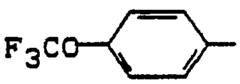 | 0 | $-CH_3$ | $-CH_3$ |
| 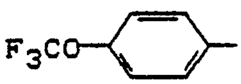 | 1 | $-CH_3$ | $-CH_3$ |
| 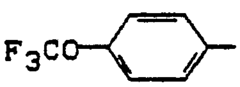 | 0 | $-CH_2-CH_2-$ | |
| 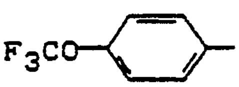 | 1 | $-CH_2-CH_2-$ | |
| 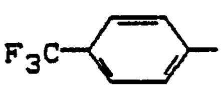 | 0 | $-CH_3$ | $-CH_3$ |

<u>T a b e l l e 2</u>: (Fortsetzung)

| Ar | n | $R^3$ | $R^4$ |
|---|---|---|---|
| 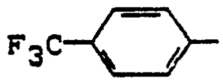 | 1 | $-CH_3$ | $-CH_3$ |
| 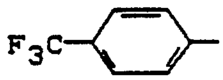 | 0 | $-CH_2-CH_2-$ | |
| 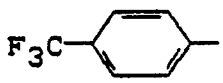 | 1 | $-CH_2-CH_2-$ | |
| 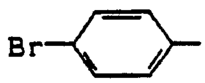 | 0 | $-CH_3$ | $-CH_3$ |
| 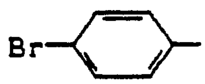 | 1 | $-CH_3$ | $-CH_3$ |
| 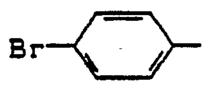 | 0 | $-CH_2-CH_2-$ | |
| 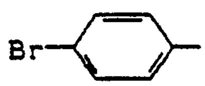 | 1 | $-CH_2-CH_2-$ | |
| 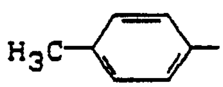 | 0 | $-CH_3$ | $-CH_3$ |
| 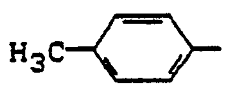 | 1 | $-CH_3$ | $-CH_3$ |
| 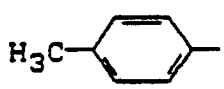 | 0 | $-CH_2-CH_2-$ | |

T a b e l l e  2: (Fortsetzung)

| Ar | n | $R^3$ | $R^4$ |
|---|---|---|---|
| 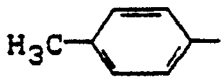 | 1 | $-CH_2-CH_2-$ | |
| 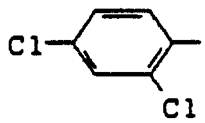 | 0 | $-CH_3$ | $-CH_3$ |
| 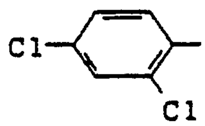 | 1 | $-CH_3$ | $-CH_3$ |
| 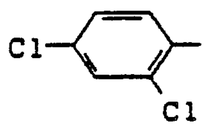 | 0 | $-CH_2-CH_2-$ | |
| 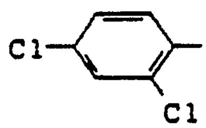 | 1 | $-CH_2-CH_2-$ | |
| 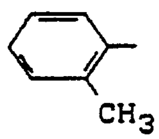 | 0 | $-CH_3$ | $-CH_3$ |
| 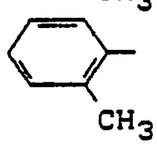 | 1 | $-CH_3$ | $-CH_3$ |
| 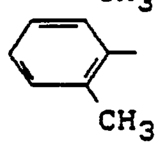 | 0 | $-CH_2-CH_2-$ | |
| 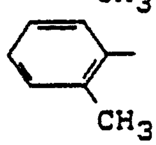 | 1 | $-CH_2-CH_2-$ | |

T a b e l l e 2: (Fortsetzung)

| Ar | n | $R^3$ | $R^4$ |
|---|---|---|---|
| 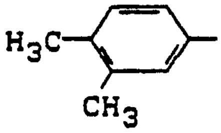 | 0 | $-CH_3$ | $-CH_3$ |
| 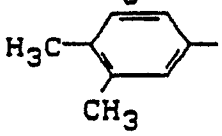 | 1 | $-CH_3$ | $-CH_3$ |
| 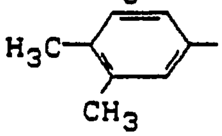 | 0 | $-CH_2-CH_2-$ | |
| 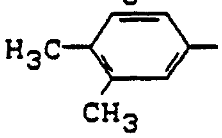 | 1 | $-CH_2-CH_2-$ | |
| 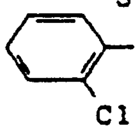 | 0 | $-CH_3$ | $-CH_3$ |
| 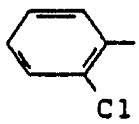 | 1 | $-CH_3$ | $-CH_3$ |
| 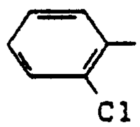 | 0 | $-CH_2-CH_2-$ | |
| 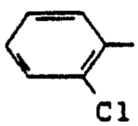 | 1 | $-CH_2-CH_2-$ | |
| 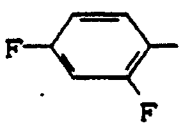 | 0 | $-CH_3$ | $-CH_3$ |
| 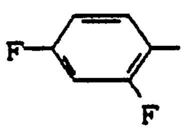 | 1 | $-CH_3$ | $-CH_3$ |

T a b e l l e 2: (Fortsetzung)

| Ar | n | $R^3$ | $R^4$ |
|---|---|---|---|
| 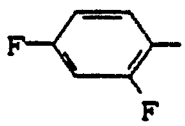 | 0 | $-CH_2-CH_2-$ | |
| 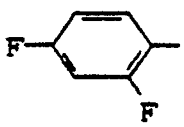 | 1 | $-CH_2-CH_2-$ | |
| 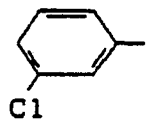 | 0 | $-CH_3$ | $-CH_3$ |
| 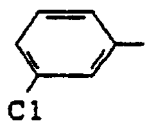 | 1 | $-CH_3$ | $-CH_3$ |
| 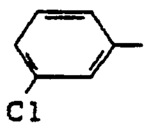 | 0 | $-CH_2-CH_2-$ | |
| 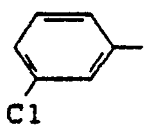 | 1 | $-CH_2-CH_2-$ | |
| 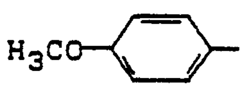 | 0 | $-CH_3$ | $-CH_3$ |
| 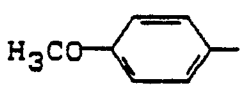 | 1 | $-CH_3$ | $-CH_3$ |
| 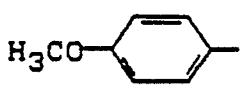 | 0 | $-CH_2-CH_2-$ | |
| 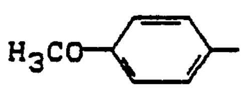 | 1 | $-CH_2-CH_2-$ | |

Tabelle 2: (Fortsetzung)

| Ar | n | $R^3$ | $R^4$ |
|---|---|---|---|
| 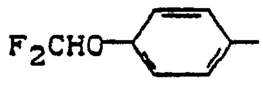 | 0 | $-CH_3$ | $-CH_3$ |
| 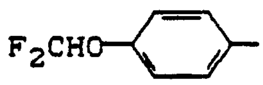 | 1 | $-CH_3$ | $-CH_3$ |
| 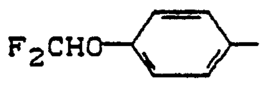 | 0 | $-CH_2-CH_2-$ | |
| 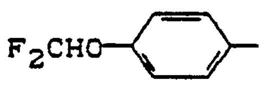 | 1 | $-CH_2-CH_2-$ | |

Die Triazolylmethylketone der Formel (II) sind bekannt oder können nach an sich bekannten Verfahren hergestellt werden (vgl. EP-OS 0 054 865).

Die beim erfindungsgemäßen Verfahren (a) weiter als Ausgangsstoffe zu verwendenden Halogenverbindungen sind durch die Formel (III) allgemein definiert.

In Formel (III) hat $R^2$ vorzugsweise bzw. insbesondere diejenige Bedeutung, die bereits oben im Zusammenhang mit der Beschreibung der erfindungsgemäßen Verbindungen der Formel (I) vorzugsweise bzw, als insbesondere bevorzugt für $R^2$ angegeben wurde, und X steht vorzugsweise für Chlor, Brom oder lod.

Als Beispiele für die Ausgangsstoffe der Formel (III) seien genannt:
Methyl-, Ethyl-, Propyl-, Isopropyl-, Butyl-, Isobutyl-, sec-Butyl, Pentyl-, Hexyl-, Cyclopropyl- und Allylchlorid, -bromid und -iodid.

Die halogenverbindungen der Formel (III) sind bekannte organische Synthesechemikalien.

Die beim erfindungsgemäßen Verfahren (a) als weitere Reaktionskomponenten zu verwenden Verbindungen sind durch die Formel (IV) allgemein definiert.

In Formel (IV) hat $R^1$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$ angegeben wurde, und Y steht vorzugsweise für Chlor, Brom oder lod.

Als Beispiele für die Ausgangsstoffe der Formel (IV) seien genannt:
Hydrogenchlorid, Hydrogenbromid, Hydrogeniodid, Methyl-, Ethyl-, Propyl-, Isopropyl-, Butyl-, Isobutyl-, sec-Butyl-, Pentyl-, Cyclopropyl, und Allyl-bromid, -chlorid und -iodid.

Die Verbindungen der Formel (IV) sind bekannte organische Synthesechemikalien.

Das erfindungsgemäße Verfahren (a) wird in Gegenwart von Aluminium und gegebenenfalls in Gegenwart von Stoffen, welche zur Aktivierung von Aluminium geeignet sind, durchgeführt. Solche Stoffe sind insbesondere Halogene oder Metallhalogenide, beispielsweise Brom, lod, Aluminium-chlorid oder -bromid, Quecksilber(II)-chlorid, Kupfer(II)-chlorid und Silber(I)-chlorid.

Das erfindungsgemäße Verfahren (a) wird vorzugsweise unter Verwendung von Verdünnungsmitteln durchgeführt. Als Verdünnungsmittel kommen dabei praktisch alle inerten organischen Lösungsmittel in Frage. Vorzugsweise verwendvar sind aliphatische und aromatische Kohlenwasserstoffe wie Pentan, Hexan, Heptan, Cyclohexan, Petrolether, Benzin, Ligroin, Benzol, Toluol und Xylol, Ether wie Diethyl- und Dibutylether, Glykoldimethylether und Diglykoldimethylether, Tetrahydrofuran und Dioxan, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen -50 °C und +150 °C, vorzugsweise bei Temperaturen zwischen -30 °C und +100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man auf 1 Mol Triazolylmethylketon der Formel (II) im allgemeinen zwischen 1 und 3 Mol, vorzugsweise zwischen 1,5 und 2,5 Mol, Halogenverbindung der Formel (III) und im allgemeinen zwischen 1 und 3 Mol, vorzugsweise zwischen 1,5 und 2,5 Mol, Aluminium gegebenenfalls im Gemisch mit Aluminiumaktivator sowie zwischen 1 und 20 Mol, vorzugsweise zwischen 1 und 10 Mol, einer Verbindung der Formel (IV) ein.

Im allgemeinen wird zunächst das Aluminium gegebenenfalls mit einem oder mehreren zur Aktivierung von Aluminium geeigneten Stoffen, vorzugsweise in einem Ultraschallbad und unter Verwendung eines Verdünnungsmittels, behandelt, dann mit einer Halogenverbindung der Formel (III) zur Reaktion gebracht und anschließend mit einem Triazolylmethylketon der Formel (II) und schließlich mit einer Verbindung der Formel (IV) umgesetzt.

Für den Fall, daß es sich bei den Verbindungen der Formel (IV) um Hydrogenhalogenide handelt, kann deren Umsetzung auch quasi im Rahmen der Aufarbeitung unter Einsatz von deren wässrigen Lösungen, wie z.B. Salzsäure, durchgeführt werden.

Die Aufarbeitung kann nach üblichen Methoden durchgeführt werden. Beispielsweise wird mit Wasser bzw. mit einer wässrigen Säure verdünnt, gegebenenfalls die Hauptmenge des organischen Lösungsmittels abdestilliert, gegebenenfalls mit einer Base, wie z.B. Ammoniak, auf einen schwach alkalischen pH-Wert eingestellt, gegebenenfalls mit Wasser verdünnt, mit einem mit Wasser praktisch nicht mischbaren organischen Lösungsmittel, wie z.B. Essigsäureethylester, versetzt und filtriert oder abgesaugt. Die organische Phase des Filtrats wird abgetrennt, mit Wasser gewaschen, mit einem Trockenmittel, wie z.B. Natriumsulfat, behandelt und filtriert. Vom Filtrat wird das Lösungsmittel im Wasserstrahlvakuum abdestilliert und das als Rückstand erhaltene Rohprodukt gegebenenfalls nach üblichen Methoden, beispielsweise durch Säulenchromatographie, gereinigt.

Die beim erfindungsgemäßen Verfahren (b) als Ausgangsstoffe zu verwendenden Oxirane sind durch die Formel (V) allgemein definiert.

In Formel (V) haben n, Ar, $R^2$ $R^3$ und $R^4$ vorzugsweise bzw. insbesondere diejenigen Bedeutungen, die bereits oben im Zusammenhang mit der Beschriebung der erfindungsgemäßen Verbindungen der Formel (I) vorzugsweise bzw. als insbesondere bevorzugt für n, Ar, $R^2$ $R^3$ und $R^4$ angegeben wurden.

Beispiele für die Ausgangsstoffe der Formel (V) sind in der nachfolgenden Tabelle 3 aufgeführt:

Tabelle 3:

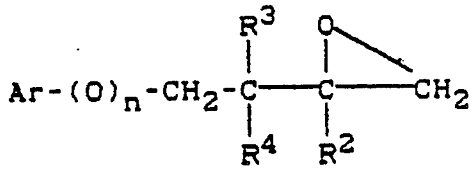

| Ar | n | $R^2$ | $R^3$ | $R^4$ |
|---|---|---|---|---|
| 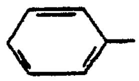 | 0 | $-CH_3$ | $-CH_3$ | $-CH_3$ |
| 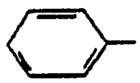 | 1 | $-CH_3$ | $-CH_3$ | $-CH_3$ |
| 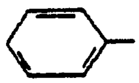 | 0 | $-CH_3$ | $-CH_2-CH_2-$ | |

Tabelle 3: (Fortsetzung)

| Ar | n | $R^2$ | $R^3$ | $R^4$ |
|---|---|---|---|---|
| 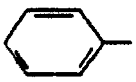 | 1 | $-CH_3$ | $-CH_2-CH_2-$ | |
| 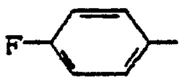 | 0 | $-CH_3$ | $-CH_3$ | |
| 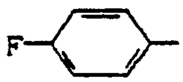 | 1 | $-CH_3$ | $-CH_3$ | |
| 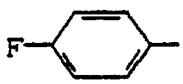 | 0 | $-CH_3$ | $-CH_2-CH_2-$ | |
| 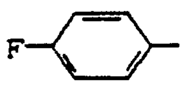 | 1 | $-CH_3$ | $-CH_2-CH_2-$ | |
| 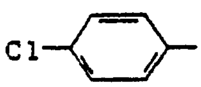 | 0 | $-CH_3$ | $-CH_3$ | $-CH_3$ |
| 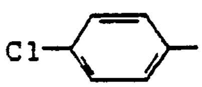 | 1 | $-CH_3$ | $-CH_3$ | $-CH_3$ |
| 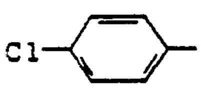 | 0 | $C_2H_5$ | $-CH_3$ | $-CH_3$ |
| 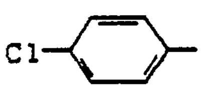 | 1 | $-C_2H_5$ | $-CH_3$ | $-CH_3$ |
| 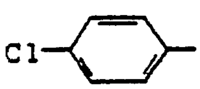 | 0 | $-CH_2CH_2CH_3$ | $-CH_3$ | $-CH_3$ |

T a b e l l e 3: (Fortsetzung)

| Ar | n | $R^2$ | $R^3$ | $R^4$ |
|---|---|---|---|---|
| 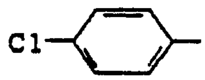 | 1 | $-CH_2CH_2CH_3$ | $-CH_3$ | $-CH_3$ |
| 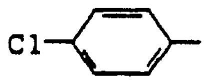 | 0 | $-CH(CH_3)_2$ | $-CH_3$ | $-CH_3$ |
| 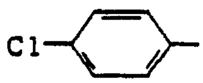 | 1 | $-CH(CH_3)_2$ | $-CH_3$ | $-CH_3$ |
| 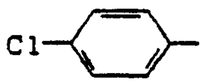 | 0 | 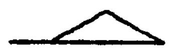 | $-CH_3$ | $-CH_3$ |
| 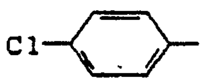 | 1 | 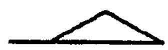 | $-CH_3$ | $-CH_3$ |
| 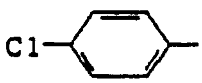 | 0 | $-CH_2CH=CH_2$ | $-CH_3$ | $-CH_3$ |
| 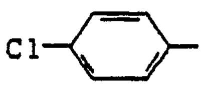 | 1 | $-CH_2CH=CH_2$ | $-CH_3$ | $-CH_3$ |
| 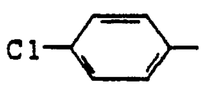 | 0 | $-(CH_2)_3-CH_3$ | $-CH_3$ | $-CH_3$ |
| 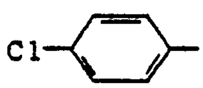 | 1 | $-(CH_2)_3-CH_3$ | $-CH_3$ | $-CH_3$ |
| 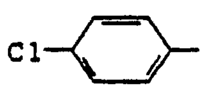 | 0 | $-CH_2CH(CH_3)_2$ | $-CH_3$ | $-CH_3$ |
| 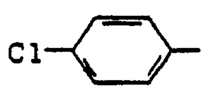 | 1 | $-CH_2CH(CH_3)_2$ | $-CH_3$ | $-CH_3$ |

Tabelle 3: (Fortsetzung)

| Ar | n | $R^2$ | $R^3$ | $R^4$ |
|---|---|---|---|---|
| 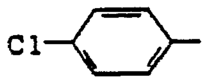 | 0 | $-(CH_2)_4-CH_3$ | $-CH_3$ | $-CH_3$ |
| 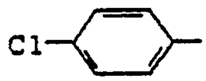 | 1 | $-(CH_2)_4-CH_3$ | $-CH_3$ | $-CH_3$ |
| 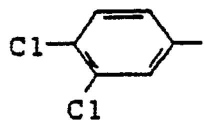 | 0 | $-CH_3$ | $-CH_3$ | $-CH_3$ |
| 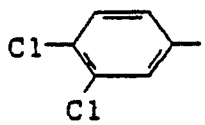 | 1 | $-CH_3$ | $-CH_3$ | $-CH_3$ |
| 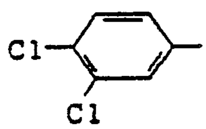 | 0 | $C_2H_5$ | $-CH_3$ | $-CH_3$ |
| 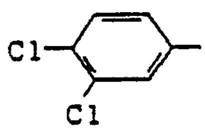 | 1 | $-C_2H_5$ | $-CH_3$ | $-CH_3$ |
| 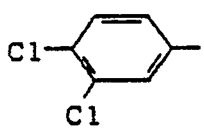 | 0 | $-CH_2CH_2CH_3$ | $-CH_3$ | $-CH_3$ |
| 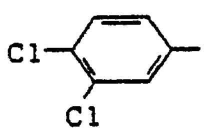 | 1 | $-CH_2CH_2CH_3$ | $-CH_3$ | $-CH_3$ |
| 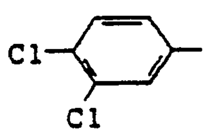 | 0 | $-CH(CH_3)_2$ | $-CH_3$ | $-CH_3$ |
| 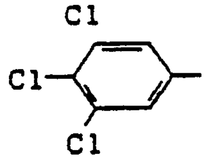 | 1 | $-CH(CH_3)_2$ | $-CH_3$ | $-CH_3$ |

T a b e l l e 3: (Fortsetzung)

| Ar | n | $R^2$ | $R^3$ | $R^4$ |
|---|---|---|---|---|
| 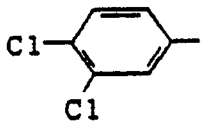 | 0 | 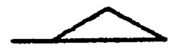 | $-CH_3$ | $-CH_3$ |
| 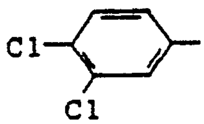 | 1 | 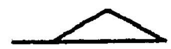 | $-CH_3$ | $-CH_3$ |
| 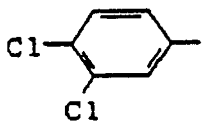 | 0 | $-CH_2CH=CH_2$ | $-CH_3$ | $-CH_3$ |
| 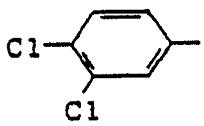 | 1 | $-CH_2CH=CH_2$ | $-CH_3$ | $-CH_3$ |
| 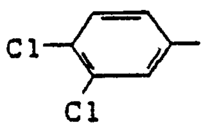 | 0 | $-(CH_2)_3-CH_3$ | $-CH_3$ | $-CH_3$ |
| 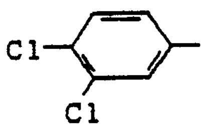 | 1 | $-(CH_2)_3-CH_3$ | $-CH_3$ | $-CH_3$ |
| 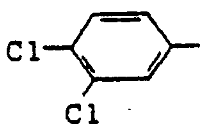 | 0 | $-CH_2CH(CH_3)_2$ | $-CH_3$ | $-CH_3$ |
| 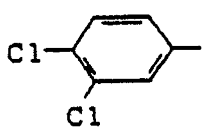 | 1 | $-CH_2CH(CH_3)_2$ | $-CH_3$ | $-CH_3$ |
| 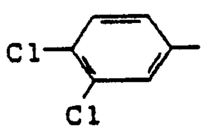 | 0 | $-(CH_2)_4-CH_3$ | $-CH_3$ | $-CH_3$ |
| 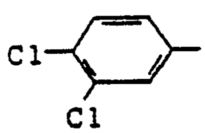 | 1 | $-(CH_2)_4-CH_3$ | $-CH_3$ | $-CH_3$ |
| 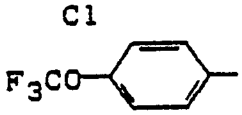 | 0 | $-CH_3$ | $-CH_3$ | $-CH_3$ |
| 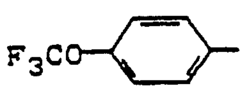 | 1 | $-CH_3$ | $-CH_3$ | $-CH_3$ |

Tabelle 3: (Fortsetzung)

| Ar | n | $R^2$ | $R^3$ | $R^4$ |
|---|---|---|---|---|
| 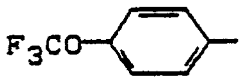 | 0 | $C_2H_5$ | $-CH_3$ | $-CH_3$ |
| 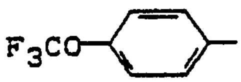 | 1 | $-C_2H_5$ | $-CH_3$ | $-CH_3$ |
| 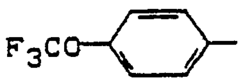 | 0 | $-CH_2CH_2CH_3$ | $-CH_3$ | $-CH_3$ |
| 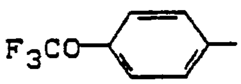 | 1 | $-CH_2CH_2CH_3$ | $-CH_3$ | $-CH_3$ |
| 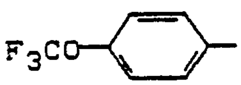 | 0 | $-CH(CH_3)_2$ | $-CH_3$ | $-CH_3$ |
| 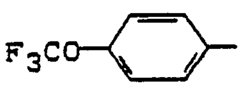 | 1 | $-CH(CH_3)_2$ | $-CH_3$ | $-CH_3$ |
| 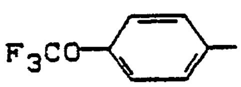 | 0 | 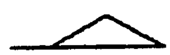 | $-CH_3$ | $-CH_3$ |
| 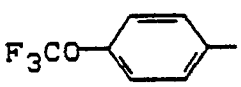 | 1 | 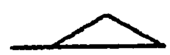 | $-CH_3$ | $-CH_3$ |
| 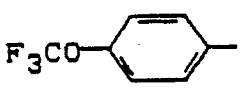 | 0 | $-CH_2CH{=}CH_2$ | $-CH_3$ | $-CH_3$ |
| 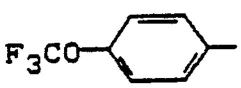 | 1 | $-CH_2CH{=}CH_2$ | $-CH_3$ | $-CH_3$ |

Tabelle 3: (Fortsetzung)

| Ar | n | $R^2$ | $R^3$ | $R^4$ |
|---|---|---|---|---|
| 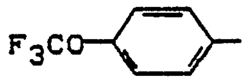 | 0 | $-(CH_2)_3-CH_3$ | $-CH_3$ | $-CH_3$ |
| 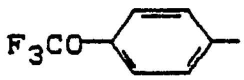 | 1 | $-(CH_2)_3-CH_3$ | $-CH_3$ | $-CH_3$ |
| 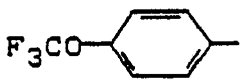 | 0 | $-CH_2CH(CH_3)_2$ | $-CH_3$ | $-CH_3$ |
| 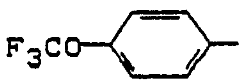 | 1 | $-CH_2CH(CH_3)_2$ | $-CH_3$ | $-CH_3$ |
| 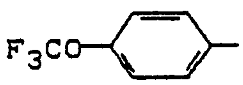 | 0 | $-(CH_2)_4-CH_3$ | $-CH_3$ | $-CH_3$ |
| 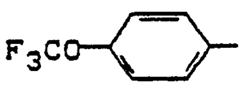 | 1 | $-(CH_2)_4-CH_3$ | $-CH_3$ | $-CH_3$ |
| 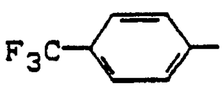 | 0 | $-CH_3$ | $-CH_3$ | $-CH_3$ |
| 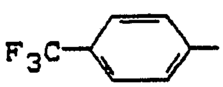 | 1 | $-CH_3$ | $-CH_3$ | $-CH_3$ |
| 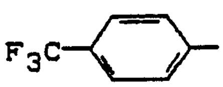 | 0 | $-C_2H_5$ | $-CH_3$ | $-CH_3$ |
| 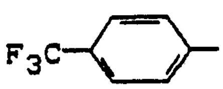 | 1 | $-C_2H_5$ | $-CH_3$ | $-CH_3$ |

T a b e l l e 3: (Fortsetzung)

| Ar | n | $R^2$ | $R^3$ | $R^4$ |
|---|---|---|---|---|
| 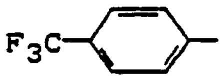 | 0 | $-CH_2CH_2CH_3$ | $-CH_3$ | $-CH_3$ |
| 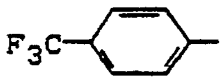 | 1 | $-CH_2CH_2CH_3$ | $-CH_3$ | $-CH_3$ |
| 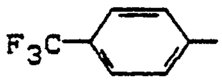 | 0 | $-CH(CH_3)_2$ | $-CH_3$ | $-CH_3$ |
| 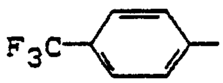 | 1 | $-CH(CH_3)_2$ | $-CH_3$ | $-CH_3$ |
| 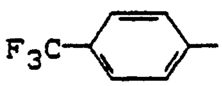 | 0 | 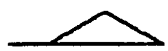 | $-CH_3$ | $-CH_3$ |
| 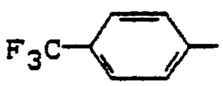 | 1 | 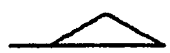 | $-CH_3$ | $-CH_3$ |
| 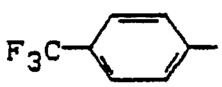 | 0 | $-CH_2CH=CH_2$ | $-CH_3$ | $-CH_3$ |
| 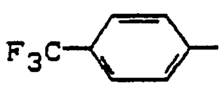 | 1 | $-CH_2CH=CH_2$ | $-CH_3$ | $-CH_3$ |
| 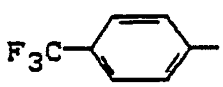 | 0 | $-(CH_2)_3-CH_3$ | $-CH_3$ | $-CH_3$ |
| 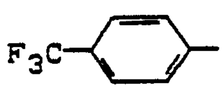 | 1 | $-(CH_2)_3-CH_3$ | $-CH_3$ | $-CH_3$ |

T a b e l l e 3: (Fortsetzung)

| Ar | n | $R^2$ | $R^3$ | $R^4$ |
| --- | --- | --- | --- | --- |
| 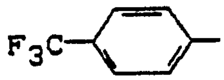 | 0 | $-CH_2CH(CH_3)_2$ | $-CH_3$ | $-CH_3$ |
| 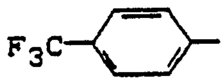 | 1 | $-CH_2CH(CH_3)_2$ | $-CH_3$ | $-CH_3$ |
| 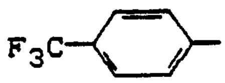 | 0 | $-(CH_2)_4-CH_3$ | $-CH_3$ | $-CH_3$ |
| 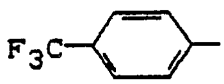 | 1 | $-(CH_2)_4-CH_3$ | $-CH_3$ | $-CH_3$ |
| 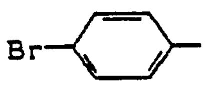 | 0 | $-CH_3$ | $-CH_3$ | $-CH_3$ |
| 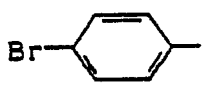 | 1 | $-CH_3$ | $-CH_3$ | $-CH_3$ |
| 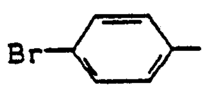 | 0 | $-C_2H_5$ | $-CH_3$ | $-CH_3$ |
| 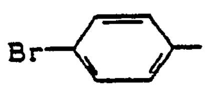 | 1 | $-C_2H_5$ | $-CH_3$ | $-CH_3$ |
| 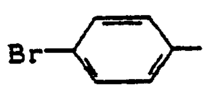 | 0 | $-CH_2CH_2CH_3$ | $-CH_3$ | $-CH_3$ |
| 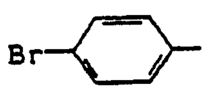 | 1 | $-CH_2CH_2CH_3$ | $-CH_3$ | $-CH_3$ |

Tabelle 3: (Fortsetzung)

| Ar | n | $R^2$ | $R^3$ | $R^4$ |
|---|---|---|---|---|
| 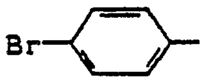 | 0 | $-CH(CH_3)_2$ | $-CH_3$ | $-CH_3$ |
| 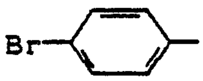 | 1 | $-CH(CH_3)_2$ | $-CH_3$ | $-CH_3$ |
| 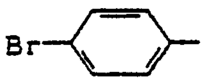 | 0 | 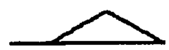 | $-CH_3$ | $-CH_3$ |
| 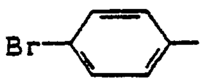 | 1 | 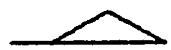 | $-CH_3$ | $-CH_3$ |
| 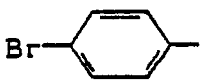 | 0 | $-CH_2CH=CH_2$ | $-CH_3$ | $-CH_3$ |
| 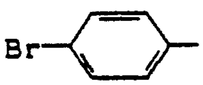 | 1 | $-CH_2CH=CH_2$ | $-CH_3$ | $-CH_3$ |
| 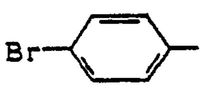 | 0 | $-(CH_2)_3-CH_3$ | $-CH_3$ | $-CH_3$ |
| 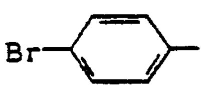 | 1 | $-(CH_2)_3-CH_3$ | $-CH_3$ | $-CH_3$ |
| 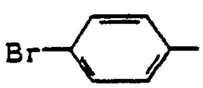 | 0 | $-CH_2CH(CH_3)_2$ | $-CH_3$ | $-CH_3$ |
| 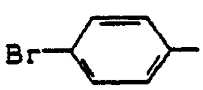 | 1 | $-CH_2CH(CH_3)_2$ | $-CH_3$ | $-CH_3$ |

Tabelle 3: (Fortsetzung)

| Ar | n | $R^2$ | $R^3$ | $R^4$ |
|---|---|---|---|---|
| 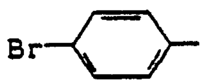 | 0 | $-(CH_2)_4-CH_3$ | $-CH_3$ | $-CH_3$ |
| 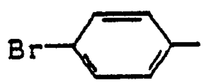 | 1 | $-(CH_2)_4-CH_3$ | $-CH_3$ | $-CH_3$ |
| 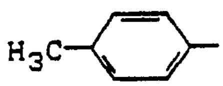 | 0 | $-CH_3$ | $-CH_3$ | $-CH_3$ |
| 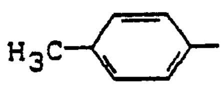 | 1 | $-CH_3$ | $-CH_3$ | $-CH_3$ |
| 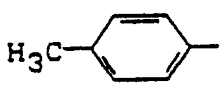 | 0 | $-C_2H_5$ | $-CH_3$ | $-CH_3$ |
| 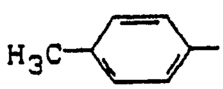 | 1 | $-C_2H_5$ | $-CH_3$ | $-CH_3$ |
| 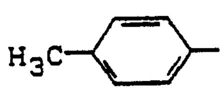 | 0 | $-CH_2CH_2CH_3$ | $-CH_3$ | $-CH_3$ |
| 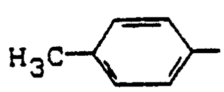 | 1 | $-CH_2CH_2CH_3$ | $-CH_3$ | $-CH_3$ |
| 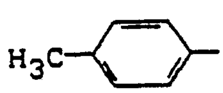 | 0 | $-CH(CH_3)_2$ | $-CH_3$ | $-CH_3$ |
| 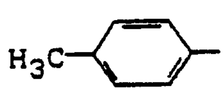 | 1 | $-CH(CH_3)_2$ | $-CH_3$ | $-CH_3$ |

T a b e l l e 3: (Fortsetzung)

| Ar | n | $R^2$ | $R^3$ | $R^4$ |
|---|---|---|---|---|
| 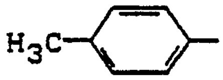 | 0 | 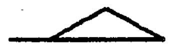 | $-CH_3$ | $-CH_3$ |
| 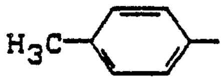 | 1 | 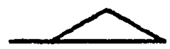 | $-CH_3$ | $-CH_3$ |
| 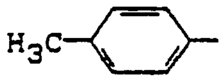 | 0 | $-CH_2CH=CH_2$ | $-CH_3$ | $-CH_3$ |
| 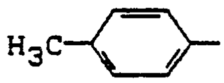 | 1 | $-CH_2CH=CH_2$ | $-CH_3$ | $-CH_3$ |
| 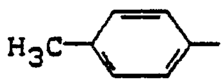 | 0 | $-(CH_2)_3-CH_3$ | $-CH_3$ | $-CH_3$ |
| 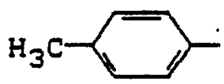 | 1 | $-(CH_2)_3-CH_3$ | $-CH_3$ | $-CH_3$ |
| 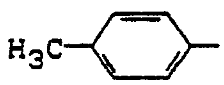 | 0 | $-CH_2CH(CH_3)_2$ | $-CH_3$ | $-CH_3$ |
| 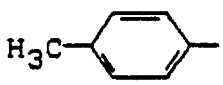 | 1 | $-CH_2CH(CH_3)_2$ | $-CH_3$ | $-CH_3$ |
| 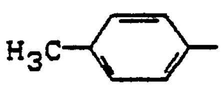 | 0 | $-(CH_2)_4-CH_3$ | $-CH_3$ | $-CH_3$ |
| 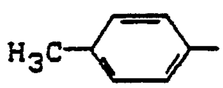 | 1 | $-(CH_2)_4-CH_3$ | $-CH_3$ | $-CH_3$ |

T a b e l l e 3: (Fortsetzung)

| Ar | n | $R^2$ | $R^3$ | $R^4$ |
|---|---|---|---|---|
| 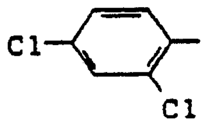 | 0 | $-CH_3$ | $-CH_3$ | $-CH_3$ |
| 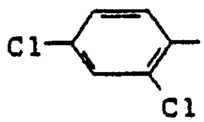 | 1 | $-CH_3$ | $-CH_3$ | $-CH_3$ |
| 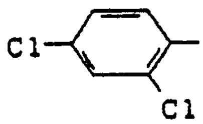 | 0 | $-C_2H_5$ | $-CH_3$ | $-CH_3$ |
| 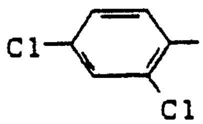 | 1 | $-C_2H_5$ | $-CH_3$ | $-CH_3$ |
| 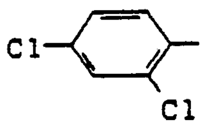 | 0 | $-CH_2CH_2CH_3$ | $-CH_3$ | $-CH_3$ |
| 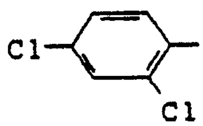 | 1 | $-CH_2CH_2CH_3$ | $-CH_3$ | $-CH_3$ |
| 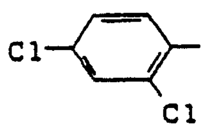 | 0 | $-CH(CH_3)_2$ | $-CH_3$ | $-CH_3$ |
| 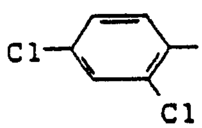 | 1 | $-CH(CH_3)_2$ | $-CH_3$ | $-CH_3$ |
| 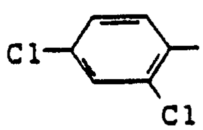 | 0 | 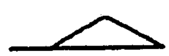 | $-CH_3$ | $-CH_3$ |
| 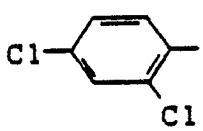 | 1 | 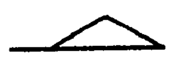 | $-CH_3$ | $-CH_3$ |

T a b e l l e 3: (Fortsetzung)

| Ar | n | $R^2$ | $R^3$ | $R^4$ |
|---|---|---|---|---|
| 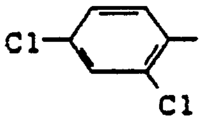 | 0 | $-CH_2CH{=}CH_2$ | $-CH_3$ | $-CH_3$ |
| 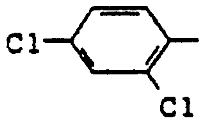 | 1 | $-CH_2CH{=}CH_2$ | $-CH_3$ | $-CH_3$ |
| 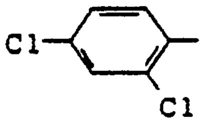 | 0 | $-(CH_2)_3-CH_3$ | $-CH_3$ | $-CH_3$ |
| 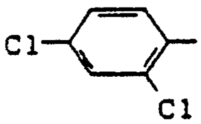 | 1 | $-(CH_2)_3-CH_3$ | $-CH_3$ | $-CH_3$ |
| 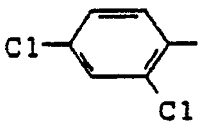 | 0 | $-CH_2CH(CH_3)_2$ | $-CH_3$ | $-CH_3$ |
| 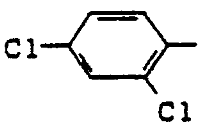 | 1 | $-CH_2CH(CH_3)_2$ | $-CH_3$ | $-CH_3$ |
| 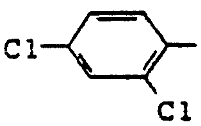 | 0 | $-(CH_2)_4-CH_3$ | $-CH_3$ | $-CH_3$ |
| 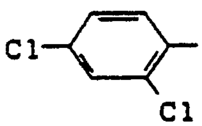 | 1 | $-(CH_2)_4-CH_3$ | $-CH_3$ | $-CH_3$ |
| 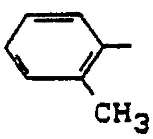 | 0 | $-CH_3$ | $-CH_3$ | $-CH_3$ |
| 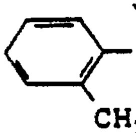 | 1 | $-CH_3$ | $-CH_3$ | $-CH_3$ |

Tabelle 3: (Fortsetzung)

| Ar | n | $R^2$ | $R^3$ | $R^4$ |
|---|---|---|---|---|
| 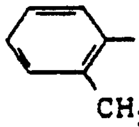 | 0 | $-C_2H_5$ | $-CH_3$ | $-CH_3$ |
| 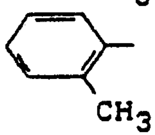 | 1 | $-C_2H_5$ | $-CH_3$ | $-CH_3$ |
| 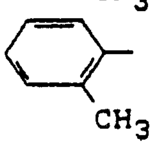 | 0 | $-CH_2CH_2CH_3$ | $-CH_3$ | $-CH_3$ |
| 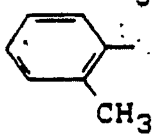 | 1 | $-CH_2CH_2CH_3$ | $-CH_3$ | $-CH_3$ |
| 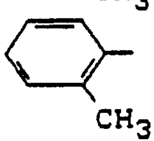 | 0 | $-CH(CH_3)_2$ | $-CH_3$ | $-CH_3$ |
| 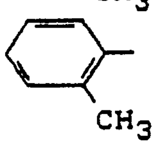 | 1 | $-CH(CH_3)_2$ | $-CH_3$ | $-CH_3$ |
| 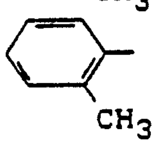 | 0 | 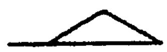 | $-CH_3$ | $-CH_3$ |
| 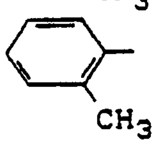 | 1 | 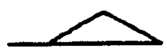 | $-CH_3$ | $-CH_3$ |
| 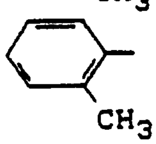 | 0 | $-CH_2CH=CH_2$ | $-CH_3$ | $-CH_3$ |
| 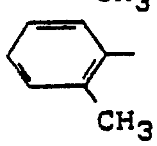 | 1 | $-CH_2CH=CH_2$ | $-CH_3$ | $-CH_3$ |

T a b e l l e 3: (Fortsetzung)

| Ar | n | $R^2$ | $R^3$ | $R^4$ |
|---|---|---|---|---|
| 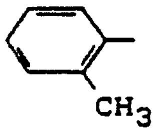 | 0 | $-(CH_2)_3-CH_3$ | $-CH_3$ | $-CH_3$ |
| 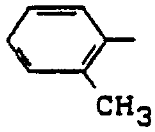 | 1 | $-(CH_2)_3-CH_3$ | $-CH_3$ | $-CH_3$ |
| 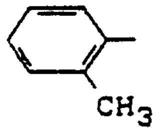 | 0 | $-CH_2CH(CH_3)_2$ | $-CH_3$ | $-CH_3$ |
| 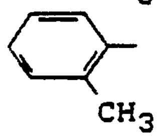 | 1 | $-CH_2CH(CH_3)_2$ | $-CH_3$ | $-CH_3$ |
| 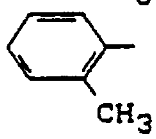 | 0 | $-(CH_2)_4-CH_3$ | $-CH_3$ | $-CH_3$ |
| 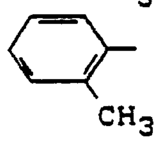 | 1 | $-(CH_2)_4-CH_3$ | $-CH_3$ | $-CH_3$ |
| 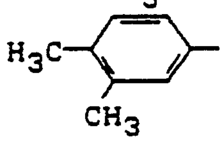 | 0 | $-CH_3$ | $-CH_3$ | $-CH_3$ |
| 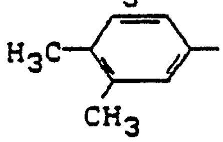 | 1 | $-CH_3$ | $-CH_3$ | $-CH_3$ |
| 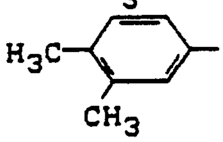 | 0 | $-C_2H_5$ | $-CH_3$ | $-CH_3$ |
| 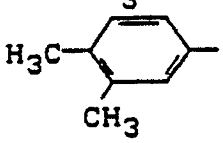 | 1 | $-C_2H_5$ | $-CH_3$ | $-CH_3$ |

Tabelle 3: (Fortsetzung)

| Ar | n | $R^2$ | $R^3$ | $R^4$ |
|---|---|---|---|---|
| 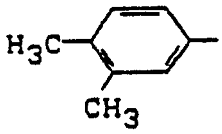 | 0 | $-CH_2CH_2CH_3$ | $-CH_3$ | $-CH_3$ |
| 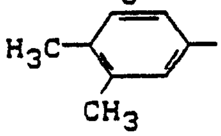 | 1 | $-CH_2CH_2CH_3$ | $-CH_3$ | $-CH_3$ |
| 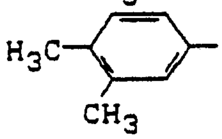 | 0 | $-CH(CH_3)_2$ | $-CH_3$ | $-CH_3$ |
| 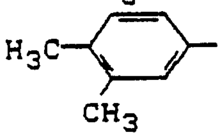 | 1 | $-CH(CH_3)_2$ | $-CH_3$ | $-CH_3$ |
| 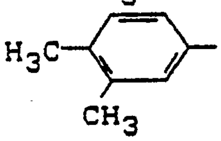 | 0 | 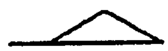 | $-CH_3$ | $-CH_3$ |
| 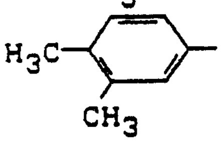 | 1 | 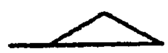 | $-CH_3$ | $-CH_3$ |
| 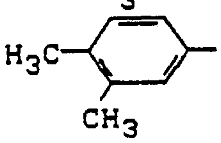 | 0 | $-CH_2CH=CH_2$ | $-CH_3$ | $-CH_3$ |
| 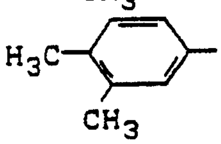 | 1 | $-CH_2CH=CH_2$ | $-CH_3$ | $-CH_3$ |
| 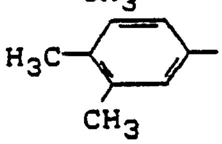 | 0 | $-(CH_2)_3-CH_3$ | $-CH_3$ | $-CH_3$ |
| 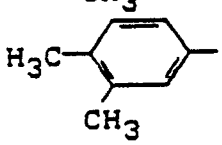 | 1 | $-(CH_2)_3-CH_3$ | $-CH_3$ | $-CH_3$ |

T a b e l l e 3: (Fortsetzung)

| Ar | n | $R^2$ | $R^3$ | $R^4$ |
|---|---|---|---|---|
| 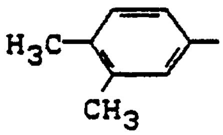 | 0 | $-CH_2CH(CH_3)_2$ | $-CH_3$ | $-CH_3$ |
| 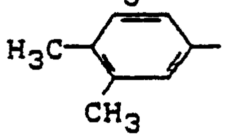 | 1 | $-CH_2CH(CH_3)_2$ | $-CH_3$ | $-CH_3$ |
| 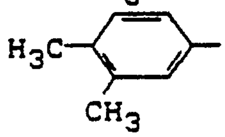 | 0 | $-(CH_2)_4-CH_3$ | $-CH_3$ | $-CH_3$ |
| 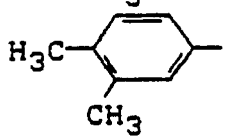 | 1 | $-(CH_2)_4-CH_3$ | $-CH_3$ | $-CH_3$ |
| 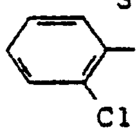 | 0 | $-CH_3$ | $-CH_3$ | $-CH_3$ |
| 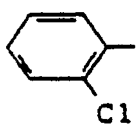 | 1 | $-CH_3$ | $-CH_3$ | $-CH_3$ |
| 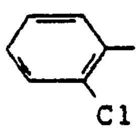 | 0 | $-C_2H_5$ | $-CH_3$ | $-CH_3$ |
| 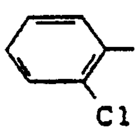 | 1 | $-C_2H_5$ | $-CH_3$ | $-CH_3$ |
| 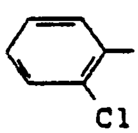 | 0 | $-CH_2CH_2CH_3$ | $-CH_3$ | $-CH_3$ |
| 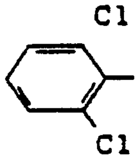 | 1 | $-CH_2CH_2CH_3$ | $-CH_3$ | $-CH_3$ |

<u>Tabelle 3</u>: (Fortsetzung)

| Ar | n | $R^2$ | $R^3$ | $R^4$ |
|---|---|---|---|---|
| 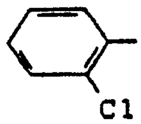 | 0 | $-CH(CH_3)_2$ | $-CH_3$ | $-CH_3$ |
| 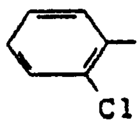 | 1 | $-CH(CH_3)_2$ | $-CH_3$ | $-CH_3$ |
| 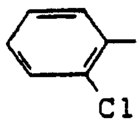 | 0 | 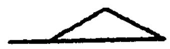 | $-CH_3$ | $-CH_3$ |
| 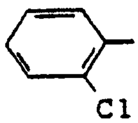 | 1 | 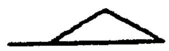 | $-CH_3$ | $-CH_3$ |
| 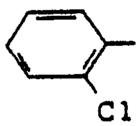 | 0 | $-CH_2CH=CH_2$ | $-CH_3$ | $-CH_3$ |
| 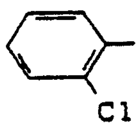 | 1 | $-CH_2CH=CH_2$ | $-CH_3$ | $-CH_3$ |
| 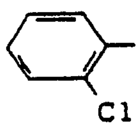 | 0 | $-(CH_2)_3-CH_3$ | $-CH_3$ | $-CH_3$ |
| 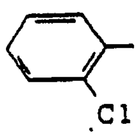 | 1 | $-(CH_2)_3-CH_3$ | $-CH_3$ | $-CH_3$ |
| 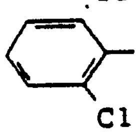 | 0 | $-CH_2CH(CH_3)_2$ | $-CH_3$ | $-CH_3$ |
| 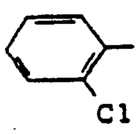 | 1 | $-CH_2CH(CH_3)_2$ | $-CH_3$ | $-CH_3$ |

T a b e l l e 3: (Fortsetzung)

| Ar | n | $R^2$ | $R^3$ | $R^4$ |
|---|---|---|---|---|
| 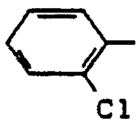 | 0 | $-(CH_2)_4-CH_3$ | $-CH_3$ | $-CH_3$ |
| 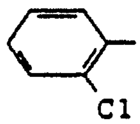 | 1 | $-(CH_2)_4-CH_3$ | $-CH_3$ | $-CH_3$ |
| 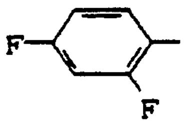 | 0 | $-CH_3$ | $-CH_3$ | $-CH_3$ |
| 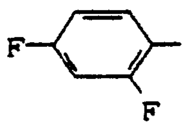 | 1 | $-CH_3$ | $-CH_3$ | $-CH_3$ |
| 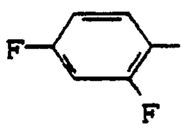 | 0 | $-C_2H_5$ | $-CH_3$ | $-CH_3$ |
| 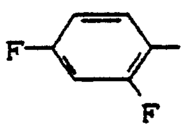 | 1 | $-C_2H_5$ | $-CH_3$ | $-CH_3$ |
| 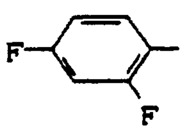 | 0 | $-CH_2CH_2CH_3$ | $-CH_3$ | $-CH_3$ |
| 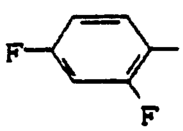 | 1 | $-CH_2CH_2CH_3$ | $-CH_3$ | $-CH_3$ |
| 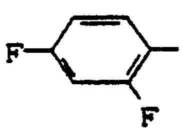 | 0 | $-CH(CH_3)_2$ | $-CH_3$ | $-CH_3$ |
| 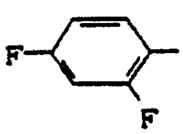 | 1 | $-CH(CH_3)_2$ | $-CH_3$ | $-CH_3$ |

Tabelle 3: (Fortsetzung)

| Ar | n | $R^2$ | $R^3$ | $R^4$ |
|---|---|---|---|---|
| 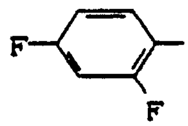 | 0 | 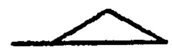 | $-CH_3$ | $-CH_3$ |
| 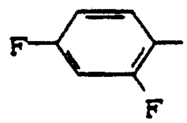 | 1 | 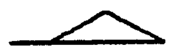 | $-CH_3$ | $-CH_3$ |
| 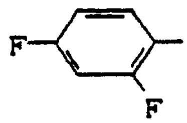 | 0 | $-CH_2CH=CH_2$ | $-CH_3$ | $-CH_3$ |
| 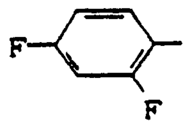 | 1 | $-CH_2CH=CH_2$ | $-CH_3$ | $-CH_3$ |
| 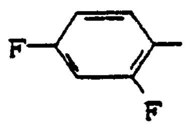 | 0 | $-(CH_2)_3-CH_3$ | $-CH_3$ | $-CH_3$ |
| 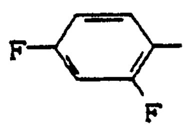 | 1 | $-(CH_2)_3-CH_3$ | $-CH_3$ | $-CH_3$ |
| 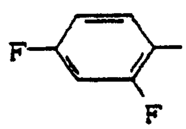 | 0 | $-CH_2CH(CH_3)_2$ | $-CH_3$ | $-CH_3$ |
| 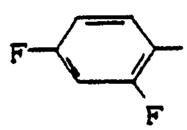 | 1 | $-CH_2CH(CH_3)_2$ | $-CH_3$ | $-CH_3$ |
| 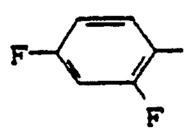 | 0 | $-(CH_2)_4-CH_3$ | $-CH_3$ | $-CH_3$ |
| 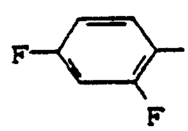 | 1 | $-(CH_2)_4-CH_3$ | $-CH_3$ | $-CH_3$ |

T a b e l l e 3: (Fortsetzung)

| Ar | n | $R^2$ | $R^3$ | $R^4$ |
|---|---|---|---|---|
| 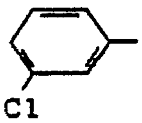 | 0 | $-CH_3$ | $-CH_3$ | $-CH_3$ |
| 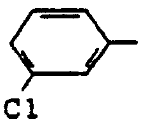 | 1 | $-CH_3$ | $-CH_3$ | $-CH_3$ |
| 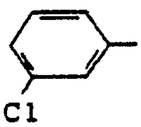 | 0 | $-C_2H_5$ | $-CH_3$ | $-CH_3$ |
| 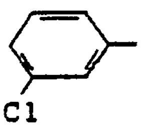 | 1 | $-C_2H_5$ | $-CH_3$ | $-CH_3$ |
| 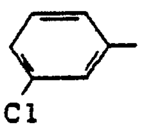 | 0 | $-CH_2CH_2CH_3$ | $-CH_3$ | $-CH_3$ |
| 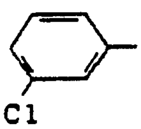 | 1 | $-CH_2CH_2CH_3$ | $-CH_3$ | $-CH_3$ |
| 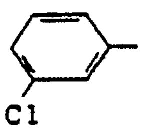 | 0 | $-CH(CH_3)_2$ | $-CH_3$ | $-CH_3$ |
| 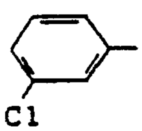 | 1 | $-CH(CH_3)_2$ | $-CH_3$ | $-CH_3$ |
| 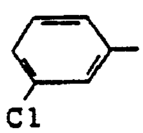 | 0 | 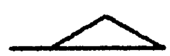 | $-CH_3$ | $-CH_3$ |
| 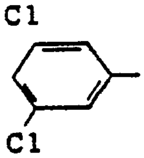 | 1 | 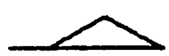 | $-CH_3$ | $-CH_3$ |

Tabelle 3: (Fortsetzung)

| Ar | n | $R^2$ | $R^3$ | $R^4$ |
|---|---|---|---|---|
| 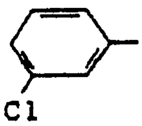 | 0 | $-CH_2CH=CH_2$ | $-CH_3$ | $-CH_3$ |
| 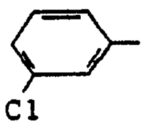 | 1 | $-CH_2CH=CH_2$ | $-CH_3$ | $-CH_3$ |
| 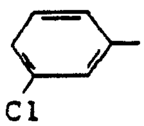 | 0 | $-(CH_2)_3-CH_3$ | $-CH_3$ | $-CH_3$ |
| 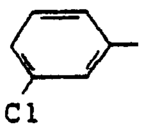 | 1 | $-(CH_2)_3-CH_3$ | $-CH_3$ | $-CH_3$ |
| 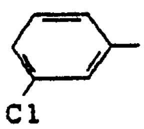 | 0 | $-CH_2CH(CH_3)_2$ | $-CH_3$ | $-CH_3$ |
| 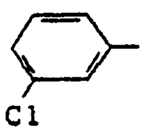 | 1 | $-CH_2CH(CH_3)_2$ | $-CH_3$ | $-CH_3$ |
| 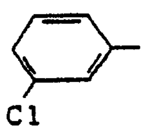 | 0 | $-(CH_2)_4-CH_3$ | $-CH_3$ | $-CH_3$ |
| 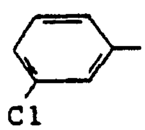 | 1 | $-(CH_2)_4-CH_3$ | $-CH_3$ | $-CH_3$ |
| 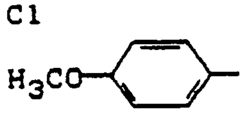 | 0 | $-CH_3$ | $-CH_3$ | $-CH_3$ |
| 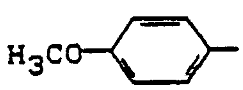 | 1 | $-CH_3$ | $-CH_3$ | $-CH_3$ |

Tabelle 3: (Fortsetzung)

| Ar | n | $R^2$ | $R^3$ | $R^4$ |
|---|---|---|---|---|
| 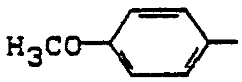 | 0 | $-C_2H_5$ | $-CH_3$ | $-CH_3$ |
| 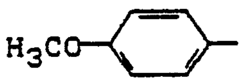 | 1 | $-C_2H_5$ | $-CH_3$ | $-CH_3$ |
| 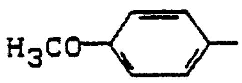 | 0 | $-CH_2CH_2CH_3$ | $-CH_3$ | $-CH_3$ |
| 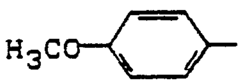 | 1 | $-CH_2CH_2CH_3$ | $-CH_3$ | $-CH_3$ |
| 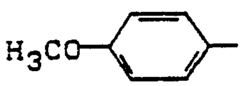 | 0 | $-CH(CH_3)_2$ | $-CH_3$ | $-CH_3$ |
| 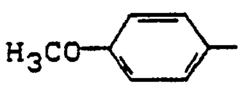 | 1 | $-CH(CH_3)_2$ | $-CH_3$ | $-CH_3$ |
| 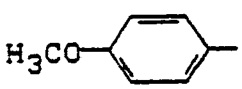 | 0 | 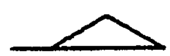 | $-CH_3$ | $-CH_3$ |
| 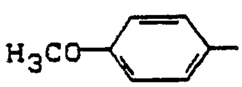 | 1 | 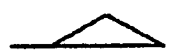 | $-CH_3$ | $-CH_3$ |
| 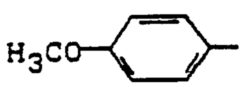 | 0 | $-CH_2CH=CH_2$ | $-CH_3$ | $-CH_3$ |
| 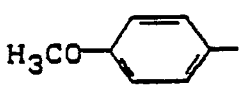 | 1 | $-CH_2CH=CH_2$ | $-CH_3$ | $-CH_3$ |

Tabelle 3: (Fortsetzung)

| Ar | n | $R^2$ | $R^3$ | $R^4$ |
|---|---|---|---|---|
| 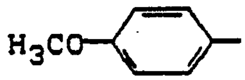 | 0 | $-(CH_2)_3-CH_3$ | $-CH_3$ | $-CH_3$ |
| 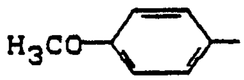 | 1 | $-(CH_2)_3-CH_3$ | $-CH_3$ | $-CH_3$ |
| 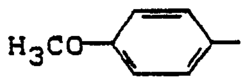 | 0 | $-CH_2CH(CH_3)_2$ | $-CH_3$ | $-CH_3$ |
| 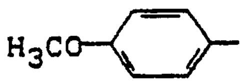 | 1 | $-CH_2CH(CH_3)_2$ | $-CH_3$ | $-CH_3$ |
| 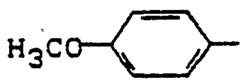 | 0 | $-(CH_2)_4-CH_3$ | $-CH_3$ | $-CH_3$ |
| 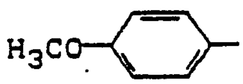 | 1 | $-(CH_2)_4-CH_3$ | $-CH_3$ | $-CH_3$ |
| 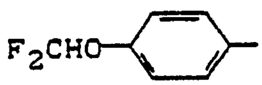 | 0 | $-CH_3$ | $-CH_3$ | $-CH_3$ |
| 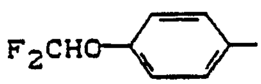 | 1 | $-CH_3$ | $-CH_3$ | $-CH_3$ |
| 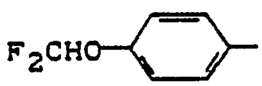 | 0 | $-C_2H_5$ | $-CH_3$ | $-CH_3$ |
| 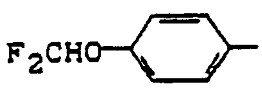 | 1 | $-C_2H_5$ | $-CH_3$ | $-CH_3$ |

T a b e l l e 3: (Fortsetzung)

| Ar | n | $R^2$ | $R^3$ | $R^4$ |
|---|---|---|---|---|
| 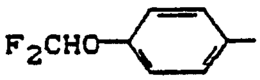 | 0 | $-CH_2CH_2CH_3$ | $-CH_3$ | $-CH_3$ |
| 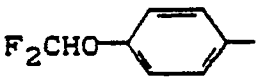 | 1 | $-CH_2CH_2CH_3$ | $-CH_3$ | $-CH_3$ |
| 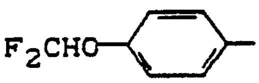 | 0 | $-CH(CH_3)_2$ | $-CH_3$ | $-CH_3$ |
| 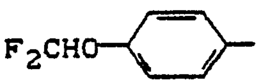 | 1 | $-CH(CH_3)_2$ | $-CH_3$ | $-CH_3$ |
| 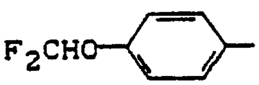 | 0 | 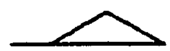 | $-CH_3$ | $-CH_3$ |
| 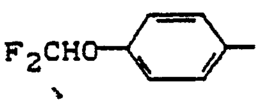 | 1 | 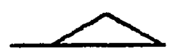 | $-CH_3$ | $-CH_3$ |
| 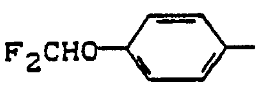 | 0 | $-CH_2CH=CH_2$ | $-CH_3$ | $-CH_3$ |
| 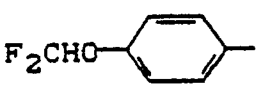 | 1 | $-CH_2CH=CH_2$ | $-CH_3$ | $-CH_3$ |
| 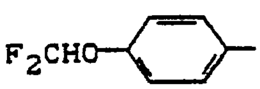 | 0 | $-(CH_2)_3-CH_3$ | $-CH_3$ | $-CH_3$ |
| 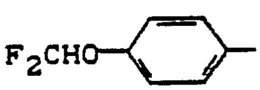 | 1 | $-(CH_2)_3-CH_3$ | $-CH_3$ | $-CH_3$ |

**Tabelle 3: (Fortsetzung)**

| Ar | n | $R^2$ | $R^3$ | $R^4$ |
|---|---|---|---|---|
| 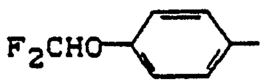 | 0 | $-CH_2CH(CH_3)_2$ | $-CH_3$ | $-CH_3$ |
| 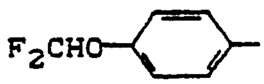 | 1 | $-CH_2CH(CH_3)_2$ | $-CH_3$ | $-CH_3$ |
| 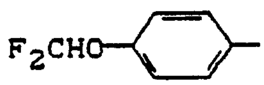 | 0 | $-(CH_2)_4-CH_3$ | $-CH_3$ | $-CH_3$ |
| 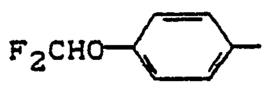 | 1 | $-(CH_2)_4-CH_3$ | $-CH_3$ | $-CH_3$ |

Die Oxirane der Formel (V) sind bisher nicht bekannt. Sie lassen sich herstellen, indem man c) Ketone der Formel

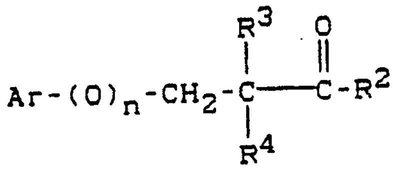 (VII)

in welcher n, Ar, $R^2$, $R^3$ und $R^4$ die oben angegebenen Bedeutungen haben, entweder

α) mit Dimethyloxosulfonium-methylid der Formel

$$\overset{\delta^+\delta^-}{(CH_3)_2SOCH_2} \qquad (VIII)$$

in Gegenwart eines Verdünnungsmittels, wie beispielsweise Dimethylsulfoxid, bei Temperaturen zwischen 20° C und 80° C umsetzt, oder

β) mit Trimethylsulfonium-methylsulfat der Formel

$[(CH_3)_3S^{\oplus}]\ CH_3SO_4^{\ominus}$ (IX)

in Gegenwart eines inerten organischen Lösungsmittels, wie z.B. Acetonitril, und in Gegenwart einer Base, wie z.B. Natriummethylat, bei Temperaturen zwischen 0° C bis 60° C, vorzugsweise bei Raumtemperatur, umsetzt, oder

γ) mit Methyl-triphenylphosphoniumbromid der Formel

$H_3C-\overset{\oplus}{P}\ (C_6H_5)_3\ \overset{\ominus}{Br}$ (X)

in Gegenwart einer Base, wie z. B. Kaliumtert-butylat und gegebenenfalls in Gegenwart eines Verdünnungsmittels, wie z. B. Diethylether, Benzol oder Toluol, bei Temperaturen zwischen 20° C und 150° C umsetzt (vergl. hierzu Syn. Comm. 1985, 15, 855) und die hierbei erhaltenen Alkene der Formel

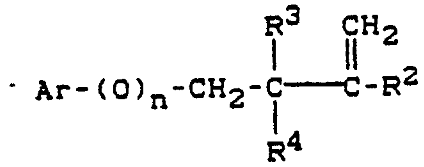 (XI)

in welcher
n, Ar, $R^2$, $R^3$ und $R^4$ die oben angegebenen Bedeutungen haben,
mit Oxigenierungsmitteln, wie z.B. m-Chlorperbenzoesäure, Perameisensäure oder Peressigsäure (bzw. Hydrogenperoxid im Gemisch mit Ameisensäure oder Essigsäure), in Gegenwart eines Verdünnungsmittels wie z.B. Methylenchlorid oder Chloroform, bei Temperaturen zwischen 0 °C und 100 °C umsetzt.

Die als Zwischenprodukte zu verwendenden Ketone sind durch die Formel (VII) allgemein definiert.

In Formel (VII) haben n, Ar, $R^2$, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Ar, $R^2$, $R^3$ und $R^4$ angegeben wurden.

Die Ketone der Formel (VII), in denen $R^2$ für Methyl steht, sind bereits bekannt (vergl. DE-OS 3 021 516). Die Ketone der Formel

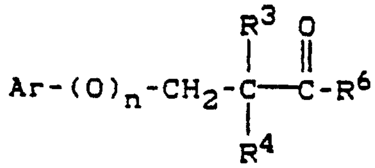 (VII-a)

in welcher
Ar für gegebenenfalls substituiertes Phenyl oder für gegebenenfalls substituiertes Naphthyl steht,
n für die Zahlen 0 oder 1 steht,
$R^3$ für Methyl steht,
$R^4$ für Methyl steht oder
$R^3$ und $R^4$ gemeinsam für Ethan-1,2-diyl stehen und
$R^6$ für Alkyl mit mehr als einem Kohlenstoffatom, Cycloalkyl, Cycloalkylalkyl oder Alkenyl steht,
sind bisher noch nicht bekannt.

Die Ketone der Formel (VII-a) lassen sich herstellen, indem man

d) Verbindungen der Formel

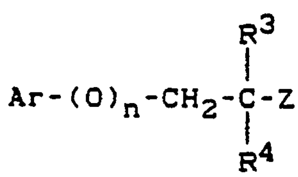 (XII)

in welcher
n, Ar, $R^3$ und $R^4$ die oben angegebenen Bedeutungen haben und
Z für Cyano, Chlorcarbonyl, Methoxycarbonyl oder Ethoxycarbonyl steht,
mit Grignard-Verbindungen der Formel
$R^6$-MgBr (XIII)
in welcher
$R^6$ die oben angegebene Bedeutung hat,
gegebenenfalls in Gegenwart eines Verdünnungsmittels wie z. B. Dietylether, Tetrahydrofuran oder Toluol bzw. Gemische derselben, bei Temperaturen zwischen 0 °C und 100 °C umsetzt.

Die Ketone der Formel (VII-a), in denen $R^3$ und $R^4$ jeweils für Methyl stehen, lassen sich herstellen, indem man

e) Isopropylketone der Formel
$(CH_3)_2CH-CO-R^6$ (XIV)

in welcher
$R^6$ die oben angegebene Bedeutung hat,
mit Verbindungen der Formel
$Ar-(O)_n-CH_2-X^1$ (XV)
in welcher
n und Ar die oben angegebenen Bedeutungen haben und
$X^1$ für Chlor, Brom und Iod steht,
gegebenenfalls in Gegenwart eines Säureakzeptors wie z. B. Kaliumhydroxid, gegebenenfalls in Gegenwart eines Phasentransfer-Katalysators wie z. B. Tetrabutylammoniumbromid und gegebenenfalls in Gegenwart eines Verdünnungsmittels wie z. B. Toluol, bei Temperaturen zwischen 0° C und 100° C umsetzt.

Die Ketone der Formel (VII-a), in denen n für 1 steht und $R^3$ und $R^4$ für Methyl stehen, lassen sich herstellen, indem man

f) Isopropylketone der Formel
$(CH_3)_2CH-CO-R^6$ (XIV)
in welcher
$R^6$ die oben angegebene Bedeutung hat,
mit Formaldehyd oder einer oligomeren Form hiervon, wie z. B. Paraformaldehyd, gegebenenfalls in Gegenwart einer Base, wie z. B. Kaliumhydroxid, und gegebenenfalls in Gegenwart eines Verdünnungsmittels, wie z. B. Methanol, bei Temperaturen zwischen 0° C und 80° C umsetzt, die hierbei erhaltenen Hydroxymethylverbindungen der Formel

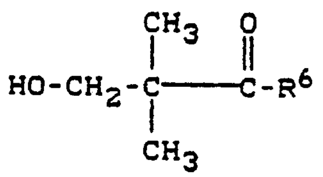 (XVI)

in welcher
$R^6$ die oben angegebene Bedeutung hat,
mit einem Sulfonsäurechlorid der Formel
$R^7-SO_2Cl$ (XVII)
in welcher
$R^7$ für Methyl oder 4-Methyl-phenyl steht,
gegebenenfalls in Gegenwart eines Säurebindemittels, wie z. B. Pyridin, und gegebenenfalls in Gegenwart eines Verdünnungsmittels, wie z. B. Chloroform, bei Temperaturen zwischen 0° C und 50° C umsetzt und die so erhaltenen Sulfonyloxyverbindungen der Formel

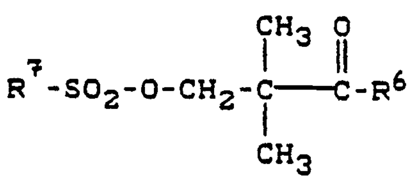 (XVIII)

in welcher
$R^6$ und $R^7$ die oben angegebenen Bedeutung haben,
mit Hydroxyarylverbindungen der Formel
Ar-OH (XIX)
in welcher
Ar die oben angegebene Bedeutung hat,
gegebenenfalls in Gegenwart von Basen, wie z. B. Natriummethylat, und gegebenenfalls in Gegenwart von Verdünnungsmitteln, wie z. B. Ethylenglycol, bei Temperaturen zwischen 0° C und 150° C umsetzt.

Die ketone der Formel (VII-a), in denen $R^3$ und $R^4$ gemeinsam für Ethan-1,2-diyl stehen, lassen sich auch herstellen, indem man

g) Enone der Formel

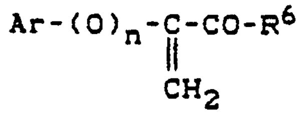

(XX)

in welcher
n, Ar und $R^6$ die oben angegebenen Bedeutungen haben, mit Dimethyloxosulfonium-methylid der Formel

$$\overset{\delta+\,\delta-}{(CH_3)_2SOCH_2} \qquad (VIII)$$

in Gegenwart eines Verdünnungsmittels, wie beispielsweise Dimethylsulfoxid, bei Temperaturen zwischen 20 °C und 80 °C umsetzt.

Die Ketone der Formel (VII-a), in denen $R^6$ für Cyclopropyl steht, lassen sich auch herstellen, indem man

h) Vinylketone der Formel

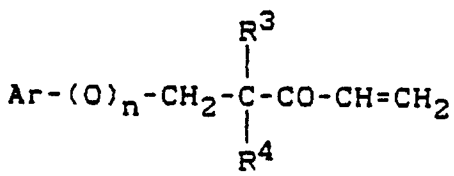

(XXI)

in welcher
n, Ar, $R^3$ und $R^4$ die oben angegebenen Bedeutungen haben, mit Dimethyloxosulfonium-methylid der Formel

$$\overset{\delta+\,\delta-}{(CH_3)_2SOCH_2} \qquad (VIII)$$

in Gegenwart eines Verdünnungsmittels, wie beispielsweise Dimethylsulfoxid, bei Temperaturen zwischen 20 °C und 80 °C umsetzt.

Die Verbundungen der Formeln (VIII), (IX), (X), (XII), (XIII), (XIV), (XV), (XIX), (XX) und (XXI) sind bekannt oder lassen sich nach bekannten Methoden herstellen.

Die Ketone der Formel (VII), in denen $R^2$ für Methyl steht, lassen sich ebenfalls nach den oben angegebenen Verfahren herstellen.

Das beim erfindungsgemäßen Verfahren (b) weiter als Reaktionskomponente benötigte 1,2,4-Triazol der Formel (VI) ist bekannt. Es kann auch als Natrium- oder Kaliumsalz eingesetzt werden.

Die bei dem erfindungsgemäßen Verfahren (b) außerdem als Reaktionskomponenten benötigten Verbindungen sind durch die Formel (IV-a) allgemein definiert. In dieser Formel hat $R^5$ mit Ausnahme von Wasserstoff vorzugsweise diejenigen Bedeutungen, die bereits im Zusammenhang mit der Beschreibung der erfindungsgemäßen Stoffe der Formel (I) vorzugsweise für den Rest $R^1$ genannt wurden. Der Rest Y steht vorzugsweise für Chlor, Brom oder Iod.

Die Verbindungen der Formel (IV-a) sind bekannt.

Als Verdünnungsmittel kommen bei der Durchführung des erfindungsgemäßen Verfahrens (b) neben Wasser alle inerten organischen Solventien in Frage. Vorzugsweise verwendbar sind Alkohole, wie Ethanol und Methoxyethanol; Ketone wie z. B. 2-Butanon; Nitrile wie z. B. Acetonitril; Ester wie z. B. Essigester; Ether wie z. B. Dioxan; aromatische Kohlenwasserstoffe wie z. B. Benzol und Toluol; Amide wie z. B. Dimethylformamid.

Als Säureakzeptoren bzw. Basen kommen bei der Durchführung des erfindungsgemäßen Verfahrens (b) alle üblicherweise verwendbaren anorganischen und organischen Basen in Betracht. Hierzu gehören vorzugsweise Alkalicarbonate wie z. B. Natrium- und Kaliumcarbonat; Alkalihydroxide wie z. B. Natriumhydroxid; Alkalialkoholate wie z. B. Natrium- und Kalium-methylat und -ethylat; Alkalihydride wie z. B. Natriumhydrid; niedere tertiäre Alkylamine, Cycloalkylamine und Aralkylamine wie insbesondere Triethylamin.

Als Radikalstarter kommen bei der Durchführung des erfindungsgemäßen Verfahrens (b) alle für derartige Umsetzungen üblichen Radikalerzeuger in Betracht. Vorzugsweise verwendbar sind $\alpha,\alpha'$-Azoisobutyronitril und Dibenzoyl-peroxid.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 60 °C und 150 °C.

Das erfindungsgemäße Verfahren (b) wird im allgemeinen unter Normaldruck durchgeführt. Es ist jedoch auch mög lich, unter erhöhtem oder vermindertem Druck zu arbeiten.

Zur Durchführung des erfindungsgemäßen Verfahrens (b) setzt man auf 1 Mol Oxiran der Formel (V) im allgemeinen zwischen 1 und 2 Mol, vorzugsweise zwischen 1,1 und 1.5 Mol, 1,2,4-Triazol der Formel (VI) sowie gegebenenfalls zwischen 1 und 10 Mol, vorzugsweise zwischen 1 und 8 Mol, einer Verbindung der Formel (IV-a) ein.

Die Reaktionskomponenten werden im allgemeinen bei Raumtemperatur vermischt und vorzugsweise bei erhöhter Temperatur gerührt, bis die Umsetzung praktisch abgeschlossen ist. Die Aufarbeitung kann nach üblichen Methoden durchgeführt werden. Im allgemeinen verfährt man in der Weise, daß man das Reaktionsgemisch einengt, den Rückstand in Methylenchlorid löst, die Lösung mit Wasser wäscht, mit Natriumsulfat trocknet und filtriert. Vom Filtrat wird das Lösungsmittel im Wasserstrahlvakuum abdestilliert, und das als Rückstand erhaltene Rohprodukt wird gegebenenfalls nach üblichen Methoden, beispielsweise durch Säulenchromatographie, gereinigt.

Die nach den erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmitel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von metallsalz-Komplexen der Verbindungen der Formel (I) kommen vorzugsweise diejenigen Salze von Metallen in Frage, die bereits im Zusammenhang mit der Beschreibung der erfindungsgemäßen Stoffe als bevorzugte Metallsalze genannt wurden.

Die metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide aur Bekämpfung von unerwünschten Mikroorganismen praktisch eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus

(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe zeigen sehr breite in-vitro-Wirksamkeit. Sie können aber auch in-vitro mit sehr gutem Erfolg protektiv gegen beispielsweise Botrytis cinerea an Buschbohnen, Pyrenophora teres an Gerste und Pyricularia oryzae an Reis sowie kurativ beispielsweise gegen Venturia inaequalis an Äpfeln eingesetzt werden. Sie zeigen ferner gute Wirkung gegen Erysiphe graminis, Leptosphaeria nodorum und Puccinia recondita.

Außerdem zeigen die erfindungsgemäßeb Wirkstoffe auch pflanzenwuchsregulierende Eigenschaften. Ferner eignen sich die erfindungsgemäßen Wirkstoffe auch zum Schutz technischer Materialien, wie z.B. Holz, Leder, Papier Textilien, vor Schäden und Zerstörung durch Mikroorganismen, insbesondere durch Pilze.

Die Wirkstoff können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergier mitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.b. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emul gatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzuberei tung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und die Verwendung der erfindungsgemäßen Wirkstoffe werden durch die folgenden Beispiele veranschaulicht.

Herstellungsbeispiele:

Beispiel 1

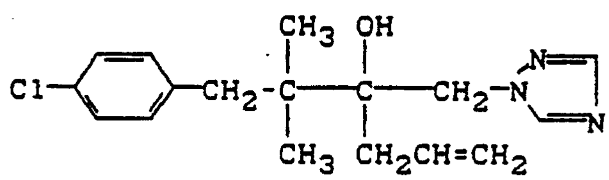

[Verfahren (a)]

Eine Suspension von 2,7 g (0,1 Mol) Aluminium-Blättchen, einem Kristall lod und einer Spatelspitze Quecksilber-(II)-chlorid in 20 ml absolutem Tetrahydrofuran wird über Nacht unter Argon stehengelassen. Ein paar Tropfen von einer Lösung von 12,1 g (0,1 Mol) Allylbromid in 20 ml absolutem Tetrahydrofuran werden zugegeben, und das Gemisch wird in einem Ultraschallbad (Typ: Bransonic 12 der Fa. Branson, Shelton; CT, USA) erwärmt. Bei 50 °C setzt die Reaktion ein, und die restliche Menge Allylbromid wird unter Rühren zugetropft. Anschließend wird auf -20 °C abgekühlt und eine Lösung von 13,9 g (0,05 Mol) 4-(4-Chlorphenyl)-3,3-dimethyl-1-(1,2,4-triazol-1-yl)-2-butanon in 100 ml absolutem Tetrahydrofuran unter Rühren eingetropft. Das Reaktionsgemisch wird 1 Stunde bei 0 °C und 15 Stunden bei 20 °C nachgerührt, dann mit 100 ml 3n-Salzsäure vorsichtig versetzt und unter vermindertem Druck eingeengt.

Der wässrige Rückstand wird mit wässrigem Ammoniak auf pH 10 gestellt, dann mit Wasser verdünnt, anschließend mit Essigester versetzt und unter vermindertem Druck filtriert. Der Nutschenrückstand wird mit Essigester gewaschen, über Natriumsulfat getrocknet und eingeengt. Nach säulenchromatographischer Reinigung des Produktes (Kieselgel; Methylenchlorid/Essigester = 1:1) werden 11,5 g (72 % der Theorie) 6-(4-Chlorphenyl)-5,5-dimethyl-4-(1,2,4-triazol-1-yl-methyl)-1-hexen-4-ol vom Schmelpunkt 97-98 °C erhalten.

Beispiel 2

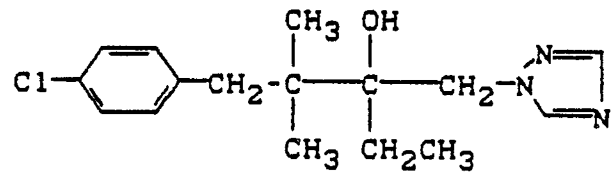

[Verfahren (b)]

Eine Lösung von 17 g (0,07 Mol) 2-[1,1-Dimethyl-2-(4-chlorphenyl)]-ethyl-2-ethyl-oxiran, 6,9 g (0,1 Mol) 1,2,4-Triazol, 0,7 g (0,0175 Mol) Natriumhydroxid, 0,7 ml Wasser und einer Spatelspitze α,α′-Azoisobutyronitril in 100 ml Dimethylformamid wird 15 Stunden auf 120 °C erwärmt. Danach kühlt man auf Raumtemperatur ab, engt durch Abziehen des Lösungsmittels unter vermindertem Druck ein, löst den verbleibenden Rückstand in Dichlormethan, wäscht zweimal mit Wasser, trocknet die organische Phase über Natriumsulfat und entfernt das Lösungsmittel unter vermindertem Druck. Der Rückstand wird durch Säulenchromatographie (Kieselgel; Dichlormethan:Essigester = 1:1) gereinigt.

Man erhält auf diese Weise 8,5 g (39 % der Theorie) an 1-(4-Chlorphenyl)-2,2-dimethyl-3-(1,2,4-triazol-1-yl-methyl)-pentan-3-ol vom Schmelpunkt 124-125 °C.

Analog zu den Beispielen 1 und 2 sowie entsprechend der allgemeinen Beschriebung der erfindungsgemäßen Herstellungsverfahren (a) und (b) werden die in der nachstehenden Tabelle 4 aufgeführten Verbindungen der Formel (I) erhalten.

<u>Tabelle 4</u>:

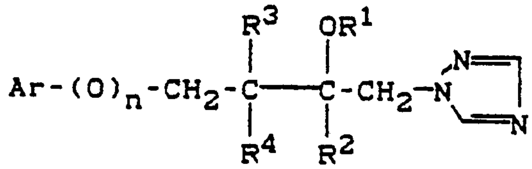 (I)

| Bsp. Nr. | Ar | n | $R^1$ | $R^2$ | $R^3$ | $R^4$ | Schmelzpunkt (°C) |
|---|---|---|---|---|---|---|---|
| 3 | 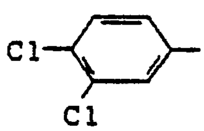 | 0 | H | $-CH_2CH=CH_2$ | $CH_3$ | $CH_3$ | 103 |
| 4 | 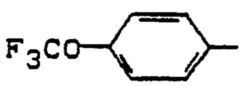 | 0 | H | $-CH_3$ | $CH_3$ | $CH_3$ | 108 |
| 5 | 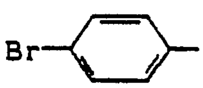 | 0 | H | $-CH_2CH_2CH_3$ | $CH_3$ | $CH_3$ | 125 |
| 6 | 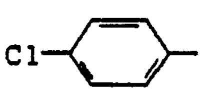 | 0 | H | $-(CH_2)_3-CH_3$ | $CH_3$ | $CH_3$ | 71 |
| 7 | 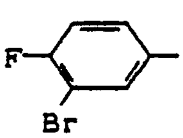 | 0 | H | $-CH_2CH=CH_2$ | $CH_3$ | $CH_3$ | 99 |
| 8 | 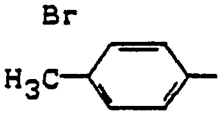 | 0 | H | $-CH_2CH=CH_2$ | $CH_3$ | $CH_3$ | 106 |

Tabelle 4: (Fortsetzung)

| Bsp. Nr. | Ar | n | $R^1$ | $R^2$ | $R^3$ | $R^4$ | Schmelzpunkt (°C) |
|---|---|---|---|---|---|---|---|
| 9 | 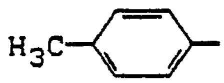 | 0 | H | $-CH_3$ | $CH_3$ | $CH_3$ | 140 |
| 10 | 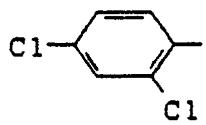 | 0 | H | $-CH_2CH=CH_2$ | $CH_3$ | $CH_3$ | 108 |
| 11 | 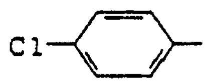 | 0 | H | $-CH(CH_3)_2$ | $CH_3$ | $CH_3$ | 130 |
| 12 | 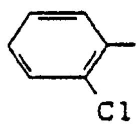 | 0 | H | $-CH_3$ | $CH_3$ | $CH_3$ | 100 |
| 13 | 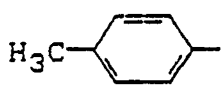 | 0 | H | 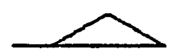 | $CH_3$ | $CH_3$ | 146 |
| 14 | 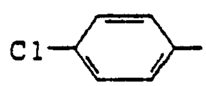 | 0 | $-CH_2CH=CH_2$ | $-CH_3$ | $CH_3$ | $CH_3$ | NMR* |
| 15 | 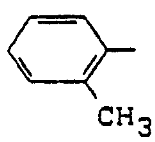 | 0 | H | $-CH_2CH=CH_2$ | $CH_3$ | $CH_3$ | 100 |
| 16 | 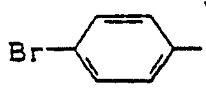 | 0 | H | $-CH_3$ | $CH_3$ | $CH_3$ | 153 |
| 17 | 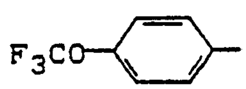 | 0 | H | $-CH_2CH=CH_2$ | $CH_3$ | $CH_3$ | NMR* |
| 18 | 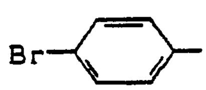 | 1 | H | $-CH_2CH=CH_2$ | $CH_3$ | $CH_3$ | NMR* |

Tabelle 4: (Fortsetzung)

| Bsp. Nr. | Ar | n | $R^1$ | $R^2$ | $R^3$ | $R^4$ | Schmelzpunkt (°C) |
|---|---|---|---|---|---|---|---|
| 19 | 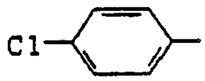 | 0 | H | $-(CH_2)_4-CH_3$ | $CH_3$ | $CH_3$ | 78 |
| 20 | 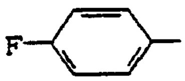 | 0 | H | $-(CH_2)_5-CH_3$ | $CH_3$ | $CH_3$ | NMR* |
| 21 | 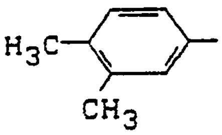 | 0 | H | $-CH_2CH=CH_2$ | $CH_3$ | $CH_3$ | 100 |
| 22 | 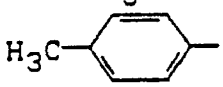 | 0 | H | $-(CH_2)_5-CH_3$ | $CH_3$ | $CH_3$ | NMR* |
| 23 | 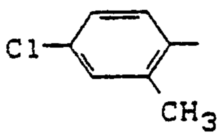 | 1 | H | $-CH_2CH=CH_2$ | $CH_3$ | $CH_3$ | NMR* |
| 24 | 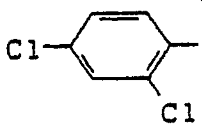 | 1 | H | $-CH_2CH=CH_2$ | $CH_3$ | $CH_3$ | NMR* |
| 25 | 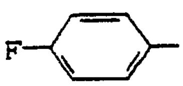 | 0 | H | $-CH_2CH_2CH_3$ | $CH_3$ | $CH_3$ | 87 |
| 26 | 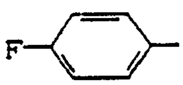 | 0 | H | $-CH(CH_3)_2$ | $CH_3$ | $CH_3$ | 93 |
| 27 | 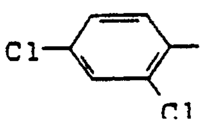 | 0 | H | $-CH_3$ | $CH_3$ | $CH_3$ | 114 |

T a b e l l e 4: (Fortsetzung)

| Bsp. Nr. | Ar | n | $R^1$ | $R^2$ | $R^3$ | $R^4$ | Schmelzpunkt (°C) |
|---|---|---|---|---|---|---|---|
| 28 | 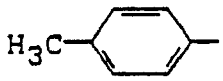 | 0 | H | $-C_2H_5$ | $CH_3$ | $CH_3$ | 120 |
| 29 | 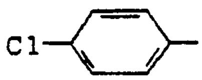 | 0 | H | $-CH_2CH_2CH_3$ | $CH_3$ | $CH_3$ | 126 |
| 30 | 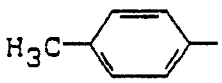 | 0 | H | $-CH(CH_3)_2$ | $CH_3$ | $CH_3$ | 109 |
| 31 | 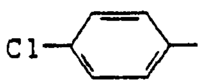 | 0 | H | $-CH_3$ | $CH_3$ | $CH_3$ | 135 |
| 32 | 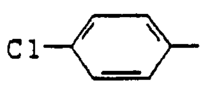 | 1 | H | $-CH_3$ | $CH_3$ | $CH_3$ | 116 |
| 33 | 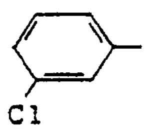 | 1 | H | $-CH_3$ | $CH_3$ | $CH_3$ | 67 |
| 34 | 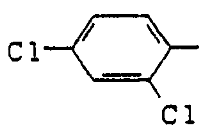 | 1 | H | $-CH(CH_3)_2$ | $CH_3$ | $CH_3$ | 84 |
| 35 | 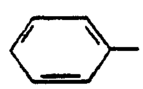 | 1 | H | $-CH(CH_3)_2$ | $CH_3$ | $CH_3$ | 104 |
| 36 | 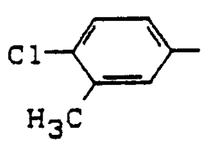 | 1 | H | $-CH(CH_3)_2-$ | $CH_3$ | $CH_3$ | 68 |

Tabelle 4: (Fortsetzung)

| Bsp. Nr. | Ar | n | $R^1$ | $R^2$ | $R^3$ | $R^4$ | Schmelzpunkt (°C) |
|---|---|---|---|---|---|---|---|
| 37 | 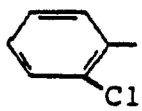 | 0 | H | $CH(CH_3)_2$ | $CH_3$ | $CH_3$ | 89 |
| 38 | 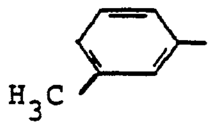 | 0 | H | $CH(CH_3)_2$ | $CH_3$ | $CH_3$ | 83 |
| 39 | 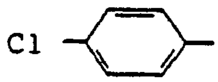 | 0 | H | $CH(CH_3)_2$ | $-CH_2CH_2-$ | | 161 |
| 40 | 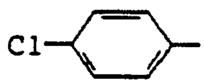 | 1 | H | $CH(CH_3)_2$ | $CH_3$ | $CH_3$ | 79 |

*) Die in den Beispielen 14, 17, 18, 20, 22, 23 und 24 aufgeführten Verbindungen sind durch ihre $^{1}H$-NMR-Spektren (300 MHz; $CDCl_3$) wie folgt charakterisiert:

Bsp. 14: $\delta$ 4,04-3,40 (2H, d/q, $-\underline{CH_2}-CH=CH_2$)
(90 MHz, $CDCl_3$)

Bsp. 17: $\delta$ 2,52-2,35 (2H, d/q, $-\underline{CH_2}-CH=CH_2$)

Bsp. 18: $\delta$ 2,45-2,24 (2H, d/q, $-\underline{CH_2}-CH=CH_2$)

Bsp. 20: $\delta$ 1,65-1,48 (2H, m, $-\underline{CH_2}-C_5H_{11}$), 1,34-1,08 (7H, m, $-CH_2-\overset{H}{\underset{\underline{H}}{C}}-(\underline{CH_2})_3-CH_3$), 0,72-0,56 (1H, m, $-CH_2-\overset{\textcircled{H}}{\underset{H}{C}}-(CH_2)_3-CH_3$)

Bsp. 22: $\delta$ 1,66-1,47 (2H, m, $-\underline{CH_2}-C_5H_{11}$),

1,37-1,10 (7H, m, $-CH_2-\overset{H}{\underset{\underline{H}}{C}}-(\underline{CH_2})_3-CH_3$),

0,71-0,55 (1H, m, $-CH_2-\overset{\textcircled{H}}{\underset{H}{C}}-(CH_2)_3-CH_3$),

Bsp. 23: $\delta$ 2,48-2,24 (2H, d/q, $-\underline{CH_2}-CH=CH_2$)

Herstellung von Ausgangsstoffen der Formel (V):

Beispiel (V-1)

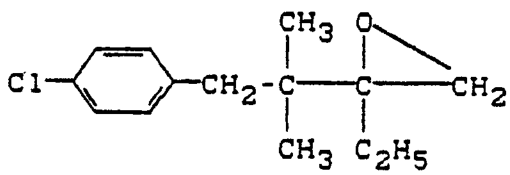

In eine gekühlte Lösung von 10,7 ml (0.13 Mol) Dimethylsulfid in 40 ml absolutem Tetrahydrofuran und 80 ml absolutem Dimethylsulfoxid werden 7,8 ml (0,125 Mol) Iodmethan eingetropft. Das Gemisch wird bei Raumtemperatur (20° C) 16 Stunden gerührt. Eine Lösung von 20,6 g (0,09 Mol) 1-(4-Chlorphenyl)-2,2-dimethyl-3-pentanon in 100 ml absolutem Toluol wird unter Rühren zugegeben. Danach wird das Reaktionsgemisch auf 0 - 5° C gekühlt und mit 7,0 g (0,13 Mol) Natriummethylat versetzt. Man rührt weitere 15 Stunden bei Raumtemperatur nach, versetzt das Gemisch dann mit 1000 ml Wasser, trennt die organische Phase ab und extrahiert die wäßrige Phase mit Toluol. Die vereinigten organischen Phasen werden zweimal mit viel Wasser gewaschen, dann über Natriumsulfat getrocknet und durch Abziehen des Lösungsmittels unter vermindertem Druck eingeengt.
Man erhält 19,3 eines farblosen Öles, das gemäß GC/MS-Analyse zu 72 % aus 2-[2-(4-Chlorphenyl)-1,1-dimethylethyl]-2-ethyl-oxiran besteht. Die Ausbeute errechnet sich danach zu 65,3 % der Theorie. Das Produkt wird ohne zusätzliche Reinigung für die weitere Umsetzung verwendet.

Herstellung von Ausgangsstoffen der Formel (VII):

Beispiel (VII-1)

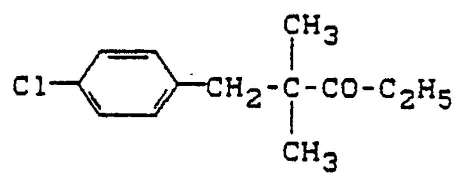

251,6 g (4,5 Mol) Kaliumhydroxid-Pulver und 15 g Tetrabutylammoniumbromid werden in 100 ml Toluol vorgelegt. Eine Lösung von 180,5 g (1,8 Mol) 2-Methyl-3-pentanon in 500 ml Toluol wird unter Rühren eingetropft, wobei die Temperatur unterhalb 20° C gehalten wird. Anschließend wird eine Lösung von 241,5 g (1,5 Mol) 4-Chlorbenzylchlorid in 150 ml Toluol unter Rühren eingetropft, und die Temperatur dabei wieder unterhalb 20° C gehalten. Man rührt weitere 15 Stunden bei Raumtemperatur nach, filtriert die Feststoffe unter Vakuum ab, wäscht mit Toluol nach und engt das Filtrat durch Abziehen des Lösungsmittels unter vermindertem Druck ein. Der Rückstand wird zunächst einer Grobdestillation unterworfen, wobei man 58 g eines Produktgemisches erhält mit Kochpunkt 75-83° C bei 0,1 mbar. Dieses Produktgemisch wird dann unter vermindertem Druck an einer Spaltrohrkolonne feindestilliert.
Man erhält 20,3 g einer farblosen Flüssigkeit, die gemäß GC und NMR zu 96 % aus 1-(4-Chlorphenyl)-2,2-dimethyl-3-pentanon besteht ($Kp_{21 mbar}$ 156-157° C; 6 % der Theorie) und 9,6 g einer ebenfalls farblosen Flüssigkeit, die gemäß GC und NMR zu 96 % aus 1-(4-Chlorphenyl)-2,4-dimethyl-3-pentanon besteht ($Kp_{21 mbar}$ 163-165° C; 2,8 % der Theorie).

Beispiel (VII-2)

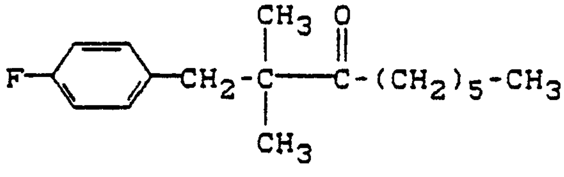

Eine Suspension von 4,8 g (0,21 Mol) Magnesium-Spänen in 80 ml absolutem Tetrahydrofuran wird tropfenweise mit 33 g (0,21 Mol) Hexylbromid versetzt. Nach Ende der Reaktion tropft man eine Lösung von 17,7 g (0,1 Mol) 2,2-Dimethyl-3-(4-fluorphenyl)-propansäurenitril in 150 ml absolutem Toluol ein und erwärmt das Reaktionsgemisch zum Sieden. Man läßt danach so viel Tetrahydrofuran herausdestillieren, bis eine Siedetemperatur von 100 °C erreicht worden ist, und erhitzt dann 15 Stunden unter Rückfluß. Das Reaktionsgemisch wird mit einer wässrigen Ammoniumchlorid-Lösung versetzt, die organische Phase wird abgetrennt und die wässrige Phase zweimal mit Ether extrahiert. Die vereinigten organischen Phasen werden 5 Stunden mit 100 ml 3n Salzsäure gerührt. Nach Abtrennung der wässrigen Phase wird die organische Phase zweimal mit Wasser gewaschen, über Natriumsulfat getrocknet und unter vermindertem Druck eingedampft.
Man erhält 19 g (72 % der Theorie) 2,2-Dimethyl-1-(4-fluorphenyl)-3-nonanon als helles Öl.

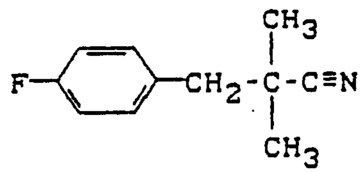

Eine Lösung von 168 g (0,86 Mol) 2,2-Dimethyl-3-(4-fluorphenyl)-propan-1-al-oxim in 400 ml Acetanhydrid wird 4 Stunden unter Rückfluß erwärmt. Danach wird abgekühlt und das Lösungsmittel durch Abziehen unter vermindertem Druck entfernt. Der Rückstand wird mit 2 l Wasser verdünnt, und die entstehende Lösung wird dreimal mit Dichlormethan extrahiert. Die vereinigten organischen Phasen werden einmal mit Wasser gewaschen, über Natriumsulfat getrocknet und durch Abziehen des Lösungsmittels unter vermindertem Druck eingeengt.
Man erhält 150,2 g (98,7 % der Theorie) 2,2-Dimethyl-3-(4-fluorphenyl)-propionsäurenitril als helles Öl.

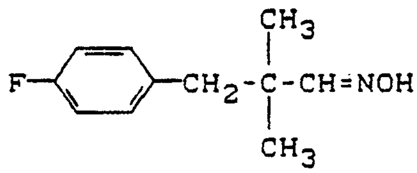

Eine Lösung von 206 g (1,14 Mol) 2,2-Dimethyl-3-(4-fluorphenyl)-propanal in 600 ml Wasser und 600 ml Ethanol wird mit 104,25 g (1,5 Mol) Hydroxylamin-Hydrochlorid und 123 g (1,5 Mol) Natriumacetat versetzt und 5 Stunden unter Rückfluß erwärmt. Das Ethanol wird danach durch Abziehen unter vermindertem Druck entfernt, und der wässrige Rückstand wird mit Wasser verdünnt und dreimal mit Essigester extrahiert. Die vereinigten organischen Phasen werden einmal mit Wasser gewaschen über Natriumsulfat getrocknet und durch Abziehen des Lösungsmittels unter vermindertem Druck eingeengt.
Man erhält 168 g (75,6 % der Theorie) an 2,2-Dimethyl-3-(4-fluorphenyl)-propan-1-al-oxim als helles Öl, das direkt für die weitere Umsetzung verwendet wird.

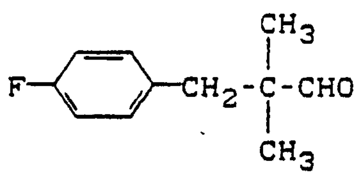

Innerhalb von 3 Stunden wird ein Gemisch von 201 g (2,8 Mol) Isobutyraldehyd und 289 g (2 Mol) 4-Fluorbenzylchlorid in ein gerührtes, auf 80 °C gehaltenes Gemisch von 15 g Tetrabutylammoniumbromid und 240 g (6 Mol) Natriumhydroxid in 600 ml Wasser und 600 ml Toluol eingetropft. Man rührt weitere 3 Stunden bei 80 °C nach, verdünnt mit 500 ml Toluol, trennt die organische Phase ab, wäscht sie einmal mit Wasser und trocknet sie über Natriumsulfat. Das Lösungsmittel wird durch Abziehen unter vermindertem Druck entfernt, und der Rückstand wird unter vermindertem Druck destilliert.
Man erhält 206 g (57,2 % der Theorie) 2,2-Dimethyl-3-(4-fluorphenyl)-propanal vom $Kp_{26mbar}$ 110-118 °C.

Verwendungsbeispiele :

In den folgenden Verwendungsbeispielen wird die Verbindung nachstehender Formel als Vergleichssubstanz eingesetzt:

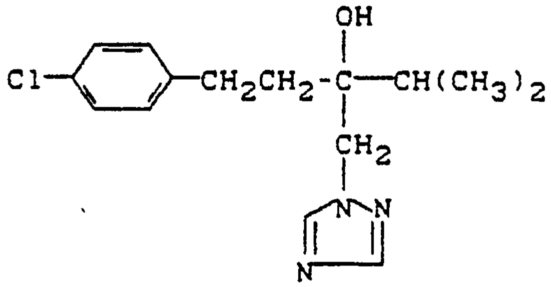

(A)

1-(4-Chlor-phenyl)-4-methyl-3-(1,2,4-triazol-1-yl-methyl)-pentan-3-ol (bekannt aus EP-OS 0 040 345).

Beispiel A

Pyricularia-Test (Reis) /protektiv

| | | |
|---|---|---|
| Lösungsmittel: | 12,5 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mid der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

In diesem Test zeigen die in den Beispielen (2), (6), (7), (10), (11), (18), (19), (24) und (29) aufgeführten erfindungsgemäßen Stoffe eine wesentlich bessere Wirksamkeit als die Vergleichssubstanz (A).

Beispiel B

Botrytis-Test (Bohnen)/protektiv

| | | |
|---|---|---|
| Lösungsmittel: | 4,7 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten

Kammer bei 20°C aufgestellt. 3 Tage nach der Inokulation wird die Größe der Befallsflecken auf den Blättern ausgewertet.

In diesem Test zeigen die in den Beispielen (11) und (18) aufgeführten erfindungsgemäßeb Stoffe eine bessere Wirksamkeit als die Vergleichssubstanz (A).

Beispiel C

Venturia-Test (Apfel) /kurativ

| | | |
|---|---|---|
| Lösungsmittel: | 4,7 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20 °C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigen die in den Beispielen (6), (11), (17) und (18) aufgeführten erfindungsgemäßen Stoffe eine wesentlich bessere Wirksamkeit als die Vergleichssubstanz (A).

Beispiel D

Pyrenophora teres-Test (Gerste) / protektiv

| | | |
|---|---|---|
| Lösungsmittel: | 100 | Gewichtsteile Dimethylformamid |
| Emulgator: | 0,25 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bein einer Temperatur von ca. 20 °C und einer relativen Luftfeuchtigkeit von ca. 80 % aufgestellt.

7 Tage nach der Inokulation erfolgt die Auswertung.

In diesem Test zeigen die in den Beispielen (3), (6), (17) und (29) aufgeführten erfindungsgemäßen Stoffe eine wesentlich bessere Wirksamkeit als die Vergleichssubstanz (A).

**Ansprüche**

1. Substituierte Triazolylmethylcarbinole der Formel

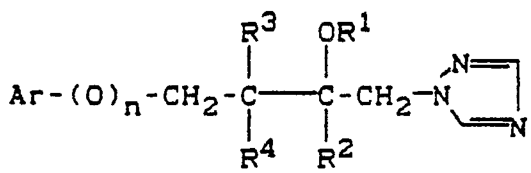

(I)

in welcher
n für die Zahlen 0 oder 1 steht,
Ar für gegebenenfalls substituiertes Phenyl oder für gegebenenfalls substituiertes Naphthyl steht,
$R^1$ für Wasserstoff, gegebenenfalls substituiertes Alkyl, gegebenenfalls substituiertes Alkenyl, gegebenenfalls substituiertes Alkinyl, gegebenenfalls substituiertes Aralkyl, Cycloalkyl oder gegebenenfalls substituiertes Heteroarylalkyl steht,
$R^2$ für Alkyl, Cycloalkyl, Cycloalkylalkyl oder Alkenyl steht,
$R^3$ für Methyl steht und
$R^4$ für Methyl steht oder
$R^3$ und $R^4$ gemeinsam für Ethan-1,2-diyl stehen,
sowie deren Säureadditions-Salze und Metallsalz-Komplexe.

2. Verfahren zur Herstellung von substituierten Triazolylmethylcarbinolen der Formel

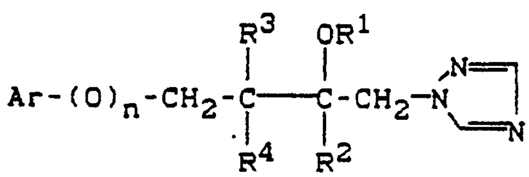

(I)

in welcher
n für die Zahlen 0 oder 1 steht,
Ar für gegebenenfalls substituiertes Phenyl oder für gegebenenfalls substituiertes Naphthyl steht,
$R^1$ für Wasserstoff, gegebenenfalls substituiertes Alkyl, gegebenenfalls substituiertes Alkenyl, gegebenenfalls substituiertes Alkinyl, gegebenenfalls substituiertes Aralkyl, Cycloalkyl oder gegebenenfalls substituiertes Heteroarylalkyl steht,
$R^2$ für Alkyl, Cycloalkyl, Cycloalkylalkyl oder Alkenyl steht,
$R^3$ für Methyl steht und
$R^4$ für Methyl steht oder
$R^3$ und $R^4$ gemeinsam für Ethan-1,2-diyl stehen,
sowie von deren Säureadditions-Salzen und Metallsalz-Komplexen,
dadurch gekennzeichnet, daß man
(a) Triazolylmethylketone der Formel

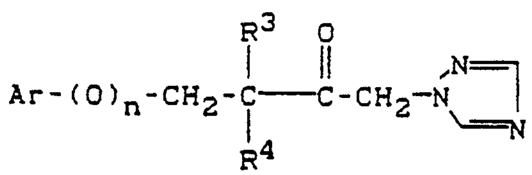

(II)

in welcher
n, Ar, $R^3$ und $R^4$ die oben angegebenen Bedeutungen haben,
mit Halogenverbindungen der Formel
$R^2 - X$ (III)
in welcher
$R^2$ die oben angegebene Bedeutung hat und

X für Halogen steht,
in Gegenwart von Aluminium und gegebenenfalls unter Ultraschall und/oder in Gegenwart von Aluminiumaktivatoren in Gegenwart eines Verdünnungsmittels umsetzt
und anschließend mit Verbindungen der Formel

$R^1$ - Y (IV)

in welcher
$R^1$ die oben angegebene Bedeutung hat und
Y für Halogen steht,
in Gegenwart eines inerten Verdünnungsmittels umsetzt oder
(b) Oxirane der Formel

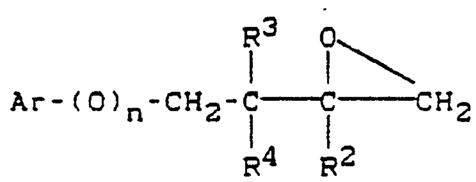 (V)

in welcher
n, Ar, $R^2$, $R^3$ und $R^4$ die oben angegebenen Bedeutungen haben,
mit 1,2,4-Triazol der Formel

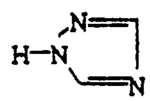 (VI)

gegebenenfalls in Gegenwart eines Säureakzeptors und gegebenenfalls in Gegenwart eines Radikalstarters sowie gegebenenfalls in Gegenwart eines Verdünnungsmittels umsetzt und gegebenenfalls die dabei anfallenden Verbindungen der Formel

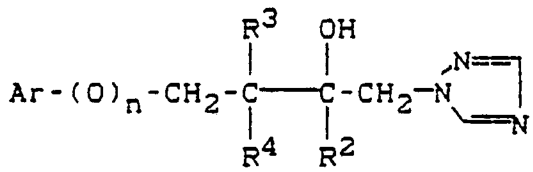 (Ia)

in welcher
Ar, $R^2$, $R^3$, $R^4$ und n die oben angegebene Bedeutung haben,
mit Verbindungen der Formel

$R^5$ - Y (IVa)

in welcher
Y die oben angegebene Bedeutung hat und
$R^5$ für gegebenenfalls substituiertes Alkyl, gegebenenfalls substituiertes Alkenyl, gegebenenfalls substituiertes Alkinyl, gegebenenfalls substituiertes Aralkyl, Cycloalkyl oder gegebenenfalls substituiertes Heteroarylalkyl steht,
in Gegenwart einer Base und gegebenenfalls in Gegenwart eines Verdünnungsmittels umsetzt,
und gegebenenfalls anschließend an die Verbindungen der Formel (I) eine Säure oder ein Metallsalz addiert.

3. Fungizide Mittel, gekennzeichnet durch einen Gehalt an mindestens einem substituierten Triazolylmethylcarbinol der Formel (I) gemäß Anspruch 1 bzw. an einem Säureadditions-Salz oder metallsalz-Komplex eines substituierten Triazolylmethylcarbinols der Formel (I).

4. Verwendung von substituierten Triazolylmethylcarbinolen der Formel (I) gemäß Anspruch 1 bzw. von deren Säureadditions-Salzen und Metallsalz-Komplexen zur Bekämpfung von Pilzen.

5. Verfahren zur Bekämpfung von Pilzen, dadurch gekennzeichnet, daß man substituierte Triazolylmethylcarbinole der Formel (I) gemäß Anspruch 1 bzw. deren Säureadditions-Salze oder Metallsalz-Komplexe auf die Pflanzen und/oder deren Lebensraum ausbringt.

6. Verfahren zur Herstellung von fungiziden Mitteln, dadurch gekennzeichnet, daß man substituierte Triazolylmethylcarbinole der Formel (I) gemäß Anspruch 1 bzw. deren Säureadditions-Salze oder metallsalz-Komplexe mit Streckmitteln und/oder oberflächenaktiven Stoffen vermischt.

7. Oxirane der Formel

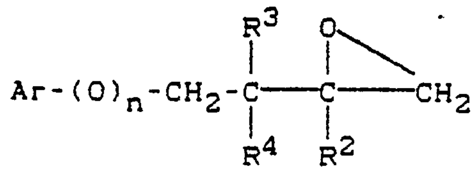

(V)

in welcher
n für die Zahlen 0 oder 1 steht,
Ar für gegebenenfalls substituiertes Phenyl oder für gegebenenfalls substituiertes Naphthyl steht,
$R^2$ für Alkyl, Cycloalkyl, Cycloalkylalkyl oder Alkenyl steht,
$R^3$ für Methyl steht und
$R^4$ für Methyl steht oder
$R^3$ und $R^4$ gemeinsam für Ethan-1,2-diyl stehen.

8. Verfahren zur Herstellung von Oxiranen der Formel

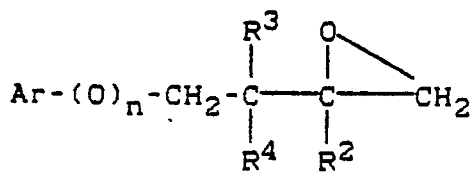

(V)

in welcher
n für die Zahlen 0 oder 1 steht,
Ar für gegebenenfalls substituiertes Phenyl oder für gegebenenfalls substituiertes Naphthyl steht,
$R^2$ für Alkyl, Cycloalkyl, Cycloalkylalkyl oder Alkenyl steht,
$R^3$ für Methyl steht und
$R^4$ für Methyl steht oder
$R^3$ und $R^4$ gemeinsam für Ethan-1,2-diyl stehen,
dadurch gekennzeichnet, daß man
c) Ketone der Formel

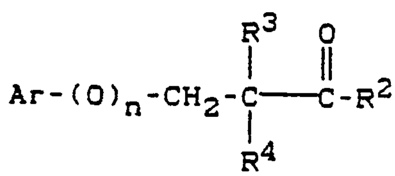

(VII)

in welcher
n, Ar, $R^2$, $R^3$ und $R^4$ die oben angegebenen Bedeutungen haben,
entweder

α) mit Dimethyloxosulfonium-methylid der Formel

$$\overset{\delta^+\delta^-}{(CH_3)_2SOCH_2} \qquad (VIII)$$

in Gegenwart eines Verdünnungsmittels umsetzt,
oder

β) mit Trimethylsulfonium-methylsulfat der Formel

$[(CH_3)_3S^{\oplus}]\ CH_3SO_4^{\ominus}$ (IX)

in Gegenwart eines inerten organischen Lösungsmittels und in Gegenwart einer Base umsetzt, oder

γ) mit Methyl-triphenylphosphoniumbromid der Formel

$H_3C-\overset{\oplus}{P}(C_6H_5)_3\ \overset{\ominus}{Br}$ (X)

in Gegenwart einer Base und gegebenenfalls in Gegenwart eines Verdünnungsmittels umsetzt und die dabei erhaltenen Alkene der Formel

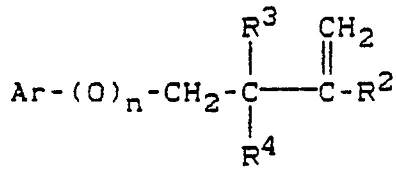

(XI)

in welcher

n, Ar, $R^2$, $R^3$ und $R^4$ die oben angegebenen Bedeutungen haben,

mit Oxigenierungsmitteln in Gegenwart eines Verdünnungsmittels umsetzt.

9. Ketone der Formel

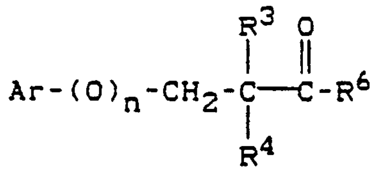

(VII-a)

in welcher

Ar für gegebenenfalls substituiertes Phenyl oder für gegebenenfalls substituiertes Naphthyl steht,

n für die Zahlen 0 oder 1 steht,

$R^3$ für Methyl steht,

$R^4$ für Methyl steht oder

$R^3$ und $R^4$ gemeinsam für Ethan-1,2-diyl stehen und

$R^6$ für Alkyl mit mehr als einem Kohlenstoffatom, Cycloalkyl, Cycloalkylalkyl oder Alkenyl steht.

10. Verfahren zur Herstellung von Ketonen der Formel

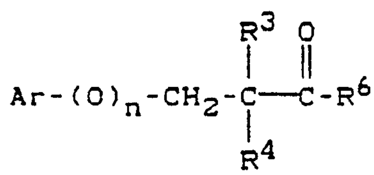

(VII-a)

in welcher

Ar für gegebenenfalls substituiertes Phenyl oder für gegebenenfalls substituiertes Naphthyl steht,

n für die Zahlen 0 oder 1 steht,

$R^3$ für Methyl steht,

$R^4$ für Methyl steht oder

$R^3$ und $R^4$ gemeinsam für Ethan-1,2-diyl stehen und

$R^6$ für Alkyl mit mehr als einem Kohlenstoffatom, Cycloalkyl, Cycloalkylalkyl oder Alkenyl steht,

dadurch gekennzeichnet, daß man

d) Verbindungen der Formel

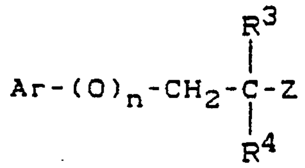 (XII)

in welcher
n, Ar, $R^3$ und $R^4$ die oben angegebenen Bedeutungen haben und
Z für Cyano, Chlorcarbonyl, Methoxycarbonyl oder Ethoxycarbonyl steht,
mit Grignard-Verbindungen der Formel
$R^6$-MgBr (XIII)
in welcher
$R^6$ die oben angegebene Bedeutung hat,
gegebenenfalls in Gegenwart eines Verdünnungsmittels umsetzt,
oder

e) zur Herstellung von Ketonen der Formel (VII-a), in welcher $R^3$ und $R^4$ für Methyl stehen,
Isopropylketone der Formel
$(CH_3)_2CH-CO-R^6$ (XIV)
in welcher
$R^6$ die oben angegebene Bedeutung hat,
mit Verbindungen der Formel
$Ar-(O)_n-CH_2-X'$ (XV)
in welcher
n und Ar die oben angegebenen Bedeutungen haben und
X' für Chlor, Brom und Iod steht,
gegebenenfalls in Gegenwart eines Säureakzeptors, gegebenenfalls in Gegenwart eines Phasentransfer-Katalysators und gegebenenfalls in Gegenwart eines Verdünnungsmittels umsetzt,
oder

f) zur Herstellung von ketonen der Formel (VII-a), in welcher n für 1 steht und $R^3$ und $R^4$ für Methyl stehen,
Isopropylketone der Formel
$(CH_3)_2CH-CO-R^6$ (XIV)
in welcher
$R^6$ die oben angegebene Bedeutung hat,
mit Formaldehyd oder einer oligomeren Form hiervon gegebenenfalls in Gegenwart einer Base und gegebenenfalls in Gegenwart eines Verdünnungsmittels umsetzt und die dabei erhaltenen Hydroxymethylverbindungen der Formel

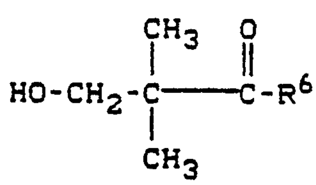 (XVI)

in welcher
$R^6$ die oben angegebene Bedeutung hat,
mit einem Sulfonsäurechlorid der Formel
$R^7-SO_2Cl$ (XVII)
in welcher
$R^7$ für Methyl oder 4-Methyl-phenyl steht,
gegebenenfalls in Gegenwart eines Säurebindemittels und gegebenenfalls in Gegenwart eines Verdünnungsmittels umsetzt und die dabei erhaltenen Sulfonyloxyverbindungen der Formel

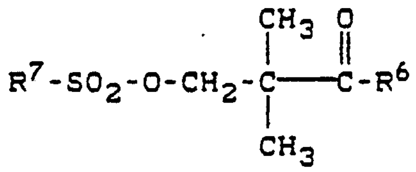

(XVIII)

in welcher
$R^6$ und $R^7$ die oben angegebenen Bedeutung haben,
mit Hydroxyarylverbindungen der Formel

Ar-OH (XIX)

in welcher
Ar die oben angegebene Bedeutung hat,
gegebenenfalls in Gegenwart einer Base und gegebenenfalls in Gegenwart eines Verdünnungsmittels umsetzt,
oder

g) zur Herstellung von ketonen der Formel (VII-a), in welcher $R^3$ und $R^4$ gemeinsam für Ethan-1,2-diyl stehen, Enone der Formel

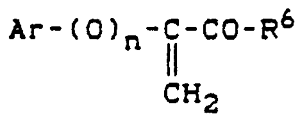

(XX)

in welcher
n, Ar und $R^6$ die oben angegebenen Bedeutungen haben,
mit Dimethyloxosulfonium-methylid der Formel

$$\overset{\delta+\delta-}{(CH_3)_2SOCH_2} \qquad (VIII)$$

in Gegenwart eines Verdünnungsmittels umsetzt,
oder

h) zur Herstellung von Ketonen der Formel (VII-a), in welcher $R^6$ für Cyclopropyl steht, Vinylketone der Formel

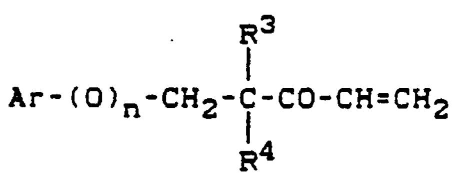

(XXI)

in welcher
n, Ar, $R^3$ und $R^4$ die oben angegebenen Bedeutungen haben,
mit Dimethyloxosulfonium-methylid der Formel

$$\overset{\delta+\delta-}{(CH_3)_2SOCH_2} \qquad (VIII)$$

in Gegenwart eines Verdünnungsmittels umsetzt.